# EUROPEAN PATENT SPECIFICATION

(11) **EP 3 889 181 B1**
(45) Date of publication and mention of the grant of the patent: **24.04.2024**
(21) Application number: 21167811.5
(22) Date of filing: 04.09.2013
(51) Int. Cl.: C07K 16/28, G01N 33/53, A61P 17/00

(54) **AN IL-4R ANTAGONIST ANTIBODY FOR USE IN TREATING ATOPIC DERMATITIS BY ADMINISTERING**
EIN IL-4R-ANTAGONISTISCHER ANTIKÖRPER ZUR VERWENDUNG IN DER BEHANDLUNG ATOPISCHER DERMATITIS
UN ANTICORPS ANTAGONISTE IL-4R POUR L'UTILISATION DANS DES PROCÉDÉS DE TRAITEMENT DE LA DERMATITE ATOPIQUE

(30) Priority: 07.09.2012 US 201261697972 P; 18.12.2012 US 201261738715 P; 03.01.2013 US 201361748588 P; 14.02.2013 US 201361764624 P; 22.02.2013 US 201361768229 P; 27.02.2013 US 201361770091 P; 14.03.2013 US 201361782420 P; 26.04.2013 US 201361816191 P; 10.07.2013 FR 1356759
(43) Date of publication of application: 06.10.2021
(62) Divisional of application: 18161065.0
(73) Proprietor: Regeneron Pharmaceuticals, Inc., Tarrytown, NY 10591 (US); Sanofi Biotechnology, 75008 Paris (FR)
(72) Inventor: ARDELEANU, Marius, New York, 10605 (US); GRAHAM, Neil, New York, 10520 (US); HAMILTON, Jennifer D, New York, 12533 (US); KIRKESSELI, Stephane C, 75008 Paris (FR); KUNDU, Sudeep, New York, 10022 (US); MING, Jeffrey, New Jersey, 08807 (US); RADIN, Allen, New York, 10128 (US); ROCKLIN, Ross E, New Jersey, 07920 (US); WEINSTEIN, Steven P, New York, 10530 (US)
(74) Representative: J A Kemp LLP

(56) References cited:
- WO-A1-2010/053751
- JUDITH HONG ET AL: "Management of Itch in Atopic Dermatitis", SEMINARS IN CUTANEOUS MEDICINE AND SURGERY, vol. 30, no. 2, 14 May 2011 (2011-05-14), pages 71-86, XP028240445, ISSN: 1085-5629, DOI: 10.1016/J.SDER.2011.05.002 [retrieved on 2011-05-14]
- ONG PECK Y: "Editorial update on emerging treatments of atopic dermatitis", EXPERT OPINION ON EMERGING DRUGS ENGLAND, INFORMA HEALTHCARE, UK, vol. 17, no. 2, 1 June 2012 (2012-06-01), pages 129-133, XP009171977, ISSN: 1744-7623, DOI: 10.1517/14728214.2012.668884
- TARUNDEEP KAKKAR ET AL: "Population PK and IgE Pharmacodynamic Analysis of a Fully Human Monoclonal Antibody Against IL4 Receptor", PHARMACEUTICAL RESEARCH, KLUWER ACADEMIC PUBLISHERS-PLENUM PUBLISHERS, NL, vol. 28, no. 10, 21 May 2011 (2011-05-21), pages 2530-2542, XP019950375, ISSN: 1573-904X, DOI: 10.1007/S11095-011-0481-Y
- JENNIFER D HAMILTON ET AL: "Biomarkers elevated in atopic dermatitis (AD) are reduced by therapeutic blockade of IL-4 receptor a (IL-4ralpha) signaling with dupilumab in patients with moderate-to-severe AD. abstract 1042", INTERNATIONAL INVESTIGATIVE DERMATOLOGY, 8 May 2013 (2013-05-08), XP055566280, Edinburgh
- J HAMILTON ET AL: "Blocking IL-4Ralpha with dupilumab (REGN668/SAR231893) rapidly suppresses major pathogenic pathways in severe atopic dermatitis, abstract 1048", INTERNATIONAL INVESTIGATIVE DERMATOLOGY, 8 May 2013 (2013-05-08), XP055566293, Edinburgh
- L BECK ET AL: "Systemic treatment of adult patients with severe atopic dermatitis with the IL-4Ralpha mAb, dupilumab, results in rapid and sustained improvements in disease signs and symptoms, abstarct 1049", INTERNATIONAL INVESTIGATIVE DERMATOLOGY, 8 May 2013 (2013-05-08), XP055566302, Edinburgh
- GIL YOSIPOVITCH ET AL: "IL-4Ralpha mAb dupilumab (REGN668/SAR231893) for the Treatment of Severe Atopic Dermatitis Itch, abstract 1050", INTERNATIONAL INVESTIGATIVE DERMATOLOGY, 8 May 2013 (2013-05-08), XP055566307, Edinburgh
- N N: "Abstracts "Human Clinical Research and Therapeutics"", JOURNAL OF INVESTIGATIVE DERMATOLOGY, vol. 133, no. suppl. 1, 1 May 2013 (2013-05-01), XP055828590,
- Charles Bankhead: "IL-4 Antibody Tames Atopic Dermatitis", Medpage Today, 3 March 2013 (2013-03-03), pages 1-3, XP055566311, Retrieved from the Internet: URL:https://www.medpagetodaY.com/meetingco verage/aad/37636 [retrieved on 2019-03-08]
- A GARRIGA: "71st ANNUAL MEETING OF THE AMERICAN ACADEMY OF DERMATOLOGY (AAD)", DRUGS OF THE FUTURE, 1 March 2013 (2013-03-01), pages 275-279, XP055566346,
- D M PATON: "Dupilumab: human monoclonal antibody against IL-4Ralpha for moderate to severe atopic dermatitis", DRUGS TODAY, vol. 53, no. 9, 1 September 2017 (2017-09-01), pages 477-487, XP055465888, DOI: 10.1358/dot.2017.53.9.2693150
- BECK LISA A ET AL: "Dupilumab treatment in adults with moderate-to-severe atopic dermatitis", NEW ENGLAND JOURNAL OF MEDICINE, THE - NEJM, MASSACHUSETTS MEDICAL SOCIETY, US, vol. 371, no. 2, 10 July 2014 (2014-07-10) , pages 130-139, XP009180485, ISSN: 1533-4406, DOI: 10.1056/NEJMOA1314768
- VAN ASSCHE G ET AL: "Management of loss of response to anti-TNF drugs: Change the dose or change the drug?", JOURNAL OF CROHN'S AND COLITIS, ELSEVIER BV, NL, vol. 2, no. 4, 1 December 2008 (2008-12-01), pages 348-351, XP025692421, ISSN: 1873-9946, DOI: 10.1016/J.CROHNS.2008.05.011 [retrieved on 2008-08-28]
- FOROOGHIAN FARZIN ET AL: "TACHYPHYLAXIS AFTER INTRAVITREAL BEVACIZUMAB FOR EXUDATIVE AGE-RELATED MACULAR DEGENERATION", RETINA, vol. 29, no. 6, 1 June 2009 (2009-06-01), pages 723-731, XP093049977, US ISSN: 0275-004X, DOI: 10.1097/IAE.0b013e3181a2c1c3 Retrieved from the Internet: URL:https://www.ncbi.nlm.nih.gov/pmc/artic les/PMC2770842/pdf/nihms-139616.pdf>
- GASPERINI JULIE L ET AL: "Bevacizumab and ranibizumab tachyphylaxis in the treatment of choroidal neovascularisation", BRITISH JOURNAL OF OPHTHALMOLOGY, vol. 96, no. 1, 26 July 2011 (2011-07-26), pages 14-20, XP093049978, GB ISSN: 0007-1161, DOI: 10.1136/bjo.2011.204685

## Description

### FIELD OF THE INVENTION

The present invention relates to an anti-interleukin-4-receptor (IL-4R) antibody for use in a method of treating moderate-to-severe atopic dermatitis (AD) in a patient.

### BACKGROUND

Atopic dermatitis (AD) is a chronic/relapsing inflammatory skin disease characterized by intense pruritus (e.g., severe itch) and by scaly and dry eczematous lesions. AD is often associated with other atopic disorders such as allergic rhinitis and asthma. Severe disease can be extremely disabling due to major psychological problems, significant sleep loss, and impaired quality of life, leading to high socioeconomic costs.

The pathophysiology of AD is influenced by a complex interplay between Immunoglobulin E (IgE)-mediated sensitization, the immune system, and environmental factors. The primary skin defect may be an immunological disturbance that causes IgE-mediated sensitization, with epithelial-barrier dysfunction that is the consequence of both genetic mutations and local inflammation. AD often begins in childhood before age 5 and may persist into adulthood.

Hamilton, J.D. et al.: "Biomarkers elevated in atopic dermatitis (AD) are reduced by therapeutic blockade of IL-4 receptor a (IL-4ralpha) signaling with dupilumab in patients with moderate-to-severe AD. abstract 1042",INTERNATIONAL INVESTIGATIVE DERMATOLOGY, 8 May 2013 teaches a treatment of moderate-to-severe AD by dupilumab with a maximum dose of 300 mg weekly for 4 weeks,

Typical treatments for AD include topical lotions and moisturizers, topical corticosteroid ointments, creams or injections. Most treatment options, however, offer only temporary, incomplete, symptom relief. Moreover, many patients with moderate-to-severe AD become resistant to treatment by topical corticosteroids or by calcineurin inhibitors. Thus, a need exists in the art for novel targeted therapies for the treatment and/or prevention of AD.

### BRIEF SUMMARY OF THE INVENTION

The present invention provides an anti-interleukin-4-receptor (IL-4R) antibody for use in a method of treating moderate-to-severe atopic dermatitis (AD) in a patient, the method comprising administering an initial dose of the antibody followed by at least eight secondary doses of the antibody, wherein:
- the initial dose is a loading dose of 300mg or 600mg, followed by the secondary doses which are maintenance doses of 300mg;
- each secondary dose is administered to the patient 1 to 2 weeks after the immediately preceding dose; and
- the antibody comprises an HCVR having the amino acid sequence of SEQ ID NO: 162 and an LCVR having the amino acid sequence of SEQ ID NO: 164. In certain embodiments of the invention the patient is resistant to treatment by a topical corticosteroid or a calcineurin inhibitor. In some embodiments, the patient is one with moderate-to-severe AD that is uncontrolled despite treatment with a topical corticosteroid or a calcineurin inhibitor. Exemplary anti-IL-4R antibodies that can be used in the context of the methods of the present invention are described elsewhere herein, including working Example 1. In certain embodiments, the IL-4R antagonist is an anti-IL-4R antibody having the binding characteristics of the reference antibody referred to herein as "mAb1" (*e.g*., an antibody or antigen-binding fragment thereof comprising the complementarity determining regions of mAb1).

Some embodiments of the invention are where the anti-interleukin-4-receptor (IL-4R) antibody is for use where the method treats pruritus in the patient, In some embodiments, the patient suffering from moderate-to-severe AD is resistant to treatment by either a topical corticosteroid or a calcineurin inhibitor.

In certain embodiments, the anti-interleukin-4-receptor (IL-4R) antibody is for use where the method further comprises determining an improvement in an AD-associated parameter. The improvement can be determined or assayed or quantitated by methods well-known in the art. AD-associated parameters and improvements therein are discussed elsewhere herein, including e.g., in working Example 7.

According to certain exemplary embodiments, the anti-interleukin-4-receptor (IL-4R) antibody provided is for use where the method improves one or more AD-associated parameter(s) in a subject in need thereof. Improvements in AD-associated parameters include, *e.g.*, a decrease in Investigator's Global Assessment (IGA) score; a decrease in Body Surface Area Involvement of Atopic Dermatitis (BSA) score; a decrease in Eczema Area and Severity Index (EASI) score; a decrease in SCORAD score; a decrease in 5-D Pruritus Scale; and/or a decrease in Pruritus Numeric Rating Scale (NRS) score. In exemplary embodiments, the improvement in an AD-associated parameter is selected from the group consisting of: (i) a decrease from baseline in IGA score of at least 25%; (ii) a decrease from baseline in BSA score of at least 35%; (iii) a decrease from baseline in EASI score of at least 45%; (iv) a decrease from baseline in SCORAD score of at least 30%; (v) a decrease from baseline in 5-D Pruritus scale of at least 15%; (vi) a decrease from baseline in Pruritus NRS score of at least 25%; and (vii) percent responders with ≥ 50% improvement in EASI (EASI50).

In some embodiments, the improvement in an AD-associated parameter comprises a decrease from baseline in IGA of at least 25% on day 22 through at least day 85 after administration of a pharmaceutical composition comprising the antibody or antigen-binding fragment thereof that binds IL-4R. In some embodiments, the improvement in an AD-associated parameter comprises a decrease from baseline in BSA score of at least 40% on day 29 through at least day 85 following administration of the pharmaceutical composition. In some embodiments, the improvement in an AD-associated parameter comprises a decrease from baseline in EASI score of at least 50% on day 29 through at least day 85 after administration of the pharmaceutical composition. In certain embodiments, the improvement in an AD-associated parameter comprises a decrease from baseline in EASI score of at least 50% on day 29 in at least 70% of subjects administered with the pharmaceutical composition. In some embodiments, the improvement in an AD-associated parameter comprises a decrease from baseline in SCORAD score of at least 30% on day 29 through at least day 85 following administration of the pharmaceutical composition. In some embodiments, the improvement in an AD-associated parameter comprises a decrease from baseline in 5-D pruritus Scale of at least 15% on day 15 through at least day 85 after administration of the pharmaceutical composition. In some embodiments, the improvement in an AD-associated parameter comprises a decrease from baseline in NRS score of at least 25% at the end of week 2 through at least the end of week 10 after administration of the pharmaceutical composition.

In some embodiments, the improvement in an AD-associated parameter comprises a decrease from baseline in IGA of at least 45% on day 85 through at least day 197 after administration of a pharmaceutical composition comprising a therapeutically effective amount of an IL-4R antagonist. In some embodiments, the improvement in an AD-associated parameter comprises a decrease from baseline in BSA score of at least 50% on day 85 through at least day 197 after administration of a pharmaceutical composition comprising a therapeutically effective amount of an IL-4R antagonist. In some embodiments, the improvement in an AD-associated parameter comprises a decrease from baseline in EASI score of at least 60% on day 85 through at least day 197 after administration of a pharmaceutical composition comprising a therapeutically effective amount of an IL-4R antagonist. In some embodiments, the improvement in an AD-associated parameter comprises a decrease from baseline in SCORAD score of at least 45% on day 85 through at least day 197 after administration of a pharmaceutical composition comprising a therapeutically effective amount of an IL-4R antagonist. In some embodiments, the improvement in an AD-associated parameter comprises a decrease from baseline in 5-D Pruritus scale of at least 30% on day 85 through at least day 197 after administration of a pharmaceutical composition comprising a therapeutically effective amount of an IL-4R antagonist. In some embodiments, the improvement in an AD-associated parameter comprises a decrease from baseline in NRS score of at least 50% on day 85 through at least day 197 after administration of a pharmaceutical composition comprising a therapeutically effective amount of an IL-4R antagonist.

According to other exemplary embodiments, the anti-interleukin-4-receptor (IL-4R) antibody is for use where the method comprises selecting a subject who exhibits an elevated level of at least one AD-associated biomarker. Exemplary AD-associated biomarkers that can be evaluated and/or measured in the context of the present invention include, but are not limited to, thymus and activation-regulated chemokine (TARC; also known as CCL17), immunoglobulin E (IgE), eotaxin-3, lactate dehydrogenase (LDH), eosinophils, antigen-specific IgE (e.g., Phadiatop^{™} test), and periostin. In some embodiments, the anti-interleukin-4-receptor (IL-4R) antibody is for use where the method comprises determining the level of an AD-associated biomarker in a patient in need thereof, selecting a patient with an elevated level of the AD-associated biomarker, and then administering the anti-IL-4R antibody. In some embodiments, the patient is selected by acquiring information about the level of an AD-associated biomarker in a patient. In some embodiments, the level of an AD-associated biomarker is determined by an assay or test known in the art or as disclosed elsewhere herein. In one embodiment, the patient is selected on the basis of exhibiting an IgE level greater than about 1500 kU/L prior to or at the time of treatment. In one embodiment, the patient is selected on the basis of exhibiting a TARC level of greater than about 1000 pg/mL prior to or at the time of treatment. According to a related aspect of the present invention, administration of a pharmaceutical composition comprising the anti-IL-4R antibody to the subject results in a decrease in at least one AD-associated biomarker by day 4, 8, 15, 22, 25, 29, 36 or later in the subject following administration. In certain embodiments, the patient exhibits between 5% and 20% decrease in IgE level from the baseline at day 36 or later following administration. In certain embodiments, the patient exhibits between 25% and 70% decrease in TARC level from baseline at day 4 or later following administration.

Also disclosed but not claimed are methods for decreasing the level of one or more AD-associated biomarker(s) in a subject, or improving one or more AD-associated parameter(s) in a subject, wherein the methods comprise sequentially administering to a subject in need thereof a single initial dose of a pharmaceutical composition comprising an IL-4R antagonist, followed by one or more secondary doses of the pharmaceutical composition comprising the IL-4R antagonist.

In some embodiments, the anti-interleukin-4-receptor (IL-4R) antibody provided is for use where the method comprises concomitant administration of the anti-IL-4R antibody and a topical corticosteroid (TCS). In some embodiments, the methods further comprise assaying for an improvement in an AD-associated parameter. In certain embodiments, the anti-interleukin-4-receptor (IL-4R) antibody is for use where the method improves one or more AD-associated parameters, the method comprising concomitantly administering an IL-4R antagonist and a TCS, wherein an improvement in an AD-associated parameter is selected from the group consisting of: (i) a decrease from baseline in IGA score of at least 45%; (ii) a decrease from baseline in BSA score of at least 40%; (iii) a decrease from baseline in EASI score of at least 65%; (iv) a decrease from baseline in SCORAD score of at least 50%; (v) a decrease from baseline in 5-D Pruritus scale of at least 25%; and (vi) a decrease from baseline in Pruritus NRS score of at least 60%. In some embodiments, the improvement in an AD-associated parameter is a decrease from baseline in IGA of at least 50% on day 29 after administration of the antibody or antigen-binding fragment thereof that binds IL-4R. In some embodiments, the improvement in an AD-associated parameter is a decrease from baseline in NRS of at least 65% on day 29 after administration. In some embodiments, the improvement in an AD-associated parameter is a decrease from baseline in EASI of at least 70% on day 29 after administration. In some embodiments, the improvement in an AD-associated parameter is a decrease from baseline in SCORAD of at least 60% on day 29 after administration.

In certain embodiments, the TCS is selected from the group consisting of a group I TCS, a group II TCS and a group III TCS. In some embodiments, the TCS is selected from the group consisting of methylprednisolone aceponate, mometasone furoate, fluticasone propionate, betamethasone valerate and hydrocortisone butyrate.

In related embodiments, the anti-interleukin-4-receptor (IL-4R) antibody provided is for use where the method reduces the dependence on TCS in a patient with moderate-to-severe AD, the method comprising concomitant administration of the anti-IL-4R antibody and a TCS, wherein the dosage of the TCS is reduced by 50% as compared to subjects without the administration of the IL-4R antagonist. In one embodiment, the anti-interleukin-4-receptor (IL-4R) antibody provided is for use where the method reduces the dosage of a TCS in treatment of moderate-to-severe AD, comprising administration of an IL-4R antagonist concomitantly with a reduced dosage of the TCS. The dosage of the TCS may be reduced by more than, for example, 10%, 20%, 30%, 40%, or 50%. In one embodiment, the dosage of the TCS may be reduced by more than, for example, 10%, 20%, 30%, 40%, or 50% as compared to the dosage used by the subject before treatment with the IL-4R antagonist.

The anti-interleukin-4-receptor (IL-4R) antibody provided may be for use wherein the method improves an AD-associated parameter, decreases the level of at least one AD-associated biomarker, and/or for treats any of the other indications of moderate-to-severe AD disclosed herein.

In some embodiments, the pharmaceutical composition is administered subcutaneously or intravenously to the patient. In certain embodiments, the pharmaceutical composition is administered to the patient before, after or concurrent with a second therapeutic agent. In some embodiments, the second therapeutic agent is a topical corticosteroid (TCS) or a calcineurin inhibitor.

Also disclosed but not claimed is a method for monitoring the effectiveness of treatment of moderate-to-severe AD in a subject with an IL-4R antagonist, the method comprising: (a) determining the expression level of an AD-associated biomarker, such as TARC or serum IgE in a biological sample acquired from the subject before treatment with the IL-4R antagonist; (b) determining the expression level of one or both of TARC and serum IgE in a biological sample acquired from the subject after treatment with the IL-4R antagonist; (c) comparing the level determined in step (a) with the level in step (b); and (d) concluding that the treatment is effective when the level determined in step (b) is lower than the level determined in step (a), or concluding that the treatment is not effective when the level determined in step (b) is the same or higher than the level determined in step (a). In one embodiment, the level in step (b) is determined 1 week, 2 weeks, 3 weeks, 4 weeks, or 5 weeks after determining the level in step (a). In one instance, the biomarker is TARC, and if TARC levels decrease following administration of the IL-4R antagonist, then treatment with the IL-4R antagonist is determined to be effective.

The expression level of the biomarker can be determined, for example, 1 week, 2 weeks, 3 weeks, 4 weeks, 5 weeks, or longer after administration of the IL-4R antagonist, and compared to the expression level prior to administration of the antagonist. The dose or the dosing regimen of the IL-4R antagonist (e.g., an anti-IL4R antibody) can be adjusted following the determination. For example, if the expression of the biomarker fails to decrease within 1 week, 2 weeks, 3 weeks, 4 weeks, 5 weeks, or longer following administration of the antagonist, then treatment with the antagonist can be stopped, or the dose of the antagonist can be increased. If expression of the biomarker decreases following administration of the antagonist, the dosage of the antagonist can be maintained or decreased, such as to identify a minimal effective dose. In some instances, treatment is maintained at the minimal effective dose.

Also disclosed but not claimed are methods for monitoring a subject's response to treatment with an IL-4R antagonist, wherein the subject has moderate-to-severe AD, the method comprising: (a) acquiring information regarding the expression level of one or both of TARC and IgE in a biological sample from the subject following administration of the IL-4R antagonist to the subject; and (b) providing an indication that the treatment should be continued if the expression level of TARC or IgE has decreased as compared to the level before treatment with the IL-4R antagonist. In one instance the biomarker is TARC, and if TARC levels are determined to decrease following administration of the antagonist, then an indication is provided to continue treatment with the IL-4R antagonist.

The moderate-to-severe atopic dermatitis (AD) may be e.g., moderate to severe eosinophilic AD, extrinsic AD, or intrinsic AD.

The IL-4R antagonist provided for use by the invention is an anti-IL-4R antibody or antigen-binding fragment thereof that comprises an HCVR having the amino acid sequence of SEQ ID NO: 162 and an LCVR having the amino acid sequence of SEQ ID NO: 164.

The patient with moderate-to-severe AD may be a patient having an elevated level of a biomarker selected from the group consisting of thymus and activation-regulated chemokine (TARC), IgE, eotaxin-3, lactate dehydrogenase (LDH), and periostin.

The invention further includes the treatment resulting in a decrease in an AD-associated biomarker in the subject by day 4, 8, 15, 22, 25, 29 or 36 following treatment as compared to the level of biomarker in the subject prior to treatment. In certain embodiments, the AD-associated biomarker is one or both of TARC and IgE.

Also disclosed but not claimed are methods of monitoring whether a therapeutic dose of an interleukin-4 receptor (IL-4R) antagonist administered to a human subject is safe, said method comprising: acquiring information regarding the safety of the antagonist following administration to a human, wherein the information includes the occurrence of one or more events selected from the group consisting of an anaphylactic reaction or acute allergic reaction requiring immediate treatment, severe injection site reaction lasting longer than 24 hours, severe infection, any parasitic infection, alanine aminotransferase (ALT) increase ≥ 2 Upper Limit Normal Range (ULN), QTc >=500 ms, pregnancy, overdose, and herpes simplex type II viral infection; determining that the one or more said events has occurred, determining that said therapeutic dose is not safe, and, optionally advising that the therapeutic dose be discontinued or lowered.

Also disclosed but not claimed are methods of monitoring whether a therapeutic dose of an interleukin-4 receptor (IL-4R) antagonist administered to a human subject is safe, said method comprising: acquiring information regarding the safety of the antagonist following administration to a human, wherein the information includes the occurrence of one or more events selected from the group consisting of: anaphylactic reaction or acute allergic reaction requiring immediate treatment, severe injection site reaction lasting longer than 24 hours, severe infection, any parasitic infection, alanine aminotransferase (ALT) increase ≥ 2 Upper Limit Normal Range (ULN), QTc >=500 ms, pregnancy, overdose, and herpes simplex type II viral infection; determining that the one or more of said events has not occurred; and determining that said therapeutic dose is safe.

In one instance, the infection is upper respiratory tract injection, pharyngitis, or sinusitis. In one embodiment, the injection site reaction is erythema, pain, nodule, hematoma or pruritus. In one instance, the pain is greater than 2 mm VAS, e.g., 3 mm to 30 mm VAS. In one instance, the erythema diameter is ≥ 9mM.

Also disclosed but not claimed are methods of quantifying or monitoring an amount of anti-drug antibodies in blood serum of a human subject following administration of drug wherein the drug is an interleukin-4 receptor (IL-4R) antagonist, said method comprising: (a) obtaining a sample of said blood serum from a human subject who was administered a dose of said IL-4R antagonist; and (b) determining the amount of anti-drug antibodies in said serum sample.

Also disclosed but not claimed are methods of comparing an interleukin-4 receptor (IL-4R) antagonist manufactured by a first process and proposed equivalent second process, said method comprising: acquiring information regarding the safety of the antagonist following administration of the antagonist manufactured by the first process to a first human, and following administration of the antagonist manufactured by the second process to a second human, wherein the information includes: one or more events selected from the group consisting of an anaphylactic reaction or acute allergic reaction requiring immediate treatment, severe injection site reaction lasting longer than 24 hours, severe infection, any parasitic infection, alanine aminotransferase (ALT) increase ≥ 2 Upper Limit Normal Range (ULN), QTc >=500 ms, pregnancy, overdose, and herpes simplex type II viral infection; and wherein if the information is not significantly different for the antagonist manufactured by the first process and the antagonist manufactured by the second process, then the two processes are determined to be acceptable for manufacturing equivalent antagonists; and wherein if the information is determined to be significantly different for the antagonist manufactured by the first process and the antagonist manufactured by the second process, then the two processes are determined to be unacceptable for manufacturing equivalent antagonists.

Also disclosed but not claimed are methods of comparing an interleukin-4 receptor (IL-4R) antagonist manufactured by a first process and proposed equivalent second process, said method comprising: acquiring information regarding a therapeutic dose of the antagonist following administration of the dose of the antagonist manufactured by the first process to a first human, and following administration of the dose of the antagonist manufactured by the second process to a second human, wherein the information includes one or more of: (a) area under the plasma concentration versus time curve calculated using the trapezoidal method from time zero to real time (AUCₗₐₛₜ) from about 4 mg·h/ml to about 20 mg·h/ml; (b) maximum plasma concentration observed (Cₘₐₓ) from about 15 ug/ml to about 42 ug/ml; (c) first time to reach a maximum plasma concentration (tₘₐₓ) from about 40 hr to about 280 hr; (d) area under the plasma concentration versus time curve extrapolated to infinity (AUC) from about 5,000,000ng/h*mL to about 25,000,000 ng/h*mL and (e) time to reach terminal half-life of (t_{1/2}^{z}) from about 50 h to about 200 h, wherein if the information is not significantly different for the antagonist manufactured by the first process and the antagonist manufactured by the second process, then the two processes are determined to be acceptable for manufacturing equivalent antagonists; and wherein if the information is determined to be significantly different for the antagonist manufactured by the first process and the antagonist manufactured by the second process, then the two processes are determined to be unacceptable for manufacturing equivalent antagonists.

Also disclosed but not claimed is a therapeutic dosage form of a pharmaceutical composition comprising an interleukin-4 receptor (IL-4R) antagonist, wherein administration of the dose form to a human provides one or more of: (a) an area under the plasma concentration versus time curve calculated using the trapezoidal method from time zero to real time (AUCₗₐₛₜ) from about 4 mg·h/ml to about 20 mg·h/ml; (b) a maximum plasma concentration observed (Cₘₐₓ) from about 15 ug/ml to about 42 ug/ml; (c) a first time to reach a maximum plasma concentration (tₘₐₓ) from about 40 hr to about 280 hr; (d) an area under the plasma concentration versus time curve extrapolated to infinity (AUC) from about 5,000,000ng/h*mL to about 25,000,000 ng/h*mL and (e) a time to reach terminal half-life of (t_{1/2}^{z}) from about 50 h to about 200 h.

In one instance, the safe therapeutic dose is equal to or less than 500 mg. In one embodiment, the safe therapeutic dose is selected from the group consisting of 75 mg, 150 mg, and 300 mg.

Also disclosed but not claimed are methods and compositions that are useful in vaccine applications Also disclosed are methods for enhancing or potentiating the immune response against an antigen in a subject. In some instances, the methods for enhancing or potentiating the immune response against an antigen in a subject comprise administering a pharmaceutical composition comprising the antigen and an IL-4R antagonist. Some instances are related to methods comprising (a) administering a vaccine composition comprising the antigen to the subject; and (b) administering an IL-4R antagonist prior to, concurrent with, and/or subsequent to administration of the vaccine composition to the subject. Also disclosed are pharmaceutical compositions to enhance or potentiate an immune response against an antigen in a subject, the compositions comprising: (a) the antigen; and (b) an IL-4R antagonist. In one exemplary instance, the IL-4R antagonist is an anti-IL-4R antibody (as exemplified in Example 1 herein). In certain instances, the IL-4R antagonist is an anti-IL-4R antibody having the binding characteristics of the reference antibody referred to herein as "mAb1" (*e.g*., an antibody or antigen-binding fragment thereof comprising the complementarity determining regions of mAb1).

Other embodiments of the present invention will become apparent from a review of the ensuing detailed description.

### BRIEF DESCRIPTION OF THE FIGURES

**Figure 1** shows a Cartesian plot of mean (SD) serum functional mAb1 concentration-time profiles following a single subcutaneous dose.
**Figure 2** shows a diagrammatic representation of the injection procedure and pain assessments as described in Example 5.
**Figure 3** shows the IGA score responder rate (score of 0 or 1) - last observation carried forward (LOCF) for the study in Example 6.
**Figure 4** shows the mean IGA score change from baseline - LOCF for the study in Example 6.
**Figure 5** shows the mean IGA score percent change from baseline - LOCF for the study in Example 6.
**Figure 6** shows mean EASI score change from baseline - LOCF for the study in Example 6.
**Figure 7** shows mean EASI score percent change from baseline - LOCF for the study in Example 6.
**Figure 8** shows EASI50 responder rate - LOCF for the study in Example 6.
**Figure 9** shows mean BSA change from baseline - LOCF for the study in Example 6.
**Figure 10** shows mean BSA percent change from baseline - LOCF for the study in Example 6.
**Figure 11** shows mean 5-D change from baseline - LOCF for the study in Example 6.
**Figure 12** shows mean 5-D percent change from baseline - LOCF for the study in Example 6.
**Figure 13** shows mean NRS change from baseline - LOCF for the study in Example 6.
**Figure 14** shows mean NRS percent change from baseline -LOCF for the study in Example 6.
**Figure 15** shows percent change from baseline in BSA in patients administered 75 mg, 150 mg or 300 mg of anti-IL-4R antibody vs. placebo for the study in Example 8.
**Figure 16** shows percent change from baseline in IGA in patients administered 75 mg, 150 mg or 300 mg of anti-IL-4R antibody vs. placebo for the study in Example 8.
**Figure 17** shows percent change from baseline in EASI in patients administered 75 mg, 150 mg or 300 mg of anti-IL-4R antibody vs. placebo for the study in Example 8.
**Figure 18** shows percent change from baseline in Pruritus NRS in patients administered 75 mg, 150 mg or 300 mg of anti-IL-4R antibody vs. placebo for the study in Example 8.
**Figure 19** shows EASI response time course in patients with moderate-to-severe AD to 300 mg anti-IL-4R antibody for the study in Example 8.
**Figure 20** shows the percent responders in the EASI score administered with 75 mg, 150 mg or 300 mg anti-IL-4R antibody vs. placebo for the study in Example 8.
**Figure 21** shows EASI responses at week 4 (day 29) to anti-IL-4R antibody administered at 75 mg, 150 mg or 300 mg doses vs. placebo for the study in Example 8.
**Figure 22** shows proportion of patients achieving IGA ≤ 1 for the study in Example 8.
**Figure 23** shows mean EASI score percent change from baseline to the last observation carried forward (LOCF) for the study in Example 10.
**Figure 24** shows IGA score responder rate 9 score of 0 or 1) up to LOCF for the study in Example 10.
**Figure 25** shows IGA score responder rate (reduction in score of 2 or more) up to LOCF for the study in Example 10.
**Figure 26** shows EASI score responder rate (50% score reduction from baseline) up to LOCF for the study in Example 10.
**Figure 27** shows mean EASI score change from baseline up to LOCF for the study in Example 10.
**Figure 28** shows mean IGA score change from baseline up to LOCF for the study in Example 10.
**Figure 29** shows mean IGA score percent change from baseline up to LOCF for the study in Example 10.
**Figure 30** shows mean BSA change from baseline up to LOCF for the study in Example 10.
**Figure 31** shows mean SCORAD score change from baseline up to LOCF for the study in Example 10.
**Figure 32** shows mean NRS score change from baseline up to LOCF for the study in Example 10.
**Figure 33** shows mean 5-D Pruritus score change from baseline up to LOCF for the study in Example 10.
**Figure 34** shows mean EASI score percent change from baseline - censored LOCF, for the study in Example 11.
**Figure 35** shows mean EASI score change from baseline - censored LOCF, for the study in Example 11.
**Figure 36** shows EASI50 responder rate - censored LOCF, for the study in Example 11.
**Figure 37** shows a Kaplan-Meier plot of Time to first EASI50 - censored LOCF, for the study in Example 11.
**Figure 38** shows mean IGA score percent change from baseline - censored LOCF, for the study in Example 11.
**Figure 39** shows mean IGA score change from baseline - censored LOCF, for the study in Example 11.
**Figure 40** shows IGA score responder rate (score of 0 or 1) - censored LOCF, for the study in Example 11.
**Figure 41** shows a Kaplan-Meier plot of time to first IGA ≤ 1 - censored LOCF, for the study in Example 11.
**Figure 42** shows proportion of patients with IGA≤ 1 at each visit who remained relapse-free - censored LOCF, for the study in Example 11.
**Figure 43** shows proportion of patients with reduction from baseline in IGA ≥ 2 at each visit - censored LOCF, for the study in Example 11.
**Figure 44** shows mean SCORAD score percent change from baseline - censored LOCF, for the study in Example 11.
**Figure 45** shows mean SCORAD score change from baseline - censored LOCF, for the study in Example 11.
**Figure 46** shows mean pruritus NRS percent change from baseline - censored LOCF, for the study in Example 11.
**Figure 47** shows mean pruritus NRS change from baseline - censored LOCF, for the study in Example 11.
**Figure 48** shows serum IgE levels at baseline (A) and median percentage change in response to various doses of mAb1 or placebo (B) for the study in Example 12.
**Figure 49** shows serum TARC levels at baseline (A) and mean percentage change in response to various doses of mAb1 or placebo (B) for the study in Example 12.
**Figure 50** shows change in TARC levels for pooled mAb1 group compared to placebo for the study in Example 12.
**Figure 51** shows the distribution of baseline levels of (A) TARC, (B) total serum IgE, and (C) lactate dehydrogenase (LDH) in patients in the study in section B of Example 12.
**Figure 52** shows the median percent change in IgE from baseline for the study in section B of Example 12.
**Figure 53** shows the median percent change in LDH from baseline for the study in section B of Example 12.
**Figure 54** shows the median percent change in TARC from baseline for the study in section B of Example 12.
**Figure 55** shows the median percent change in IgE from baseline for the study in section C of Example 12.
**Figure 56** shows the median percent change in TARC from baseline for the study in section C of Example 12.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims. The technical disclosure set out below may in some respects go beyond the scope of the claims. Elements of the disclosure which do not fall within the scope of the claims are provided for information.

The present invention does not encompass methods of treatment practiced on the human or animal body. Hence, reference herein to methods of treatment practiced on the human or animal body should be construed as the products discussed for use in such methods.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (*e.g*., 99.1, 99.2, 99.3, 99.4, etc.). As used herein, the terms "treat", "treating", or the like, mean to alleviate symptoms, eliminate the causation of symptoms either on a temporary or permanent basis, or to prevent or slow the appearance of symptoms of the named disorder or condition.

The present invention provides an anti-interleukin-4-receptor (IL-4R) antibody for use in a method of treating moderate-to-severe atopic dermatitis (AD) in a patient, the method comprising administering an initial dose of the antibody followed by at least eight secondary doses of the antibody, wherein:
- the initial dose is a loading dose of 300mg or 600mg, followed by the secondary doses which are maintenance doses of 300mg;
- each secondary dose is administered to the patient 1 to 2 weeks after the immediately preceding dose; and
- the antibody comprises an HCVR having the amino acid sequence of SEQ ID NO: 162 and an LCVR having the amino acid sequence of SEQ ID NO: 164.

As used herein, the expression "a subject in need thereof" or "patient" means a human or non-human animal that exhibits one or more symptoms or indicia of atopic dermatitis, and/or who has been diagnosed with moderate-to-severe atopic dermatitis. In certain embodiments, the anti-interleukin-4-receptor (IL-4R) antibody is for use in methods to treat patients that show elevated levels of one or more AD-associated biomarkers (described elsewhere herein). For example, the methods comprise administering an IL-4R antagonist to patients with elevated levels of IgE or TARC or periostin. In some embodiments, the methods herein may be used to treat AD in children who are ≤ 1 year old. For example, the present methods may be used to treat infants who are less than 1 month, 1 month, 2 months, 3 months, 4 months, 5 months, 6 months, 7 months, 8 months, 9 months, 10 months, 11 months or less than 12 months old. In other embodiments, the anti-interleukin-4-receptor (IL-4R) antibody may be for use in methods to treat children and/or adolescents who are ≤ 18 years old. For example, the present methods may be for children or adolescents less than 17 years, 16 years, 15 years, 14 years, 13 years, 12 years, 11 years, 10 years, 9 years, 8 years, 7 years, 6 years, 5 years, 4 years, 3 years, or less than 2 years old.

In the context of the present invention, "a subject in need thereof" or "patient" is one with moderate-to-severe atopic dermatitis (AD) and may include, *e.g*., subjects who, prior to treatment, exhibit (or have exhibited) one or more AD-associated parameters such as, *e.g*., elevated IGA, BSA, EASI, SCORAD, 5D-Pruritus, and/or NRS score, and/or an elevated level of one or more AD-associated biomarker such as, *e.g*., IgE and/or TARC (as described elsewhere herein). In certain embodiments, "a subject in need thereof" may include a subset of population which is more susceptible to AD or may show an elevated level of an AD-associated biomarker. For example, "a subject in need thereof" or "patient" may include a subset of population defined by a race or an ethnicity present in the population.

"Atopic dermatitis" (AD), as used herein, means an inflammatory skin disease characterized by intense pruritus (*e.g*., severe itch) and by scaly and dry eczematous lesions. The term "atopic dermatitis" includes, but is not limited to, AD caused by or associated with epidermal barrier dysfunction, allergy (*e.g*., allergy to certain foods, pollen, mold, dust mite, animals, etc.), radiation exposure, and/or asthma. The anti-interleukin-4-receptor (IL-4R) antibody is for use wherein the patient is one with moderate-to-severe or severe AD. As used herein, "moderate-to-severe AD", is characterized by intensely pruritic, widespread skin lesions that are often complicated by persistent bacterial, viral or fungal infections. Moderate-to-severe AD also includes chronic AD in patients. In many cases, the chronic lesions include thickened plaques of skin, lichenification and fibrous papules. Patients affected by moderate-to-severe AD also, in general, have more than 20% of the body's skin affected, or 10% of skin area in addition to involvement of the eyes, hands and body folds. Moderate-to-severe AD is also considered to be present in patients who require frequent treatment with topical corticosteroids. A patient may also be said to have moderate-to-severe AD when the patient is resistant or refractory to treatment by either a topical corticosteroid or a calcineurin inhibitor or any other commonly used therapeutic agent known in the art.

The present invention includes the anti-interleukin-4-receptor (IL-4R) antibody for use wherein the methods treat both the extrinsic and the intrinsic forms of AD. The extrinsic form of AD associated with IgE-mediated sensitization and increased levels of Th2 cytokines involves 70% to 80% of patients with AD. The intrinsic form without IgE-mediated sensitization involves 20% to 30% of patients with AD; these patients have lower levels of IL-4 and IL-13 than extrinsic AD.

The anti-interleukin-4-receptor (IL-4R) antibody for use of the present invention includes treating AD in patients resistant, non-responsive or inadequately responsive to treatment with a topical corticosteroid (TCS) or a calcineurin inhibitor. The term "resistant, non-responsive or inadequately responsive to a TCS or a calcineurin inhibitor", as used herein, refers to subjects or patients with AD who have been treated with a TCS or a calcineurin inhibitor and wherein the TCS/calcineurin inhibitor does not have a therapeutic effect. In some embodiments, the term refers to reduced patient compliance and/or toxicity and side effects and/or ineffectiveness of the administered TCS/calcineurin inhibitor to reduce, ameliorate or decrease the symptoms of AD. In some embodiments, the term refers to patients suffering from moderate-to-severe AD who are refractory to treatment by a TCS/calcineurin inhibitor. In some embodiments, the term refers to patients with AD which is uncontrolled despite treatment with a TCS and/or calcineurin inhibitor. In some embodiments, the patients who are "resistant, non-responsive or inadequately responsive to a TCS or a calcineurin inhibitor" may show no improvement in one or more AD-associated parameters. Examples of AD-associated parameters are described elsewhere herein. For example, treatment with a TCS/calcineurin inhibitor may result in no decrease in pruritus or EASI score or BSA score. In some embodiments, the present invention includes the anti-interleukin-4-receptor (IL-4R) antibody for use in methods to treat moderate-to-severe AD in patients who have been treated earlier with a TCS/calcineurin inhibitor for ≥ 1 month and do not show a decrease in one or more AD-associated parameters. For example, the patient may have chronic AD who has been on a stable regimen of a TCS/calcineurin inhibitor and has a BSA score of ≥ 10% or an IGA score ≥ 3.

In alternate embodiments, the term "subject in need thereof" includes patients with moderate-to-severe AD who have been administered one or more TCS for more than 6 months, more than 1 year, more than 2 years, more than about 5 years, more than about 7 years, or more than about 10 years. The patients may desire to minimize or avoid the adverse side effects of the TCS. The present invention includes the anti-interleukin-4-receptor (IL-4R) antibody for use in methods for long-term safer and more effective management of moderate-to-severe AD in a patient, the methods comprising administering the IL-4R antagonist concomitantly with a TCS wherein the dosage of the TCS is adjusted to minimize or prevent adverse side effects of the TCS. In certain embodiments, the present invention includes the anti-interleukin-4-receptor (IL-4R) antibody for use in methods to reduce dependence on TCS in a patient with moderate-to-severe AD; the methods comprising administering the IL-4R antagonist concomitantly with a potent TCS wherein the amount of TCS used by the patient is reduced by about 50% as compared to a patient not administered the IL-4R antagonist. In certain embodiments, the present invention includes the anti-interleukin-4-receptor (IL-4R) antibody for use in methods that reduce dependence on TCS in a patient with moderate-to-severe AD, the methods comprising administering the IL-4R antagonist concomitantly with a potent TCS wherein the amount of TCS used by the patient is reduced by about 50% as compared to the amount used by the patient before treatment with the IL-4R antagonist. In certain embodiments, the administration of the IL-4R antagonist and a TCS results in additive or synergistic activity in treating AD as compared to monotherapy.

The term "TCS", as used herein includes group I, group II, group III and group IV topical corticosteroids. According to the Anatomical Therapeutic Classification System of World Health Organization, the corticosteroids are classified as weak (group I), moderately potent (Group II) and potent (Group III) and very potent (Group IV), based on their activity as compared to hydrocortisone. Group IV TCS (very potent) are up to 600 times as potent as hydrocortisone and include clobetasol propionate and halcinonide. Group III TCS (potent) are 50 to 100 times as potent as hydrocortisone and include, but are not limited to, betamethasone valerate, betamethasone dipropionate, diflucortolone valerate, hydrocortisone-17-butyrate, mometasone furoate, and methylprednisolone aceponate. Group II TCS (moderately potent) are 2 to 25 times as potent as hydrocortisone and include, but are not limited to, clobetasone butyrate, and triamcinolone acetonide. Group I TCS (mild) includes hydrocortisone.

### Improving Atopic Dermatitis (AD)-Associated Parameters

Also disclosed but not claimed is an anti-interleukin-4-receptor (IL-4R) antibody for use in methods for improving one or more atopic dermatitis (AD)-associated parameters in a subject in need thereof, wherein the methods comprise administering a pharmaceutical composition comprising the interleukin-4 receptor (IL-4R) antagonist to the subject.

Examples of "AD-associated parameters" include: (a) Investigators Global Assessment (IGA); (b) Body Surface Area Involvement of Atopic Dermatitis (BSA); (c) Eczema Area and Severity Index (EASI); (d) SCORAD; (e) 5-D Pruritus Scale; and (f) Pruritus Numeric Rating Scale (NRS). An "improvement in an AD-associated parameter" means a decrease from baseline of one or more of IGA, BSA, EASI, SCORAD, 5-D Pruritus Scale, or NRS. As used herein, the term "baseline," with regard to an AD-associated parameter, means the numerical value of the AD-associated parameter for a subject prior to or at the time of administration of a pharmaceutical composition of the present invention.

To determine whether an AD-associated parameter has "improved," the parameter is quantified at baseline and at one or more time points after administration of the pharmaceutical composition comprising the anti-interleukin-4-receptor (IL-4R) antibody. For example, an AD-associated parameter may be measured at day 1, day 2, day 3, day 4, day 5, day 6, day 7, day 8, day 9, day 10, day 11, day 12, day 14, day 15, day 22, day 25, day 29, day 36, day 43, day 50, day 57, day 64, day 71, day 85; or at the end of week 1, week 2, week 3, week 4, week 5, week 6, week 7, week 8, week 9, week 10, week 11, week 12, week 13, week 14, week 15, week 16, week 17, week 18, week 19, week 20, week 21, week 22, week 23, week 24, or longer, after the initial treatment with a pharmaceutical composition of the present invention. The difference between the value of the parameter at a particular time point following initiation of treatment and the value of the parameter at baseline is used to establish whether there has been an "improvement" (*e.g*., a decrease) in the AD associated parameter.

Investigator's Global Assessment (IGA). The IGA is an assessment scale used in clinical settings to determine the severity of AD and clinical response to treatment based on a 6-point scale ranging from 0 (clear) to 5 (very severe). According to certain embodiments of the present invention, administration of the IL-4R antagonist to a patient results in a decrease in IGA score. For example, a decrease from baseline in IGA score of at least about 10%, 15%, 20%, 25%, 30%, 35%, 40%, 45%, 50%, 55%, 60%, 65%, 70%, 75%, or more at day 4, 8, 15, 22, 25, 29, 36, 43, 50, 57, 64, 71, 85 or later follows administration of the IL-4R antagonist (*e.g*., following subcutaneous administration of about 75 mg, 150 mg, or 300 mg of an anti-IL-4R antibody or antigen-binding fragment thereof). In certain instances, administration of an IL-4R antagonist to a subject results in a decrease from baseline in IGA of at least 25%. In one instance, administration of the IL-4R antagonist to a subject results in a decrease from baseline in IGA of at least 25% by day 15 after administration. In certain instances, administration of an IL-4R antagonist to a subject results in a decrease from baseline in IGA of at least 35% by day 22 after administration. In other instances, administration of an IL-4R antagonist to a subject results in a decrease from baseline in IGA of at least 40% or at least 45% through day 85 upon treatment.

Body Surface Area Involvement of Atopic Dermatitis (BSA). BSA is assessed for each major section of the body (head, trunk, arms and legs) and is reported as a percentage of all major body sections combined. According to certain instances, administration of an IL-4R antagonist to a patient results in a decrease in BSA score. For example, therapeutic methods which result in a decrease from baseline in BSA score of at least about 10%, 15%, 20%, 25%, 30%, 35%, 40%, 45%, 50%, 55%, 60%, 65%, 70%, 75% or more at day 4, 8, 15, 22, 25, 29, 36, 43, 50, 57, 64, 71, 85 or later following administration of the IL-4R antagonist (*e.g*., following subcutaneous administration of about 300 mg of an anti-IL-4R antibody or antigen-binding fragment thereof) are disclosed but not claimed. In certain exemplary instances, administration of an IL-4R antagonist to a subject results in a decrease from baseline in BSA score of at least 35% after administration. In one instance, administration of an IL-4R antagonist to a subject results in a decrease from baseline in BSA score of at least 35% by day 29 after administration. In one instance, administration of an IL-4R antagonist to a subject results in a decrease from baseline in BSA score of at least 40% by day 29 after administration. In some instances, administration of an IL-4R antagonist to a subject results in a decrease from baseline in BSA score of at least 40% or at least 50% through day 85 upon treatment.

Eczema Area and Severity Index (EASI). The EASI is a validated measure used in clinical settings to assess the severity and extent of AD. (Hanifin et al. 2001, Exp. Dermatol. 10:11-18). Four AD disease characteristics are assessed for severity by a physician or other qualified medical professional on a scale of 0 (absent) through 3 (severe). In addition, the area of AD involvement is assessed as a percentage by body area of head, trunk, arms and legs and converted to a score of 0 to 6. According to certain instances, administration of an IL-4R antagonist to a patient results in a decrease in EASI score. For example, therapeutic methods may result in a decrease from baseline in EASI score of at least about 10%, 15%, 20%, 25%, 30%, 35%, 40%, 45%, 50%, 55%, 60%, 65%, 70%, 75% or more at day 4, 8, 15, 22, 25, 29, 36, 43, 50, 57, 64, 71, 85 or later following administration of the IL-4R antagonist (e.g., following subcutaneous administration of about 300 mg of an anti-IL-4R antibody or antigen-binding fragment thereof). In certain exemplary instances, administration of an IL-4R antagonist to a subject results in a decrease from baseline in EASI score of at least 45%. In one instance, administration of an IL-4R antagonist to a subject results in a decrease from baseline in EASI score of at least 45% by day 15 after administration. In one instance, administration of an IL-4R antagonist to a subject results in a decrease from baseline in EASI score of at least 50% by day 29 after administration. In some instances, administration of an IL-4R antagonist to a subject results in a decrease from baseline in EASI score of at least 55% or at least 60% through day 85 upon treatment.

SCORAD. SCORing Atopic Dermatitis (SCORAD) is a clinical assessment of the severity (*e.g*., extent or intensity) of atopic dermatitis developed by the European Task Force on Atopic Dermatitis (Consensus Report of the European Task Force on Atopic Dermatitis, 1993, Dermatology (Basel) 186(1):23-31). The extent of AD is assessed as a percentage of each defined body area and reported as the sum of all areas, with a maximum score of 100% (assigned as "A" in the overall SCORAD calculation). The severity of 6 specific symptoms of AD is assessed using the following scale: none (0), mild (1), moderate (2), or severe (3) (for a maximum of 18 total points, assigned as "B" in the overall SCORAD calculation). Subjective assessment of itch and sleeplessness is recorded for each symptom by the patient or relative on a visual analogue scale (VAS), where 0 is no itch (or sleeplessness) and 10 is the worst imaginable itch (or sleeplessness), with a maximum possible score of 20. This parameter is assigned as "C" in the overall SCORAD calculation. The SCORAD is calculated as: A/5 + 7B/2 + C. According to certain instances, administration of an IL-4R antagonist to a patient results in a decrease in SCORAD score. For example, therapeutic methods which result in a decrease from baseline in SCORAD of at least about 10%, 15%, 20%, 25%, 30%, 35%, 40%, 45%, 50%, 55%, 60%, 65%, 70%, 75% or more at day 4, 8, 15, 22, 25, 29, 36, 43, 50, 57, 64, 71, 85 or later following administration of the IL-4R antagonist (*e.g*., following subcutaneous administration of about 300 mg of an anti-IL-4R antibody or antigen-binding fragment thereof) are disclosed. In certain instances, administration of an IL-4R antagonist to a subject results in a decrease from baseline in SCORAD score of at least 30%. In one instance, administration of an IL-4R antagonist to a subject results in a decrease from baseline in SCORAD score of at least 30% by day 29 after administration. In one instance of the present invention, administration of an IL-4R antagonist to a subject results in a decrease from baseline in SCORAD score of at least 35% by day 29 after administration. In some instances, administration of an IL-4R antagonist to a subject results in a decrease from baseline in SCORAD score of at least 40% or at least 45% through day 85 upon treatment.

5-D Pruritus Scale. The 5-D Pruritus Scale is a 1-page, 5-question tool used in clinical settings to assess 5 dimensions of background itch: degree, duration, direction, disability, and distribution. (Elman and Hynan, 2010, Brit. J. Dermatol. 162:587-593). Each question corresponds to 1 of the 5 dimensions of itch; patients rate their symptoms as "present" or on a 1 to 5 scale, with 5 being the most affected. According to certain instances, administration of an IL-4R antagonist to a patient results in a decrease in 5-D Pruritus Scale. For example, therapeutic methods which result in a decrease from baseline in 5-D Pruritus Scale of at least about 10%, 15%, 20%, 25%, 30%, 35%, 40%, 45%, 50%, 55%, 60%, 65%, 70%, 75% or more at day 4, 8, 15, 22, 25, 29, 36, 43, 50, 57, 64, 71, 85 or later following administration of the IL-4R antagonist (*e.g*., following subcutaneous administration of about 300 mg of an anti-IL-4R antibody or antigen-binding fragment thereof) are disclosed but not claimed. In certain instances, administration of an IL-4R antagonist to a subject results in a decrease from baseline in 5-D Pruritus Scale of at least 15%. In one instance, administration of an IL-4R antagonist to a subject results in a decrease from baseline in 5-D Pruritus Scale of at least 15% by day 15 after administration. In one instances, administration of an IL-4R antagonist to a subject results in a decrease from baseline in 5-D Pruritus Scale of at least 20% by day 15 after administration. In some instances, administration of an IL-4R antagonist to a subject results in a decrease from baseline in 5-D Pruritus Scale of at least 25% or at least 30% through day 85 upon treatment.

Pruritus Numeric Rating Scale (NRS). The Pruritus NRS is a single-question assessment tool that is used to assess a subject's worst itch, on a scale of 1 to 10, as a result of AD in the previous 12 hours. According to certain instances, administration of an IL-4R antagonist to a patient results in a decrease in NRS score. For example, therapeutic methods which result in a decrease from baseline in NRS score of at least about 10%, 15%, 20%, 25%, 30%, 35%, 40%, 45%, 50%, 55%, 60%, 65%, 70%, 75% or more at the end of week 1, 2, 3, 4, 5, 6, 7, 8, 9, 10, 11, 12, or later following administration of the IL-4R antagonist (*e.g*., following subcutaneous administration of about 300 mg of an anti-IL-4R antibody or antigen-binding fragment thereof) are disclosed but not claimed. In certain exemplary instances, administration of an IL-4R antagonist to a subject results in a decrease from baseline in NRS score of at least 25%. In one instance, administration of an IL-4R antagonist to a subject results in a decrease from baseline in NRS score of at least 25% by the end of week 2 after administration. In one instances, administration of an IL-4R antagonist to a subject results in a decrease from baseline in NRS score of at least 30% by the end of week 2 after administration. In some instances, administration of an IL-4R antagonist to a subject results in a decrease from baseline in NRS score of at least 45% or at least 50% through day 85 upon treatment.

Global Individual Signs Score (GISS). Individual components of the AD lesions (erythema, infiltration/population, excoriations, and lichenification) is rate globally (i.e., each assessed for the whole body, not by anatomical region) on a 4-point scale (from 0=none to 3=severe) using the EASI severity grading criteria.

Pruritus Categorical Scale. The Pruritus categorical scale is a 4-point scale used to assess symptoms that has been used in clinical studies of AD and has less of a "middling" effect (Kaufmann 2006). The scale is rated as follows: 0: absence of pruritus; 1: mild, pruritus (occasional slight itching/scratching); 2: moderate pruritus (constant or intermittent itching/scratching that does not disturb sleep) and 3: severe pruritus (bothersome itching/scratching that disturbs sleep).

Patient Oriented Eczema Measure (POEM). The POEM is a 7-item, validated questionnaire used in clinical practice and clinical trials to assess disease symptoms in children and adults (Charman 2004). The format is a response to 7 items (dryness, itching, flaking, cracking, sleep loss, bleeding, and weeping) with a scoring system of 0 to 28; a high score is indicative of a poor QOL.

Dermatology Life Quality Index (DLQI). The DLQI is a 10-item, validated questionnaire used in clinical practice and clinical trials to assess the impact of AD disease symptoms and treatment on QOL (Badia 1999). The format is a simple response to 10 items, which assess QOL over the past week, with an overall scoring system of 0 to 30; a high score is indicative of a poor QOL.

Itchy QOL. Itchy QOL is a validated pruritus-specific instrument that addresses the symptom, emotional, and functional impact of pruritus. There is an overall score as well as subscale scores to address the 3 types of impact. This is a reliable, valid, and responsive questionnaire (Desai 2008).

EQ-5D. The EQ-5D is a standardized measure of health status developed by the EuroQOI Group in order to provide a simple, generic measure of health for clinical and economic appraisal. The EQ-5D as a measure of health related QOL, defines health in terms of 5 dimensions: mobility, self-care, usual activities, pain/discomfort, and anxiety/depression. Each dimension has 3 ordinal levels of severity: "no problem" (1), "some problems" (2), "severe problems" (3). Overall health state is defined as a 5-digit number. Health states defined by the 5-dimensional classification can be converted into corresponding index scores that quantify health status, where 0 represents "death" and 1 represents "perfect health."

HADS. The HADS is a general Likert scale used to detect states of anxiety and depression (Bjelland 2002). The 14 items on the questionnaire include 7 that are related to anxiety and 7 that are related to depression. Each item on the questionnaire is scored; a person can score between 0 and 21 for either anxiety or depression.

Patient Global Assessment of Disease Status and Treatment Effect. Patients rate their overall wellbeing based on a 5-point Likert scale from poor to excellent. Patients are asked: "Considering all the ways in which your eczema affects you, indicate how well you are doing". Response choices are: "Poor"; "Fair"; "Good"; "Very Good"; "Excellent".

For treatment effect, patients rate their satisfaction with the study treatment based on a 5-point Likert scale from poor to excellent. Patients are asked: "How would you rate the way your eczema responded to the study medication?" Response choices are: "Poor"; "Fair"; "Good"; "Very Good"; "Excellent".

### Methods for Long-term Management of Atopic Dermatitis

Also disclosed but not claimed are methods for long term management of moderate-to-severe AD in a patient. In certain instances, the methods comprise administering the IL-4R antagonist concomitantly with a conventional therapeutic agent such as a topical corticosteroid (TCS).

The term "conventional therapeutic agent", as used herein, refers to therapeutic agents and drugs commonly or routinely used to treat AD in patients. Conventional therapeutic agents include systemic as well as topical therapeutics. For example, the most commonly or frequently prescribed drugs are the topical corticosteroids (TCS). Other examples of such agents include, but are not limited to, topical calcineurin inhibitors, anti-histamines, oral immunosuppressants, and glucocorticoids, systemic immunosuppressants such as methotrexate, cyclosporine, and azathioprine. Conventional therapeutic agents are used to relieve the symptoms of AD; however have numerous and considerable adverse side effects including diabetes, hypertension, osteoporosis, myelosuppression, nephrotoxicity, hepatotoxicity, leucopenia, an increased risk of microbial infections. Topical agents such as corticosteroids and calcineurin inhibitors are not recommended for long-term application due to the risk of irreversible skin atrophy, dyspigmentation, acneiform eruptions and risks associated with systemic absorption including skin malignancies and lymphomas. Also repetitive application of any topical therapies over a long period of time can erode patient compliance.

The term "long-term management of AD", as used herein, refers to treatment or containment of one or more symptoms or disease conditions of AD over a long period of time, typically more than about 2 years, more than about 5 years, more than about 10 years, or more than about 20 years. Long-term management of AD includes methods of treatment or methods to improve one or more AD-associated parameters over a period of more than 6 months, more than 1 year, more than 2 years, or more than about 5 years, the methods comprising administering the anti-IL-4R antibody in combination with a conventional therapeutic agent such as TCS. The administration regimen and dosage of the IL-4R antibody and the TCS is adjusted or varied such that one or more AD-associated parameters is significantly improved as well as the toxicity due to the conventional agent is prevented or minimized. In some instances, the IL-4R antibody may be administered in higher loading doses for significant improvement in an AD-associated parameter followed by lower regular doses to sustain or maintain the improvement. The concomitantly administered TCS may be administered at a reduced dose, typically reduced by about 20%, about 30%, about 40%, about 50% or about 60% as compared to a patient not treated with the IL-4R antibody. The administration regimens and dosage amounts are described elsewhere herein. Also disclosed but not claimed are methods to reduce dependence on TCS in a patient with moderate-to-severe AD.

In certain embodiments, the anti-interleukin-4-receptor (IL-4R) antibody for use provided is for use in methods that treat patients who have AD for more than 1 year, more than about 5 years, more than about 10 years, or more than about 15 years, the methods comprising administering a therapeutically effective amount of an IL-4R antagonist in combination with a conventional therapeutic agent such as TCS.

Also disclosed but not claimed are methods for a safer and/or more effective therapy in the long-term management of moderate-to-severe AD in patients. The term "safer and/or more effective therapy", as used herein, refers to methods of treatment comprising administering an IL-4R antagonist in combination with a conventional therapeutic agent such as TCS such that one or more AD-associated parameters is significantly improved as well as the side effects and toxicity due to the conventional agent is minimized or prevented. In certain instances, the improvement in an AD-associated parameter is selected from the group consisting of: (a) a decrease from baseline in Investigator's Global Assessment (IGA) score of at least 50%; (b) a decrease from baseline in Pruritus Numeric Rating Scale (NRS) score of at least 65%; (c) a decrease from baseline in Eczema Area and Severity Index (EASI) score of at least 70%; and (d) a decrease from baseline in SCORAD score of at least 60%. In some instances, the dosage of the conventional agent is reduced or lowered to minimize the adverse side effects. In some instances, the methods of treatment as described herein may reduce or eliminate the risk of rebound after steroid reduction or discontinuation.

Also disclosed but not claimed are methods for more effective and safer therapy in long-term management of AD in patients including in children or young adults who may be more susceptible or sensitive to a conventional therapeutic agent.

Also disclosed but not claimed are methods for reducing or eliminating an AD patient's dependence or conventional therapeutics such as TCS during the treatment of moderate-to-severe AD. In some instances, the methods comprise: selecting a patient with moderate-to-severe AD that is uncontrolled or partially controlled with a background therapy; administering to the patient a defined dose of the anti-IL-4R antibody, for an initial treatment period while maintaining the patient's background therapy for the initial treatment period; and gradually reducing the dosage of one or more components of the background therapy over a subsequent period of treatment, while continuing to administer the IL-4R antagonist. The term "background therapy" as used herein, refers to standard or conventional therapeutic agents known in the art which are used for treating AD (described elsewhere herein). In certain instances, the background therapy comprises a TCS, or a topical calcineurin inhibitor. In one instance, the background therapy is a potent Group III TCS such as mometasone furoate or methylprednisolone aceponate. In some instances, the dosage of the conventional therapeutic such as TCS is eliminated or completely withdrawn upon the initial treatment period. For example, a TCS is administered in an initial treatment period and completely stopped or withdrawn in the subsequent treatment period. In certain instances, the TCS is reduced by about 10%, about 20%, about 30%, about 40%, about 50%, or more as compared to the dose during the initial treatment period.

In one example of a treatment regimen for a patient with moderate-to-severe AD wherein an IL-4R antagonist is administered to a patient with moderate-to-severe AD, during an initial treatment period (also called the "stable phase"), a conventional therapeutic such as a TCS administered to the patient as background therapy. During a subsequent treatment period (also called "withdrawal phase"), the administration of the TCS is gradually reduced by about 5 - 60% as compared to the initial treatment period. In one instance, the TCS is stopped, i.e., the TCS is gradually reduced over the subsequent treatment period until it is withdrawn or eliminated.

Also disclosed but not claimed are methods of treating AD comprising an add-on therapy to background therapy with systematic background therapy withdrawal. In certain instances, an IL-4R antagonist is administered as an add-on therapy to an AD patient who is on background therapy for a certain period of time (e.g., 1 week, 2 weeks, 3 weeks, 1 month, 2 months, 5 months, 12 months, 18 months, 24 months, or longer) (also called the "stable phase"). In some instances, the background therapy comprises a TCS. The stable phase is followed by a background therapy withdrawal phase, wherein one or more components comprising the background therapy are withdrawn, or reduced or eliminated, while the add-on therapy continues. In some instances, the background therapy may be reduced by about 5%, about 10%, about 20%, about 30%, about 40%, about 50% or by more during the withdrawal phase. The withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more.

### Atopic Dermatitis-Associated Biomarkers

Also disclosed are methods involving the use, quantification, and analysis of AD-associated biomarkers. As used herein, the term "AD-associated biomarker" means any biological response, cell type, parameter, protein, polypeptide, enzyme, enzyme activity, metabolite, nucleic acid, carbohydrate, or other biomolecule which is present or detectable in an AD patient at a level or amount that is different from (e.g., greater than or less than) the level or amount of the marker present or detectable in a non-AD patient. In some instances, the term "AD-associated biomarker" includes a biomarker associated with Type 2 helper T-cell (Th2)-driven inflammation. Exemplary AD-associated biomarkers include, but are not limited to, e.g., thymus and activation-regulated chemokine (TARC; also known as CCL17), immunoglobulin E (IgE), eotaxin-3 (also known as CCL26), lactate dehydrogenase (LDH), eosinophils, antigen-specific IgE (e.g., Phadiatop^{™} test), and periostin. The term "AD-associated biomarker" also includes a gene or gene probe known in the art which is differentially expressed in a subject with AD as compared to a subject without AD. For example, genes which are significantly up-regulated in a subject with AD include, but are not limited to, T-helper 2 (Th2)-associated chemokines such as CCL13, CCL17, CCL18 and CCL26, markers of epidermal proliferation such as K16, Ki67, and T-cell and dendritic cell antigens CD2, CD1b, and CD1c (Tintle et al 2011; J. Allergy Clin. Immunol. 128: 583-593). Alternatively, "AD-associated biomarker" also includes genes which are down regulated due to AD such as terminal differentiation proteins (e.g., loricrin, filaggrin and involucrin) (Tintle et al 2011; J. Allergy Clin. Immunol. 128: 583-593). Certain embodiments of the invention pertain to use of these biomarkers for monitoring disease reversal with the administration of the IL-4R antagonist. Methods for detecting and/or quantifying such AD-associated biomarkers are known in the art; kits for measuring such AD-associated biomarkers are available from various commercial sources; and various commercial diagnostic laboratories offer services which provide measurements of such biomarkers as well.

According to certain aspects of the invention, the anti-interleukin-4-receptor (IL-4R) antibody is for use in methods which comprise: selecting a subject who exhibits a level of at least one AD-associated biomarker prior to or at the time of treatment which signifies the disease state administering to the subject a pharmaceutical composition comprising the anti-interleukin-4-receptor (IL-4R) antibody. In certain embodiments, the patient is selected by determining if the level of an AD-associated biomarker is elevated. The level of an AD-associated biomarker is determined or quantified by acquiring a sample from the patient for a biomarker assay known in the art. In certain other embodiments, a patient is selected by acquiring information relating to an elevated level of an AD-associated biomarker from the patient. In certain embodiments of this aspect of the invention, the subject is selected on the basis of an elevated level of IgE or TARC or periostin.

For purposes of the present invention, a normal IgE level in healthy subjects is less than about 114 kU/L (e.g., as measured using the ImmunoCAP^{®} assay [Phadia, Inc. Portage, Ml]). Thus, the anti-interleukin-4-receptor (IL-4R) antibody may be for use in methods that first comprise selecting a subject who exhibits a serum IgE level greater than about 114 kU/L, greater than about 150 kU/L, greater than about 500 kU/L, greater than about 1000 kU/L, greater than about 1500 kU/L, greater than about 2000 kU/L, greater than about 2500 kU/L, greater than about 3000 kU/L, greater than about 3500 kU/L, greater than about 4000 kU/L, greater than about 4500 kU/L, or greater than about 5000 kU/L, prior to administering to the subject a pharmaceutical composition comprising the IL-4R antagonist.

TARC levels in healthy subjects are in the range of 106 ng/L to 431 ng/L, with a mean of about 239 ng/L. (An exemplary assay system for measuring TARC level is the TARC quantitative ELISA kit offered as Cat. No. DDN00 by R&D Systems, Minneapolis, MN.) Thus, a subject may be selected who exhibits a serum TARC level greater than about 431 ng/L, greater than about 500 ng/L, greater than about 1000 ng/L, greater than about 1500 ng/L, greater than about 2000 ng/L, greater than about 2500 ng/L, greater than about 3000 ng/L, greater than about 3500 ng/L, greater than about 4000 ng/L, greater than about 4500 ng/L, or greater than about 5000 ng/L.

Another AD-associated biomarker is antigen-specific IgE. Phadiatop^{™} is a commercially available variant of serum specific or antigen-specific IgE assay test that was introduced for the screening of allergic sensitization (Merrett et al 1987, Allergy 17: 409-416). The test provides for simultaneous testing for serum specific IgE to a mixture of relevant allergens causing common inhalant allergies. The test gives a qualitative result, either positive or negative depending upon a fluorescence response obtained. When a patient sample gives a fluorescence response higher than or equal to the reference, a positive test result is indicated. A patient sample with a lower fluorescence response indicates a negative test result. The present invention includes the anti-interleukin-4-receptor (IL-4R) antibody for use in methods which comprise selecting a subject who exhibits a positive test result prior to administering to the subject the IL-4R antagonist.

Periostin is an extracellular matrix protein involved in the Th2-mediated inflammatory processes. Periostin levels are found to be up regulated in patients with AD (Masuoka et al 2012 J Clin Invest. 122(7):2590-2600. doi:10.1172/JCI58978). The present invention includes the anti-interleukin-4-receptor (IL-4R) antibody for use in methods to treat patients with elevated levels of periostin.

Lactate dehydrogenase (LDH) is used as a marker of tissue damage and is found to be elevated in patients with AD (Kou et al 2012; Arch. Dermatol. Res. 304: 305-312). The present invention includes the anti-interleukin-4-receptor (IL-4R) antibody for use in methods to treat patients with elevated levels of LDH.

According to other aspects of the invention the invention may result in a decrease in at least one AD-associated biomarker (*e.g*., IgE, TARC, eosinophils, eotaxin-3, antigen-specific IgE, LDH, etc.) at a time after administration of the pharmaceutical composition, as compared to the level of the biomarker in the subject prior to the administration.

As will be appreciated by a person of ordinary skill in the art, an increase or decrease in an AD-associated biomarker can be determined by comparing (i) the level of the biomarker measured in a subject at a defined time point after administration of the pharmaceutical composition comprising an IL-4R antagonist to (ii) the level of the biomarker measured in the patient prior to the administration of the pharmaceutical composition comprising an IL-4R antagonist (*i.e*., the "baseline measurement"). The defined time point at which the biomarker is measured can be, *e.g*., at about 4 hours, 8 hours, 12 hours, 1 day, 2 days, 3 days, 4 days, 5 days, 6 days, 7 days, 8 days, 9 days, 10 days, 15 days, 20 days, 35 days, 40 days, 50 days, 55 days, 60 days, 65 days, 70 days, 75 days, 80 days, 85 days, or more after administration of the of the pharmaceutical composition comprising an IL-4R antagonist.

According to certain particular embodiments of the present invention, a subject may exhibit a decrease in the level of one or more of TARC and/or IgE following administration of a pharmaceutical composition comprising an IL-4R antagonist (*e.g.*, an anti-IL-4R antibody). For example, at about day 4, day 8, day 15, day 22, day 25, day 29, day 36, day 43, day 50, day 57, day 64, day 71 or day 85, following administration of a first, second, third or fourth dose of a pharmaceutical composition comprising about 75, 150 or 300 mg of an anti-hIL-4R antibody (*e.g.*, mAb1), the subject, according to the present invention, may exhibit a decrease in TARC of about 1%, 2%, 5%, 10%, 15%, 20%, 25%, 30%, 35%, 40%, 45%, 50%, 55%, 60%, 65%, 70%, 75%, 80%, 85%, 90%, 95% or more from baseline (wherein "baseline" is defined as the level of TARC in the subject just prior to the first administration). Similarly, at about day 4, day 8, day 15, day 22, day 25, day 29, day 36, day 43, day 50, day 57, day 64, day 71 or day 85, following administration of a first, second, third or fourth dose of a pharmaceutical composition comprising about 75, 150 or 300 mg of an anti-hIL-4R antibody (*e.g.*, mAb1), the subject, according to the present invention, may exhibit a decrease in IgE of about 1%, 2%, 5%, 10%, 15%, 20%, 25%, 30%, 35%, 40%, 45%, 50%, 55%, 60%, 65%, 70%, 75%, 80%, 85%, 90%, 95% or more from baseline (wherein "baseline" is defined as the level of IgE in the subject just prior to the first administration).

Also disclosed but not claimed are methods for determining whether a subject is a suitable subject for whom administration of a pharmaceutical composition comprising an IL-4R antagonist would be beneficial. For example, if an individual, prior to receiving a pharmaceutical composition comprising an IL-4R antagonist, exhibits a level of an AD-associated biomarker which signifies the disease state, moderate-to-severe atopic dermatitis, the individual is therefore identified as a suitable patient for whom administration of a pharmaceutical composition of the invention (a composition comprising the anti-IL-4R antibody) would be beneficial. In a related embodiment, the subject with moderate-to-severe atopic dermatitis may be more susceptible to AD, for example, due to race or ethnicity. For example, African-American subjects who may be more susceptible to AD may be the subjects treated. Such a subject population may have an elevated level of an AD-associated biomarker.

According to certain exemplary embodiments, an individual may be identified as a good candidate for anti-IL-4R therapy if the individual exhibits one or more of the following: (i) an IgE level greater than about 114 kU/L, greater than about 150 kU/L, greater than about 500 kU/L, greater than about 1000 kU/L, greater than about 1500 kU/L, greater than about 2000 kU/L, greater than about 2500 kU/L, greater than about 3000 kU/L, greater than about 3500 kU/L, greater than about 4000 kU/L, greater than about 4500 kU/L, or greater than about 5000 kU/L; or (ii) a TARC level greater than about 431 ng/L, greater than about 500 ng/L, greater than about 1000 ng/L, greater than about 1500 ng/L, greater than about 2000 ng/L, greater than about 2500 ng/L, greater than about 3000 ng/L, greater than about 3500 ng/L, greater than about 4000 ng/L, greater than about 4500 ng/L, or greater than about 5000 ng/L; or (iii) a positive Phadiatop^{™} test. Additional criteria, such as other clinical indicators of AD (*e.g.*, an elevated IGA, BSA, EASI, SCORAD, 5-D Pruritus, and/or NRS score indicative of AD), may be used in combination with any of the foregoing AD-associated biomarkers to identify an individual as a suitable candidate for anti-IL-4R therapy as described elsewhere herein.

### Interleukin-4 Receptor Antagonists

The anti-interleukin-4-receptor (IL-4R) antibody for use according to the present invention comprises an HCVR having the amino acid sequence of SEQ ID NO: 162 and an LCVR having the amino acid sequence of SEQ ID NO: 164. Other IL-4R antagonists are also disclosed but not claimed as possible additional therapeutic agents.

As used herein, an "IL-4R antagonist" is any agent which binds to or interacts with IL-4R and inhibits the normal biological signaling function of IL-4R when IL-4R is expressed on a cell *in vitro* or *in vivo.* Non-limiting examples of categories of IL-4R antagonists include small molecule IL-4R antagonists, anti-IL-4R aptamers, peptide-based IL-4R antagonists (*e.g*., "peptibody" molecules), and antibodies or antigen-binding fragments of antibodies that specifically bind human IL-4R.

The terms "IL-4R," "hIL-4R," and the like, as used herein, are intended to refer to the alpha chain of the human cytokine receptor that specifically binds interleukin-4 (IL-4), IL-4Rα (SEQ ID NO:274). Unless specifically designated as being from a non-human species, the term "IL-4R", as used herein, shall be understood to mean the human interleukin-4 receptor alpha chain.

The term "antibody," as used herein, is intended to refer to immunoglobulin molecules comprising four polypeptide chains, two heavy (H) chains and two light (L) chains interconnected by disulfide bonds, as well as multimers thereof (*e.g*., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (Ci_1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-IL-4R antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, *e.g.*, commercial sources, DNA libraries (including, *e.g*., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g*., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e.g*. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain instances, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g*., 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (*e.g*., by disulfide bond(s)).

As with full antibody molecules, antigen-binding fragments may be monospecific or multispecific (*e.g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format may be adapted for use in the context of an antigen-binding fragment of an antibody of the present invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody," as used herein, is intended to include antibodies having variable and constant regions derived from human germline immunoglobulin sequences. The human antibodies of the invention may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody," as used herein, is not intended to include antibodies in which CDR sequences derived from the germline of another mammalian species, such as a mouse, have been grafted onto human framework sequences.

The term "recombinant human antibody," as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. Also disclosed but not claimed are antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" IL-4R, as used in the context of the present invention, includes antibodies that bind IL-4R or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human IL-4R may, however, have cross-reactivity to other antigens, such as IL-4R molecules from other (non-human) species.

Anti-IL-4R antibodies may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. Also disclosed but not claimed are methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain embodiments, all of the framework and/or CDR residues within the V_{H} and/or V_{L} domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (*i.e*., a germline sequence that is different from the germline sequence from which the antibody was originally derived). Furthermore, the antibodies of the present invention may contain any combination of two or more germline mutations within the framework and/or CDR regions, *e.g*., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc.

Also disclosed are variants of any of the HCVR, LCVR, and/or CDR amino acid sequences disclosed but not claimed herein having one or more conservative substitutions. For example, also disclosed are anti-IL-4R antibodies having HCVR, LCVR, and/or CDR amino acid sequences with, *e.g*., 10 or fewer, 8 or fewer, 6 or fewer, 4 or fewer, etc. conservative amino acid substitutions relative to any of the HCVR, LCVR, and/or CDR amino acid sequences disclosed herein but not claimed.

The term "surface plasmon resonance," as used herein, refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "K_{D}," as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

### Preparation of Human Antibodies

Methods for generating human antibodies in transgenic mice are known in the art. Any such known methods can be used to make human antibodies that specifically bind to human IL-4R.

Using VELOCIMMUNE^{™} technology (see, for example, US 6,596,541, Regeneron Pharmaceuticals) or any other known method for generating monoclonal antibodies, high affinity chimeric antibodies to IL-4R are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods disclosed herein possess high affinities, as described above but not claimed, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind IL-4R which can be used include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs: 2, 18, 22, 26, 42, 46, 50, 66, 70, 74, 90, 94, 98, 114, 118, 122, 138, 142, 146, 162, 166, 170, 186, 190, 194, 210, 214, 218, 234, 238, 242, 258 and 262. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs: 10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260 and 264. Methods and techniques for identifying CDRs within HCVR and LCVR amino acid sequences are well known in the art and can be used to identify CDRs within the specified HCVR and/or LCVR amino acid sequences disclosed herein. Exemplary conventions that can be used to identify the boundaries of CDRs include, *e.g*., the Kabat definition, the Chothia definition, and the AbM definition. In general terms, the Kabat definition is based on sequence variability, the Chothia definition is based on the location of the structural loop regions, and the AbM definition is a compromise between the Kabat and Chothia approaches. *See, e.g.,* Kabat, "Sequences of Proteins of Immunological Interest," National Institutes of Health, Bethesda, Md. (1991); Al-Lazikani et al., J. Mol. Biol. 273:927-948 (1997); and Martin et al., Proc. Natl. Acad. Sci. USA 86:9268-9272 (1989). Public databases are also available for identifying CDR sequences within an antibody.

In certain instances, the antibody or antigen-binding fragment thereof comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260 and 262/264.

In certain instances, the antibody or antigen-binding fragment thereof comprises six CDRs (HCDR1/HCDR2/HCDR3/LCDR1/LCDR2/LCDR3) having the amino acid sequences selected from the group consisting of SEQ ID NOs: 4/6/8/12/14/16; 28/30/32/36/38/40; 52/54/56/60/62/64; 76/78/80/84/86/88; 100/102/104/108/110/112; 124/126/128/132/134/136; 148/150/152/156/158/160; 172/174/176/180/182/184; 196/198/200/204/206/208; 220/222/224/228/230/232; and 244/246/248/252/254/256.

In certain instances, the antibody or antigen-binding fragment thereof comprises HCVR/LCVR amino acid sequence pairs selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260 and 262/264.

### Pharmaceutical Compositions

The anti-interleukin-4-receptor (IL-4R) antibody may be contained within a pharmaceutical composition. The pharmaceutical compositions of the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Interspecies scaling of dosages can be performed using well-known methods in the art (*e.g.,* Mordenti et al., 1991, Pharmaceut. Res. 8:1351). Specific exemplary doses of anti-IL4R antibodies, and administration regimens involving the same, that can be used in the context of the present invention are disclosed elsewhere herein.

Various delivery systems are known and can be used to administer the pharmaceutical composition of the invention, *e.g*., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (sanofi-aventis, Frankfurt, Germany), to name only a few. Examples of disposable pen delivery devices having applications in subcutaneous delivery of a pharmaceutical composition of the present invention include, but are not limited to the SOLOSTAR^{™} pen (sanofi-aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e.g*., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g*., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared can be filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

Exemplary pharmaceutical compositions comprising an anti-IL-4R antibody that can be used in the context of the present invention are disclosed, *e.g*., in US Patent Application Publication No. 2012/0097565.

### Dosage

The present invention provides an anti-interleukin-4-receptor (IL-4R) antibody for use in a method of treating moderate-to-severe atopic dermatitis (AD) in a patient, the method comprising administering an initial dose of the antibody followed by at least eight secondary doses of the antibody, wherein:
- the initial dose is a loading dose of 300mg or 600mg, followed by the secondary doses which are maintenance doses of 300mg;
- each secondary dose is administered to the patient 1 to 2 weeks after the immediately preceding dose; and
- the antibody comprises an HCVR having the amino acid sequence of SEQ ID NO: 162 and an LCVR having the amino acid sequence of SEQ ID NO: 164.

The amount of IL-4R antagonist contained within the individual doses may be expressed in terms of milligrams of antibody per kilogram of patient body weight (*i.e*., mg/kg).

### Combination Therapies

According to certain embodiments the anti-interleukin-4-receptor (IL-4R) antibody is for use in methods that comprise administering to the subject one or more additional therapeutic agents in combination with the IL-4R antagonist. As used herein, the expression "in combination with" means that the additional therapeutic agents are administered before, after, or concurrent with the pharmaceutical composition comprising the IL-4R antagonist. The term "in combination with" also includes sequential or concomitant administration of IL-4R antagonist and a second therapeutic agent.

For example, when administered "before" the pharmaceutical composition comprising the IL-4R antagonist, the additional therapeutic agent may be administered about 72 hours, about 60 hours, about 48 hours, about 36 hours, about 24 hours, about 12 hours, about 10 hours, about 8 hours, about 6 hours, about 4 hours, about 2 hours, about 1 hour, about 30 minutes, about 15 minutes or about 10 minutes prior to the administration of the pharmaceutical composition comprising the IL-4R antagonist. When administered "after" the pharmaceutical composition comprising the IL-4R antagonist, the additional therapeutic agent may be administered about 10 minutes, about 15 minutes, about 30 minutes, about 1 hour, about 2 hours, about 4 hours, about 6 hours, about 8 hours, about 10 hours, about 12 hours, about 24 hours, about 36 hours, about 48 hours, about 60 hours or about 72 hours after the administration of the pharmaceutical composition comprising the IL-4R antagonist. Administration "concurrent" or with the pharmaceutical composition comprising the IL-4R antagonist means that the additional therapeutic agent is administered to the subject in a separate dosage form within less than 5 minutes (before, after, or at the same time) of administration of the pharmaceutical composition comprising the IL-4R antagonist, or administered to the subject as a single combined dosage formulation comprising both the additional therapeutic agent and the IL-4R antagonist.

The additional therapeutic agent may be, *e.g*., another IL-4R antagonist, an IL-1 antagonist (including, *e.g*., an IL-1 antagonist as set forth in US 6,927,044), an IL-6 antagonist, an IL-6R antagonist (including, *e.g*., an anti-IL-6R antibody as set forth in US 7,582,298), an IL-13 antagonist, a TNF antagonist, an IL-8 antagonist, an IL-9 antagonist, an IL-17 antagonist, an IL-5 antagonist, an IgE antagonist, a CD48 antagonist, an IL-31 antagonist (including, e.g., as set forth in US7,531 ,637), a thymic stromal lymphopoietin (TSLP) antagonist (including, e.g., as set forth in US 2011/027468), interferon-gamma (IFNγ) antibiotics, topical corticosteroids, tacrolimus, pimecrolimus, cyclosporine, azathioprine, methotrexate, cromolyn sodium, proteinase inhibitors, or combinations thereof. In certain embodiments, the pharmaceutical composition comprising an anti-IL4R antagonist is administered to a subject in conjunction with a non-pharmaceutical therapy such as ultraviolet (UV) light therapy.

The anti-interleukin-4-receptor (IL-4R) antibody may be for use in methods that comprise administering the IL-4R antagonist in combination with a second therapeutic agent for additive or synergistic activity to treat AD. Certain embodiments of the invention include anti-interleukin-4-receptor (IL-4R) antibody for use in methods to treat moderate-to-severe AD by administering the IL-4R antagonist concomitantly with a TCS. The TCS may be a potent TCS such as a Group III TCS. Examples of Group II TCS include methylprednisolone aceponate, mometasone furoate, fluticasone propionate and betamethasone valerate. In some embodiments, the TCS may be a moderate TCS such as Group II TCS or a weak TCS such as Group I TCS.

### Administration Regimens

The anti-interleukin-4-receptor (IL-4R) antibody for use in a method of treating moderate-to-severe atopic dermatitis (AD) in a patient, is one wherein the method comprises
- administering an initial dose of the antibody followed by at least eight secondary doses of the antibody, wherein:
- the initial dose is a loading dose of 300mg or 600mg, followed by the secondary doses which are maintenance doses of 300mg;
- each secondary dose is administered to the patient 1 to 2 weeks after the immediately preceding dose; and
- the antibody comprises an HCVR having the amino acid sequence of SEQ ID NO: 162 and an LCVR having the amino acid sequence of SEQ ID NO: 164.

The anti-interleukin-4-receptor (IL-4R) antibody for use of present invention includes use in methods which comprise sequentially administering to the patient a single initial dose of an IL-4R antagonist, followed at least eight secondary doses of the IL-4R antagonist, and optionally followed by one or more tertiary doses of the IL-4R antagonist.

The terms "initial dose," "secondary doses," and "tertiary doses," refer to the temporal sequence of administration of the IL-4R antagonist. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of IL-4R antagonist, but generally may differ from one another in terms of frequency of administration. In one exemplary embodiment of the present invention, each tertiary dose is administered 1 to 14 (*e.g*., 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, or more) weeks after the immediately preceding dose. The phrase "the immediately preceding dose," as used herein, means, in a sequence of multiple administrations, the dose of IL-4R antagonist which is administered to a patient prior to the administration of the very next dose in the sequence with no intervening doses.

The anti-interleukin-4-receptor (IL-4R) antibody for use according to this aspect of the invention may comprise administering to a patient any number of tertiary doses of an IL-4R antagonist. In certain embodiments, only a single tertiary dose is administered to the patient. In other embodiments, two or more (*e.g*., 2, 3, 4, 5, 6, 7, 8, or more) tertiary doses are administered to the patient.

In embodiments involving multiple tertiary doses, each tertiary dose may be administered at the same frequency as the other tertiary doses. For example, each tertiary dose may be administered to the patient 2 to 4 weeks after the immediately preceding dose. Alternatively, the frequency at which the tertiary doses are administered to a patient can vary over the course of the treatment regimen. The frequency of administration may also be adjusted during the course of treatment by a physician depending on the needs of the individual patient following clinical examination.

The present invention includes anti-interleukin-4-receptor (IL-4R) antibody for use in methods comprising sequential administration of the IL-4R antagonist and a second therapeutic agent, to a patient to treat AD. In some embodiments, the present methods comprise administering the IL-4R antagonist followed by one or more doses of a second therapeutic agent. For example, after doses of the IL-4R antagonist one or more doses of a second therapeutic agent (e.g., a topical corticosteroid or a calcineurin inhibitor or any other therapeutic agent, as described elsewhere herein) may be administered to treat, alleviate, reduce or ameliorate one or more symptoms of AD. In some embodiments, the IL-4R antagonist is administered at one or more doses resulting in an improvement in one or more AD-associated parameters followed by the administration of a second therapeutic agent to prevent recurrence of at least one symptom of AD. Alternative embodiments of the invention pertain to concomitant administration of the IL-4R antagonist and a second therapeutic agent. For example, one or more doses of the IL-4R antagonist are administered and a second therapeutic agent is administered at a separate dosage at a similar or different frequency relative to the IL-4R antagonist. In some embodiments, the second therapeutic agent is administered before, after or concurrently with the IL-4R antagonist.

### IL-4R Antagonists as Vaccine Adjuvants

Also disclosed are compositions and methods that are useful in vaccine applications. For example, an IL-4R antagonist (*e.g*., an anti-IL-4R antibody disclosed herein) may be administered to a subject in conjunction with a vaccine to improve or potentiate the immune response (including humoral and cellular immune responses) elicited by the vaccine, *i.e.,* as a vaccine adjuvant. In certain instances, an IL-4R antagonist is administered just prior to, concurrent with, and/or subsequent to administration of a vaccine composition to a subject. For example, also disclosed are methods of eliciting or enhancing an immune response to an antigen in a subject by first administering to the subject a pharmaceutical composition comprising an IL-4R antagonist, followed by administering to the subject a vaccine composition comprising the antigen (by itself or in combination with the IL-4R antagonist), and optionally administering additional doses of the IL-4R antagonist for a period of time following administration of the vaccine antigen to the subject.

The IL-4R antagonists may be administered as adjuvants with any type of vaccine including, e.g., live vaccines, live/attenuated vaccines, killed vaccines, subunit vaccines, DNA vaccines, and cancer immunotherapeutic vaccines. The vaccines that may be used in connection with the IL-4R antagonist include vaccines against bacterial pathogens, viruses, parasites, and other infectious agents. Non-limiting examples of infectious agents and diseases against which the vaccine compositions and methods of the invention may be targeted include, *e.g*., HIV, HCV, RSV, Neisseria meningitides, streptococcus, tuberculosis, malaria, smallpox, diphtheria, pertussis, tetanus, polio, measles, rubella, mumps, influenza, Anthrax, SARS, Ebola virus, Hanta virus, Dengue virus, etc.

Also disclosed but not claimed are pharmaceutical compositions comprising an IL-4R antagonist and one or more vaccine antigen. The pharmaceutical compositions may comprise one or more additional immune potentiators such as MPL, MDP, CpG oligonucleotides, lipopeptides, saponins, dsRNA, small molecule immune potentiators, etc.

Besides IL-4R antagonists, other inhibitors of the IL-4/IL-13 signaling pathway (*e.g*., anti-IL-4 antibodies, anti-IL-13 antibodies, bispecific anti-IL-4/anti-IL-13 antibodies, etc.) may be used in the context of vaccine methods and compositions as disclosed herein.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human IL-4R

Human anti-hlL-4R antibodies were generated as described in US Patent No. 7,608,693. Table 1 sets forth the sequence identifiers for the heavy and light chain variable region amino acid sequence pairs, and CDR amino acid sequences, of selected anti-IL-4R antibodies and their corresponding antibody designations.

**Table 1**

| | **SEQ ID NOs:** | | | | | | | |
|---|---|---|---|---|---|---|---|---|
| **Antibody Designation** | **HCVR** | **HCDR1** | **HCDR2** | **HCDR3** | **LCVR** | **LCDR1** | **LCDR2** | **LCDR3** |
| H1H095-a | 2 | 4 | 6 | 8 | 10 | 12 | 14 | 16 |
| H1H095-b | 18 | 4 | 6 | 8 | 20 | 12 | 14 | 16 |
| H1H095-c | 22 | 4 | 6 | 8 | 24 | 12 | 14 | 16 |
| H1H097-a | 26 | 28 | 30 | 32 | 34 | 36 | 38 | 40 |
| H1H097-b | 42 | 28 | 30 | 32 | 44 | 36 | 38 | 40 |
| H1 H097-c | 46 | 28 | 30 | 32 | 48 | 36 | 38 | 40 |
| H1H093-a | 50 | 52 | 54 | 56 | 58 | 60 | 62 | 64 |
| H1H093-b | 66 | 52 | 54 | 56 | 68 | 60 | 62 | 64 |
| H1H093-c | 70 | 52 | 54 | 56 | 72 | 60 | 62 | 64 |
| H1H093-d | 74 | 76 | 78 | 80 | 82 | 84 | 86 | 88 |
| H1H093-e | 90 | 76 | 78 | 80 | 92 | 84 | 86 | 88 |
| H1H093-f | 94 | 76 | 78 | 80 | 96 | 84 | 86 | 88 |
| H1H094-a | 98 | 100 | 102 | 104 | 106 | 108 | 110 | 112 |
| H1H094-b | 114 | 100 | 102 | 104 | 116 | 108 | 110 | 112 |
| H1H094-c | 118 | 100 | 102 | 104 | 120 | 108 | 110 | 112 |
| H1H096-a | 122 | 124 | 126 | 128 | 130 | 132 | 134 | 136 |
| H1H096-b | 138 | 124 | 126 | 128 | 140 | 132 | 134 | 136 |
| H1H096-c | 142 | 124 | 126 | 128 | 144 | 132 | 134 | 136 |
| H1H098-a | 146 | 148 | 150 | 152 | 154 | 156 | 158 | 160 |
| H1H098-b | 162 | 148 | 150 | 152 | 164 | 156 | 158 | 160 |
| H1H098-c | 166 | 148 | 150 | 152 | 168 | 156 | 158 | 160 |
| H1H099-a | 170 | 172 | 174 | 176 | 178 | 180 | 182 | 184 |
| H1H099-b | 186 | 172 | 174 | 176 | 188 | 180 | 182 | 184 |
| H1H099-c | 190 | 172 | 174 | 176 | 192 | 180 | 182 | 184 |
| H4H083-a | 194 | 196 | 198 | 200 | 202 | 204 | 206 | 208 |
| H4H083-b | 210 | 196 | 198 | 200 | 212 | 204 | 206 | 208 |
| H4H083-c | 214 | 196 | 198 | 200 | 216 | 204 | 206 | 208 |
| H4H121-a | 218 | 220 | 222 | 224 | 226 | 228 | 230 | 232 |
| H4H121-b | 234 | 220 | 222 | 224 | 236 | 228 | 230 | 232 |
| H4H121-c | 238 | 220 | 222 | 224 | 240 | 228 | 230 | 232 |
| H4H118-a | 242 | 244 | 246 | 248 | 250 | 252 | 254 | 256 |
| H4H118-b | 258 | 244 | 246 | 248 | 260 | 252 | 254 | 256 |
| H4H118-c | 262 | 244 | 246 | 248 | 264 | 252 | 254 | 256 |

The exemplary IL-4R antagonist used in the following Examples is the human anti-IL-4R antibody designated in Table 1 as H1H098-b (also referred to herein as "mAb1").

### Example 2: Single Ascending Dose Clinical Trial of Intravenously and Subcutaneously Administered anti-IL-4R Antibody (mAb1) In Healthy Subjects

### A. Study Design

This study was a randomized, double-blind, placebo-controlled, sequential, single ascending-dose study of intravenous (IV) and subcutaneous (SC) administered mAb1 in healthy subjects. The main purpose of this study was to evaluate the safety and tolerability of intravenously and subcutaneously administered mAb1 in healthy subjects.

Screening occurred from day -21 to day -3. On day 1 (baseline), subjects were randomized to receive either IV or SC study drug (mAb1 or placebo) infused over a 2-hour period. Subjects returned on days 4, 8, 11, 15, 22, 29, 43, 57 and 85 (end-of-study) for safety assessments and blood sampling for clinical laboratory testing.

Forty-eight total subjects participated in the study. Four sequential ascending dose cohorts (1.0, 3.0, 8.0, and 12.0 mg/kg) were planned for IV dosing and 2 sequential ascending dose cohorts (150 and 300 mg) were planned for SC dosing. Each dose cohort consisted of 8 subjects (if there was no cohort expansion): 6 randomized to receive mAb1 and 2 randomized to receive placebo. In order to optimize safety, the first 3 subjects in IV cohort 1 (1.0 mg/kg) were dosed at least 24 hours apart and the remaining 5 subjects were dosed 5 to 7 days later. In subsequent IV cohorts, 3 of the 8 subjects were dosed on day 1 and the remaining 5 subjects were dosed 5 to 7 days later. All 8 subjects in SC dose cohort 1 (150 mg) were dosed on the same day, and all 8 subjects in the subsequent SC cohort (300 mg) were dosed on the same day. The SC cohorts were administered after the IV cohorts were completed.

Inclusion criteria for the study were as follows: (1) Male or female 18 to 65 years of age; (2) Weight > 50 kg and < 120 kg; (3) For women of childbearing potential, a negative serum pregnancy test at the screening visit (visit 1) and a negative urine pregnancy test on day -1; (4) Willingness to refrain from the consumption of more than 2 standard alcoholic drinks in any 24-hour period during the duration of the study. A standard alcoholic drink was considered to be the equivalent of 12 ounces of beer, 5 ounces of wine, or 1.5 ounces of hard liquor; (5) Willingness to refrain from the consumption of alcohol for 24 hours prior to each study visit; (6) For men and women of childbearing potential, willingness to utilize adequate contraception and not become pregnant (or have their partner[s] become pregnant) during the full duration of the study. Adequate contraceptive measures include intrauterine device (IUD); bilateral tubal ligation; vasectomy; condom or diaphragm plus either contraceptive sponge, foam or jelly; and (7) Willingness, commitment, and ability to return for all clinic visits and complete all study-related procedures.

Exclusion criteria for the study were as follows: (1) Onset of a new exercise routine or major change to a previous exercise routine within 4 weeks prior to screening (visit 1). Subjects had to be willing to maintain a similar level of exercise for the duration of the study and to refrain from unusually strenuous exercise for the duration of the trial; (2) Pregnant or breast-feeding women; (3) Significant concomitant illness or history of significant illness such as cardiac, renal, neurological, endocrinological, metabolic or lymphatic disease, or any other illness or condition that would have adversely affected the subject's participation in this study; (4) Any clinically significant abnormalities observed during the screening visit; (5) Hospitalization for any reason within 60 days of screening (visit 1); (6) Known history of human immunodeficiency virus (HIV), hepatitis B or hepatitis C, and/or positive hepatitis B surface antigen, positive hepatitis C antibody or positive HIV serology at the screening visit; (7) History of or positive drug screen for drug or alcohol abuse within a year prior to the screening visit; (8) History of a hypersensitivity to doxycycline or similar compound; (9) Participation in any clinical research study evaluating another investigational drug or therapy within 30 days or at least 5 half-lives (whichever was longer), of the investigational drug prior to the screening visit; (10) Previous exposure to any therapeutic or investigational biological agent; (11) Any medical or psychiatric condition which, in the opinion of the investigator, would have placed the subject at risk; interfered with participation in the study or interfered with the interpretation of study results; (12) Subjects with a positive QuantiFERON tuberculosis (TB) test; (13) History of a parasitic infection or recent (within the previous 6 months) travel to a parasitic endemic area; (14) History of alcohol or substance abuse within previous 5 years; (15) Positive urine drug screen result at screening (visit 1) or baseline (visit 2); and/or (16) Live/attenuated vaccinations within 12 weeks of screening or during the study.

### B. Investigational Treatment

mAb1 drug product was supplied as a lyophilized powder in a 20 ml glass vial for either IV or SC administration. When delivered IV, mAb1 drug product was reconstituted in a single use vial with 7.8 ml of sterile water for injection yielding a solution containing 50 mg/mL of mAb1. The pharmacist or designee withdrew the required amount of reconstituted mAb1 (dependent upon the subject's dose and weight) or placebo, and injected it into an infusion bag with 0.9% saline for IV delivery. The infusion was given over a 2-hour period.

When delivered SC, the mAb1 drug product was reconstituted with 2.3 ml of sterile water for injection, yielding a solution containing 150 mg/mL of mAb1. The pharmacist or designee administered the injections in the abdomen; administration to the extremities was not allowed due to the possibility of different absorption and bioavailability. If administration of multiple injections were required on the same day, each injection was delivered at a different injection site.

The dose levels of mAb1 tested were: 1.0, 3.0, 8.0, and 12.0 mg/kg for IV administration, and 150 and 300 mg for SC administration.

Placebo matching mAb1 was prepared in the same formulation as mAb1, but without addition of antibody.

### C. Results and Conclusions

mAb1 was generally well-tolerated with a favorable safety profile. The overall adverse event (AE) profile was characteristic of a healthy population. Less than 55% of subjects treated with mAb1 (19/36) experienced 1 or more treatment-emergent adverse event (TEAE) as compared to less than 59% for the subjects treated with placebo (7/12). The most frequently reported TEAEs were: Blood creatine phosphokinase (CPK) Increased, Blood Pressure Increased, Nasopharyngitis, and Toothache. Most subjects experienced an intensity of TEAEs as mild or moderate; only 3 subjects reported TEAEs that were considered severe. Only 1 severe TEAE (Blood CPK Increased) was considered by the investigator to be related to treatment. One serious adverse event (SAE) was reported during the study, which was considered by the investigator to be unrelated to the study drug. No subjects were withdrawn from the study due to an AE and no deaths were reported. No other clinically significant laboratory test results (blood chemistry, hematology, or urinalysis) were reported during the study. No trends were seen in mean/median baseline in any laboratory parameter. There were no significant trends in mean or median changes from baseline in temperature or pulse throughout the study. No clinically significant abnormalities were seen on physical examination results, ECGs or vital signs.

This study was significant in that the subject population consisted of a high proportion of Black/African-American subjects (Table 2).

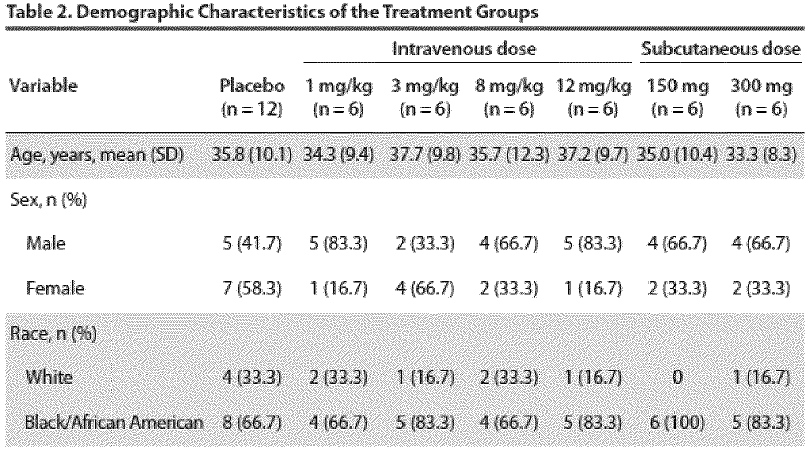

Although the subjects were healthy volunteers, African-Americans as a group may be more susceptible to atopic diseases (Caggana et al 1999; Genet. Med. 1: 267-271), and this population may therefore be considered appropriate for evaluation of proof of mechanism based on the exploratory biomarker analysis.

With regard to pharmacokinetic (PK) analysis, nonlinear kinetics were observed. The target-mediated pathway of elimination appeared saturated at IV doses of 8 and 12 mg/kg, when concentrations of functional mAb1 were above about 30 mg/L. Low anti-drug antibody (ADA) titers were observed in 9 subjects. No sudden and persistent drop in concentrations of functional mAB1 was observed indicating that the ADAs did not have a major impact on PK.

### Example 3: Clinical Trial of Two Different Drug Products of Anti-IL-4R Antibody (mAb1) Following Subcutaneous Administration of Anti-IL-4R Antibody (mAb1) In Healthy Patients

### A. Study Design

This study was a single-center, single-dose, double-blind, randomized, no placebo-controlled study to assess the safety and pharmacokinetic profile of subcutaneous administration of two different anti-IL-4R mAb (mAb1) drug products generated from different cell lines and manufacturing processes. The drug products were provided in 150 mg/mL 2 mL doses, and 300 mg (2mL) were administered subcutaneously to 30 healthy adults in two parallel groups (15 subjects per group). Subjects included 30 subjects represented by 22 males (73.3%) and 8 females (26.7%) aged 19 to 45 years old, with weights ranging from 54.8 to 94.3 kg.

Serum concentration of mAb1 was used to determine the following PK parameters: maximum serum concentration (Cₘₐₓ), area under the [serum concentration versus time] curve from time 0 to the real time corresponding to the last concentration above the lower limit of quantification (tₗₐₛₜ (AUCₗₐₛₜ), and area under the serum concentration versus time curve from time zero extrapolated to infinity (AUC). Also measured with the time to reach maximum concentration (tₘₐₓ) and terminal half-life (t_{1/2z}).

### B. Criteria for evaluation and methods

Safety was assessed by measuring adverse events, including treatment-emergent adverse events (TEAEs) up to two months postdose, clinical laboratory evaluations (biochemistry, hematology, urinalysis), vital signs, electrocardiograms (ECGs) with automatic reading, anti-mAb1 antibodies (negative or titer), and local tolerability assessments (including injection site pain using a Visual Analog Scale [VAS; 100 mm ungraduated line], Erythema [diameter in mm at injection site], and edema [diameter in mm at injection site]).

Adverse events of special interest (AESI) were AEs (serious or nonserious) of scientific and medical concern that needed specific monitoring, documentation, and management as described in the protocol. The following AEs were defined as AESI:
hypersensitivity/anaphylaxis: anaphylactic reaction or acute allergic reaction requiring immediate treatment, severe injection site reaction lasting longer than 24 hours, severe infection, any parasitic infection, alanine aminotransferase (ALT) increase ≥2 ULN, QTc ≥500 ms, pregnancy, or overdose.

Blood samples for hematology and biochemistry evaluations were collected predose on Day-1 and on Days 2 (i.e., 24 hours postdose) and 57, and biochemistry limited to liver function on Days 8, 15, 22, 29, 36, 43, and 50.

Blood samples for the determination of anti-mAb1 antibodies in serum were collected on Day 1 and on Days 15, 29 and 57.

Local tolerability assessments were performed at predose on Day 1 and at 2 minutes, 2 hours, 6 hours and 12 hours postdose and on Days 2 (i.e., 24 hours postdose), 3, 4 and 8 following mAb1 administration.

For pharmacokinetic and pharmacogenetic sampling, blood samples were collected at predose on Day 1 and 12 hours post-dose, and on Days 2, 3, 4, 8, 11, 15, 22, 29, 36, 43, 50, and 57 following mAb1 administration. Serum concentrations of functional mAb1 were determined using a validated enzyme-linked immunosorbent assay (ELISA) with a lower limit of quantification (LLOQ) of 78 ng/mL.

Samples were collected at baseline (Day 1 predose) for optional pharmacogenetic analyses.

Pharmacokinetic parameters of serum functional mAb1 were summarized by treatment group using descriptive statistics (mean, geometric mean, median, standard deviation (SD), coefficient of variation [CV], minimum, and maximum). For log transformed Cₘₐₓ, AUCₗₐₛₜ, and AUC, the test/reference treatment ratios were assessed using a linear fixed effects model with gender and treatment as fixed effects, and with weight as covariate. Estimates and 90% confidence intervals (Cls) for treatment ratios were provided for Cₘₐₓ, AUCₗₐₛₜ, and AUC.

Evaluation of safety was based on the review of individual values and descriptive statistics. All AEs were coded using Medical Dictionary for Regulatory Activities (MedDRA) version 15.0, and frequencies of treatment emergent adverse events (TEAEs) were classified and tabulated (counts and percents) by primary system organ class, preferred term, and treatment group. Potentially clinically significant abnormalities (PCSAs; definitions according to version 2.0 dated 14 September 2009) for clinical laboratory data, vital signs, and ECG values and out of normal range values for clinical laboratory data were flagged and summarized by treatment group.

Anti-mAb1 antibody results were listed as either negative or with a titer value if positive in the confirmation assay by treatment group, subject and visit. Data were summarized as number of subjects (counts and percent) with negative or positive anti-drug antibody (ADA) response by treatment group.

Descriptive statistics (mean, SD, minimum, median, and maximum) of the pain VAS, erythema diameter, and edema diameter were provided by treatment group for each scheduled time point. Each of these measurements was further summarized by treatment group as time-averaged (from study drug administration to Day 8 assessment included) and peak values (using post-dose assessments).

### C. Pharmacokinetic results

**Table 3. Mean ± SD (geometric mean) [CV%] of serum functional mAb1 PK parameters**

| **PK Parameters** | **Serum mAb1** | |
|---|---|---|
| | **Drug product A** | **Drug Product B** |
| N | 15 | 13 |
| Cₘₐₓ (ng/mL) | 28900 ± 9110 (27300) [31.6] | 27200 ± 9950 (25300) [36.6] |
| tₘₐₓ^{a} (h) | 168.00 (72.00 - 240.00) | 168.00 (48.00 - 240.00) |
| AUCₗₐₛₜ (ng•h/mL) | 11700000 ± 4790000 (10500000) [41.0] | 12000000 ± 4300000 (11200000) [35.7]^{c} |
| AUC (ng•h/mL) | 13300000 ± 3910000 (12800000) [29.4]^{b} | 12500000 ± 4770000 (11600000) [38.2]^{c} |
| t_{1/2}^{z} (h) | 137 ± 46.3 (129) [33.8]^{b} | 131 ± 47.4 (124) [36.2]^{c} |

| | | |
|---|---|---|
| ^{a} Median(Min - Max), ^{b} N=13 since terminal log-linear phase could not be determined in 2 subjects, ^{c} N = 11 since 2 subjects discontinued from the study. | | |

**Table 4. Point estimates of treatment ratios with 90% confidence interval**

| **Comparison** | **Parameter** | **Estimate** | **90% CI** |
|---|---|---|---|
| Drug Product A vs. Drug Product B | Cₘₐₓ | 1.10 | (0.89 to 1.35) |
| | AUCₗₐₛₜ | 0.90 | (0.71 to 1.16) |
| | AUC | 1.05 | (0.86 to 1.29) |

| | | | |
|---|---|---|---|
| Drug Product A: Group A (test), 150 mg/mL (Drug Product 1) × 2.0 mL (300 mg of mAb1); Drug Product B: Group B (reference), 150 mg/mL (Drug Product 2) × 2.0 mL (300 mg of mAb1). | | | |

The estimates are based on the linear fixed effect model with fixed terms for gender, weight, and treatment.

### D. Safety results

Twelve out of 15 subjects (80.0%) on Drug Product A (test drug product) versus 8 out of 15 subjects (53.3%) on Drug Product B (reference drug product) had TEAEs. The apparent treatment imbalance appeared in some primary system organ classes only and appeared to be due to events not related to the IMP as often another cause was identified. Four SAEs were reported in two subjects during the study.

A 23 years old male subject in the Drug Product 1 treatment group experienced a "herpes simplex type II viral infection" with symptoms of blurred vision, diaphoresis, fever, and headache starting 4 days after dosing, followed by a swollen tongue (6 days after dosing) and cough, chest congestion, and muscle cramps in both calves (7 days after dosing). During the course of this event, the subject went to the emergency room on several occasions and received multiple treatments including Solumedrol^{®} (3 IV doses), prednisone (for 9 days), Rocephin^{®} (1 IV dose), Zithromax^{®} (for 5 days). It is noteworthy that the subject had a tongue barbell piercing placed 3 months before dosing. All symptoms had resolved 19 days after dosing. Initial Herpes Simplex Virus (HSV) II Ig G titers performed 10 days after dosing were negative which converted to positive when re-assessed 7 weeks after dosing. This SAE was judged as related to the IMP by the Investigator and the company. More than 4 weeks after dosing, the subject was diagnosed with "Bell's palsy" on the left side that was considered by the Investigator to be consequent to the HSV II infection. This event was treated with prednisone (for 6 days) and acyclovir (for 10 days). This SAE was deemed to be not related to the IMP by the Investigator due to the multiple and repeated steroids administrations in the acute state of the HSV II infection, which were considered an alternative explanation. The company considered that a causal relationship to the IMP could not be excluded. Both events were recovering at the end of the study. This subject did not develop ADA at any time during the study

A 22 years old male subject in the Drug Product A treatment group experienced "elevated ALT" (ALT up to 11.4 ULN) together with "rhabdomyolysis" (creatine phosphokinase up to 392 ULN) both discovered by routine laboratory exams 7 weeks after dosing. These events followed a physical challenge (consisting of swimming, push-ups, pull-ups, sit-ups and other endurance type exercises; subject is a life guard) and an injury to his triceps muscle (NSAE). The subject was admitted to the hospital for hydration. Liver causes of the increased liver function tests were ruled out, and the elevated ALT (as well as the aspartate aminotransferase [AST] elevation, up to 50.5 ULN) was judged as related to the rhabdomyolysis. Creatinine and glomerular function remained within normal ranges during the course of the events. Both events resolved within 3 weeks. These 2 SAEs were deemed to be not related to the IMP by the Investigator. This subject also developed ADA with positive titers detected on Day 29 (titer value = 120) and at EOSV on Day 58 (titer value = 30).

Apart from the ALT increase, no other AESI were observed during the study.

Four (4) subjects experienced infection during the study. Apart from the herpes simplex infection described above, cases of mild upper respiratory tract infection, pharyngitis, and sinusitis (1 case each) were observed 54, 7 and 1 day after dosing, respectively. These 3 latter events were observed in subjects treated with C1P2.

Fifteen (15) cases of injection site reaction occurred in 12 subjects: erythema (8 cases in 8 subjects, 4 in each group), pain (3 cases, 2 on C2P1 and 1 on C1P2), nodule (2 cases, 1 in each group), haematoma (1 case on C1P2), and pruritus (1 case on C2P1). All were mild and resolved within 24 hours of injection.

Other TEAEs were not observed in more than 1 subject in each treatment group, except for 2 cases of pruritus (not at injection site) in 2 subjects treated with C2P1 and 3 cases of headache (in 1 subject treated with C2P1 and 2 subjects treated with C1P2).

Apart from the laboratory abnormalities already described, there were no other laboratory increases above the predefined thresholds for PCSAs.

Anti-mAb1 antibodies were positive in 6 out of 27 (22.22%) subjects who completed the study (no subject who did not complete the study had any detected ADA). Among the 6 subjects with positive ADA titers, 4 were treated with C2P1 and 2 were treated with C1P2. No association was observed between ADA development and TEAEs.

### E. Specific local tolerability assessments

On the pain VAS, the mean peak values were 4.4 and 4.2 mm (on the 100 mm scale) in the C2P1 and C1P2 treatment groups, respectively, with a median value at 2.0 mm in both groups. Five (5) out of 15 subjects in each group had "no pain" (peak value at 0 mm). The highest measurements were 17 and 18 mm in the C2P1 and C1P2 treatment groups, respectively, and were generally observed 2 minutes after dosing (range between 2 minutes and 12 hours postdose).

The mean peak values for erythema diameters measured were 12.5 and 10.9 mm in the C2P1 and C1P2 treatment groups, respectively. Nine (9) out of 15 subjects in each group had no erythema at any time. The maximum values observed were 40 mm in both groups, and were all observed 2 minutes after dosing, except for one subject whose maximum (3 mm) was observed 48 hours post-dose.

The mean peak values for edema diameters measured were 1.1 and 0 mm in the C2P1 and C1P2 treatment groups, respectively. Thirteen (13) out of 15 subjects and 15 out of 15 subjects in the C2P1 and C1P2 treatment groups, respectively, had no edema at any time. The maximum values were 15 and 1 mm in 2 subjects in the C2P1 treatment group, and were observed 2 hours post-dose.

### F. Conclusions

After a single subcutaneous dose of 300 mg of mAb1 to healthy subjects, serum functional mAb1 exposure was similar in the two test drug products. The geometric mean treatment ratios (DP1/DP2) with 90% Cls were 1.10 (0.89 to 1.35) for Cₘₐₓ, 0.90 (0.71 to 1.16) for AUCₗₐₛₜ, and 1.05 (0.86 to 1.29) for AUC.

mAb1 was generally well-tolerated. One subject administered with DP1 experienced a serious adverse event of "herpes simplex type II viral infection" followed by "Bell's Palsy".

There were no clinically important local tolerability issues and no apparent differences in local tolerability parameters (ie, pain, erythema, and edema) between treatment groups.

The most common TEAE was erythema at injection site (8 out of 30 subjects) and was observed with the same incidence in both treatment groups (4 out of 15 subjects [26.7%] in each group).

In conclusion, after a single 300 mg SC administration of mAb1 in healthy subjects, there was no clinically important difference identified in the PK profiles, safety, and local tolerability of the two different drug products.

### Example 4: Clinical trial of safety, tolerability and pharmacokinetics of ascending single subcutaneous dose of anti-IL-4R antibody in healthy Japanese adult male subjects

### A. Study Design

This study was a randomized, double-blind, placebo-controlled study of ascending, single subcutaneous doses of an anti-IL-4R antibody (mAb1) in healthy Japanese adult male subjects. The primary objective was to assess the safety and tolerability of mAb1 after ascending single subcutaneous doses in healthy Japanese male subjects. The secondary objectives were to assess the pharmacokinetics, the immunogenicity and exploratory pharmacodynamics of ascending single subcutaneous doses of mAb1 in healthy Japanese male subjects.

mAb1 was derived from cell line 2 and supplied in liquid formulation of either 75 mg/mL or 150 mg/mL concentration in vials. Single ascending doses of 75, 150, 300, and 600 mg of mAb1 were administered subcutaneously on day 1 (1 injection for 75 mg and for 150 mg; 2 injections for 300 mg; and 4 injections for 600 mg). Duration of observation was for approximately 11 weeks (including a screening period of 2 to 21 days prior to dosing, 5 days in the clinic [day -1 to day 4 with 1 treatment day], and outpatient follow-up visits up to 57 days after dosing) for each subject.

### B. Criteria for evaluation

Safety: Adverse events (AEs), physical examination, clinical laboratory evaluations (hematology, biochemistry, urinalysis), vital signs (supine and standing blood pressure and heart rate, body temperature), 12-lead electrocardiograms (ECGs), and anti-mAb1 antibodies

Pharmacokinetics: The following mAb1 serum functional pharmacokinetics parameters were calculated with non-compartmental analysis - maximum observed serum concentration (Cₘₐₓ), time to reach maximum serum concentration (tₘₐₓ), dose normalized Cₘₐₓ (Cₘₐₓ/Dose), area under the serum concentration versus time curve from time zero to the real time corresponding to the last concentration above the lower limit of quantification tₗₐₛₜ (AUCₗₐₛₜ), dose normalized AUCₗₐₛₜ (AUCₗₐₛₜ/Dose), area under the serum concentration versus time curve from time zero extrapolated to infinity (AUC), apparent volume of distribution at steady state (Vₛₛ/F), apparent total body clearance (CUF), mean residence time (MRT), and terminal half-life (t_{½z})

Pharmacodynamics (PD): pharmacodynamics effects of mAb1 on total IgE and TARC

Blood samples for PK evaluation were collected at predose (Day 1) and days 1, 2, 4, 8, 11, 15, 18, 22, 25, 29, 36, 43, 50, and 57 (±1 day for days 15 to 25; ±2 days for days 29 to 57) following mAb1 administration. Serum concentrations of mAb1 were determined using a validated ELISA with a lower limit of quantification (LLOQ) of 78 ng/mL (0.078 mg/mL). Blood samples for PD evaluation were collected prior to dosing at Day -1 and on day 1, then on days 8, 15, 22, 29, 43, and 57 (±1 day for days 15 to 25; ±2 days for days 29 to 57) following mAb1 administration. Serum screens for total IgE and TARC were determined using a validated method.

### C. Statistical Methods

Evaluation of safety was based on the review of individual values and descriptive statistics. All adverse events were coded using MedDRA version 15.1, and frequencies of treatment-emergent adverse events (TEAEs) were classified and tabulated (counts and percentages) by primary system organ class, preferred term, and treatment group. Potentially clinically significant abnormalities for clinical laboratory data, vital signs, and ECG data and out of normal range values for clinical laboratory data were flagged and summarized by treatment group. In addition, raw data and changes from baseline for vital signs, ECGs, and limited laboratory parameters were summarized in descriptive statistics.

Pharmacokinetic parameters of serum functional mAb1 were summarized for each dose group using descriptive statistics (mean, geometric mean, standard error of the ean [SEM], median, standard deviation [SD], and coefficient of variation [CV], minimum and maximum). Dose proportionality was assessed using a power model for Cₘₐₓ, AUCₗₐₛₜ, and AUC. The dose effect on t_{½z} was assessed with a linear fixed effect model. The distribution of tₘₐₓ values was represented by histogram plots. mAb1 PD biomarkers (total IgE and TARC: CCL17) were summarized for each dose group using descriptive statistics.

### D. Safety Results

mAb1 administration of a single subcutaneous dose of up to 600 mg was well tolerated in healthy Japanese adult male subjects with a median weight of 65.1 kg. No serious TEAEs or premature discontinuations were reported during the study. During the 57-day period of observation after dosing, a total of 3 TEAEs were reported among the 32 study subjects as follows: 1 out of 8 subjects in the placebo group (influenza), 1 out of 6 subjects in the 150 mg group (influenza) and 1 out of 6 subjects in the 600 mg group (orthostatic hypotension).

There were no local cutaneous reactions or discomfort at the site on injection at volumes up to 2.0 mL x 4 sites (600 mg).

Anti-mAb1 antibodies (ADAs) were positive in 5 out of 32 subjects with low titer levels (1 in 75 mg group, 2 in 150 mg group, 1 in 300 mg group, and 1 in 600 mg group). ADAs were undetectable at baseline and in the placebo group in all subjects. No ADA positive subject experienced any TEAE.

Very few PCSAs in hematology and biochemistry values were identified in the mAb1 treatment group without any dose-incidence relationship. In particular, there were no changes in liver enzymes observed. There were few PCSAs for vital signs or ECG, with no dose relationship. No subjects experienced a prolonged QTcB (> 450 ms) and no changes from baseline over 60 ms were observed during the study.

### E. Pharmacokinetic Results

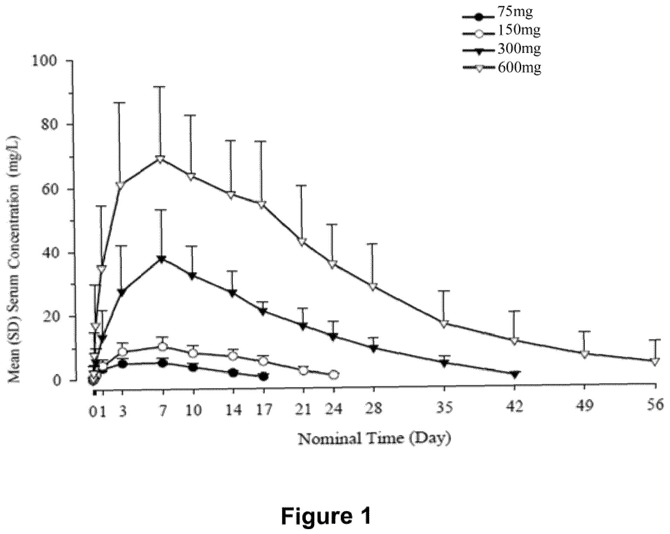

Mean (SD) serum functional mAb1 concentration-time profiles following single subcutaneous doses are shown in Figure 1. Pharmacokinetic parameters for serum functional mAb1 are summarized for all subjects treated with mAb1 in Table 5.

**Table 5: Mean ± SD (geometric mean) [CV%] of serum mAb1 PK parameters**

| PK parameter | mAb1 75 mg | mAb1 150 mg | mAb1 300 mg | mAb1 600 mg |
|---|---|---|---|---|
| N | 6 | 6 | 6 | 6 |
| Cmax (mg/L) | 5.33 ± 1.50 (5.09) [28.2] | 10.4 ± 2.95 (10.1) [28.2] | 38.3 ± 15.3 (36.1) [40.1] | 70.1 ± 24.1 (66.8) [34.4] |
| tₘₐₓ^{a} (day) | 7.01 (3.00 - 7.03) | 7.01 (3.00 - 7.03) | 7.01 (6.99 -10.00) | 7.01 (3.00 - 7.02) |
| tₗₐₛₜ^{a} (day) | 17.02 (14.01 - 21.05) | 24.03 (21.01 - 24.04) | 42.00 (35.00 - 42.02) | 52.51 (42.00 - 56.02) |
| AUCₗₐₛₜ (mg.day/L) | 59.2 ± 20.8 (55.2) [35.2] | 150 ± 41.3 (146) [27.5] | 700 ± 234 (667) [33.5] | 1780 ± 699 (1680) [39.3] |
| AUC (mg.day/L) | 72.4 ± 10.6^{b} (71.9) [14.6] | 155 ± 41.6 (151) [26.8] | 709 ± 231 (677) [32.6] | 1870 ± 852 (1740) [45.5] |
| Vₛₛ/F (L) | 7.96 ± 0.673 ^{b} (7.94) [8.5] | 10.7 ± 3.04 (10.3) [28.4] | 6.67 ± 2.34 (6.32) [35.1] | 6.60 ± 1.78 (6.41) [26.9] |
| CL/F (L/day) | 1.05 ± 0.144 ^{b} (1.04) [13.7] | 1.02 ± 0.238 (0.993) [23.4] | 0.464 ± 0.155 (0.443) [33.5] | 0.366 ± 0.126 (0.344) [34.4] |
| t_{½z} (day) | 2.77 ± 0.567 ^{b} (2.72) [20.5] | 3.18 ± 0.805 (3.11) [25.3] | 5.13 ± 1.42 (4.96) [27.7] | 8.77 ± 5.18 (7.66) [59.1] |

| | | | | |
|---|---|---|---|---|
| ^{a} Median (Min - Max); ^{b} N=4 since terminal log-linear phase could not be determined in 2 subjects | | | | |

Median tₘₐₓ of mAb1 was 7 days at all doses. Mean terminal elimination half-life (t_{½z}) was dose-dependent (p<0.01) and ranged from 2.77 days at 75 mg to 8.77 days at 600 mg. An 8-fold increase in dose from 75 mg to 600 mg resulted in 13.1-, 30.4-, and 24.2-fold increase in geometric mean Cₘₐₓ, AUCₗₐₛₜ, and AUC respectively.

### F. Pharmacodynamics Results

Serum IgE and TARC values were highly variable within the treatment groups. Regarding the serum IgE (percent change from baseline), no drug-related effect was observed over time at single administrations of subcutaneous doses at 75 mg and 150 mg. At 300 mg and 600 mg, there was a trend of decreasing serum IgE post-treatment. A treatment effect was observed on TARC. Single administrations of subcutaneous doses between 75 mg and 600 mg were associated with reduced serum TARC levels as compared to placebo. A more sustained reduction was associated with increasing dose.

### G. Conclusions

mAb1 with single subcutaneous dose up to 600 mg was well tolerated in healthy Japanese male subjects. No serious TEAEs or premature discontinuations were reported during the study. A total of 3 TEAEs were reported among the 32 study subjects. There were no local cutaneous reactions or discomfort at the site on injection at volumes up to 2.0 mL x 4 sites (600 mg). Overall, the reported TEAEs and laboratory, vital signs and ECG assessments did not suggest dose-related effects.

After single doses to healthy Japanese adult males, mAb1 was absorbed with a median tmax of 7 days and eliminated with a dose-dependent mean terminal elimination half-life (t_{½z}) ranging from 2.77 days at 75 mg to 8.77 days at 600 mg. Mean serum functional mAb1 exposure increased in a greater than dose proportional manner, with an 8-fold increase in dose from 75 mg to 600 mg resulting in a 13.1-, 30.4-, and 24.2-fold increase in geometric mean Cₘₐₓ, AUCₗₐₛₜ, and AUC, respectively.

A pharmacodynamic effect was observed. Serum levels of TARC were reduced post-treatment with mAb1. A more sustained decline was associated with increasing dose. Low titers of ADA were detected in 5 out of 32 subjects. ADAs were undetectable at baseline and in the placebo group in all subjects. No ADA positive subject experienced any TEAE.

### Example 5: Clinical trial to assess the effect of injection rate on safety and tolerability of mAb1 administered subcutaneously to healthy volunteers

### A. Overview and Study Design

This study was conducted to support the development of a large volume injection device for administering mAb1. The study assessed comparatively two different injection rates approximating the corresponding attributes of two different subcutaneous (SC) delivery devices: A fast injection representing an autoinjector and a slow injection representing a microinfuser. The primary objective of the study was to assess the comparative safety and tolerability of a single 300 mg/2 mL dose of mAb1 administered SC at 2 different rates to normal healthy volunteers. The secondary objectives of the study were: to compare the pharmacokinetic (PK) profiles of a single 300 mg/2 mL dose of mAb1 administered SC at 2 different rates in two separate cohorts of NHV; and to assess the comparative immunogenicity of a single 300 mg/2 mL dose of mAb1 administered SC at 2 different rates in NHV.

This was an open label, randomized, parallel-group, single-dose study of the safety, tolerability, PK and immunogenicity of mAb1 administered SC at 2 different injection rates. The study was randomized to avoid any potential bias in assigning subjects to study treatment and to minimize systematic differences between treatment groups with respect to baseline variables that could affect the outcome. Injection method and duration could not be effectively blinded, so the study was open-label. Thirty-six subjects (18 subjects per treatment group) were recruited and randomized at 1 site in the US. The sample size for this study was selected empirically. No formal sample size or power calculations based on the primary endpoint were used. However, it was estimated that the enrollment of 18 subjects per group would provide 80% power to detect the difference of 20 in pain VAS between 2 groups, assuming the common standard deviation is 20.8 in pain VAS with a 2-sided test at the 0.05 significance level.

Subjects underwent screening between day -14 and day -2. On day -1, subjects were admitted to the clinic for training and familiarization with the injection procedure, and were randomized to either group 1 (fast injection) or group 2 (slow injection):
- Group 1 (Fast injection): subjects received study drug via manual SC injection administered over 30 seconds.
- Group 2 (Slow injection): subjects received study drug via a SC infusion set connected to a syringe pump programmed to deliver 2 mL in 10 minutes.

On day -1, all subjects underwent a mock injection, in which the SC infusion set was briefly attached to the skin. The process included the insertion of a 27G 6-mm needle, which was left in place for about 10-15 seconds and then removed. Subjects rated their pain/discomfort related to each respective step of the mock injection procedure as follows:
➢ Immediately (within 10 seconds) of needle insertion and needle removal, subjects rated their pain/discomfort related to each respective step of the procedure.
➢ Global assessment (GA): 1 minute after needle removal subjects were asked to provide a GA by recalling and rating the pain/discomfort experienced during the entire procedure.
➢ Comparative assessment (CA): approximately 1 minute after needle removal (immediately post GA), in addition to the visual analog scale (VAS), subjects provided a CA by relating their global pain/discomfort to familiar experiences, like a bee sting or a flu shot.

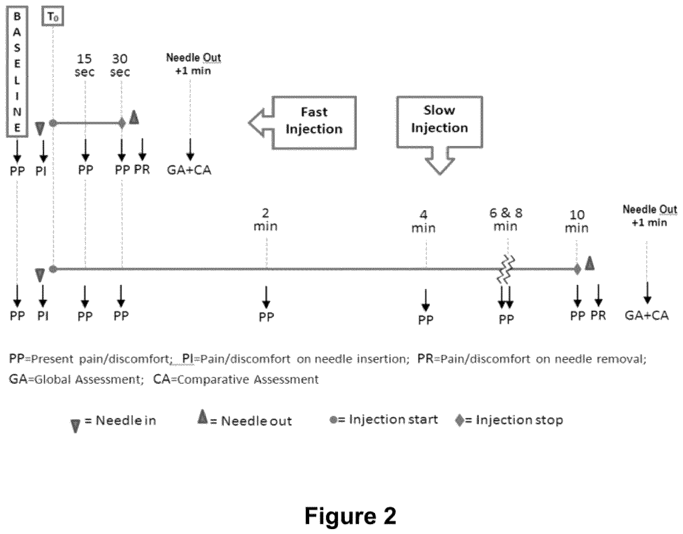

On day 1, all subjects received 300 mg mAb1 in 2 mL volume and completed VAS assessments according to the diagram in Figure 2.

Information on the incidence, extent, and severity of injection site reactions (ISRs), such as erythema, edema, induration, tenderness, and itching, were monitored for all subjects (in groups 1 and 2). The extent (largest diameter in mm) of erythema, edema, and induration, as well as the severity of erythema and edema, were assessed 1, 2, 4, and 8 hours post completion of the injection and at follow-up visits through the end of the study or until the injection site appeared normalized at 2 consecutive assessments, based on all parameters evaluated. Subjects were also asked to rate any pruritus (itching) and tenderness (pain on palpation) present using VAS.

Subjects were discharged from the clinic on day 2. Subjects returned to the clinic for outpatient follow-up visits on days 4, 8, 11, 15, 22, 29, 36, 43, 50, 57, and 64 (end of study). Day 8, 11, and 15 clinic visits could have occurred within a window of +/- 1 day. Visits from day 22 through day 64 could have occurred within a window of +/- 2 days. The total observation period for each subject was 9 weeks following day 1 dosing.

### B. Analysis Variables and Statistical Methods

The following demographic and Baseline characteristics variables were summarized: Age at screening (year), Gender, Ethnicity, Race, Baseline Weight (kg), Height (m), and BMI (kg/m2), Pain/discomfort VAS. Primary variables include the following measurements for safety and tolerability: (i) incidence and severity of treatment-emergent adverse events (TEAEs) through day 64 (end of study); Incidence, extent, severity and duration of ISRs through day 64; (ii) overall pain/discomfort associated with the injection procedure (GA); (iii) individual pain/discomfort components on needle insertion, while injecting study drug and on needle removal; and (iv) residual pain/discomfort over time: present pain/discomfort at 5 minutes, 10 minutes, 15 minutes, 30 minutes, 1, 2, 4, and 8 hours after study administration, and at subsequent study visits.

Information on the incidence, extent, and severity of ISRs, such as erythema, edema, induration, tenderness, and itching, were monitored for all subjects (in groups 1 and 2). The extent (largest diameter in mm) of erythema, edema and induration were assessed. In addition, the severity of erythema and edema were qualitatively assessed using standard 0-4 dermal tolerability scales (Draize) 1, 2, 4, and 8 hours post completion of the injection, and at follow-up visits through the end of the study or until the injection site appeared normalized at 2 consecutive assessments, based on all parameters evaluated.

The following scales were used to grade the severity of erythema and edema:
Erythema:
   0 = no erythema
   1 = very slight erythema (barely perceptible)
   2 = well defined erythema
   3 = moderate to severe erythema
   4 = severe erythema (beet redness) to slight eschar formation (injuries in depth)
Edema:
   0 = no edema
   1 = very slight edema (barely perceptible)
   2 = slight edema (edges well defined)
   3 = moderate edema (raised >1 mm)
   4 = severe edema (raised >1 mm and beyond area of exposure)

The VAS is a continuous rating scale (0-100mm) used by patients to rate their pain/discomfort related to study drug injection. The VAS was anchored by "no pain/discomfort" on the left and "worst possible pain/discomfort" on the right. The same scale was used to quantify injection site itching and tenderness, which were assessed as part of ISR.

Safety and tolerability of mAb1 were assessed by physical examination, vital signs, electrocardiograms (ECGs), and clinical laboratory evaluations. Subjects were asked to monitor and report all adverse events (AEs) experienced from the time the informed consent is signed until the end of study visit on day 64. Adverse events, serious adverse events, and treatment-emergent adverse events have been defined elsewhere herein.

Blood samples were collected for PK analyses at every study visit starting at baseline (day 1, pre-dose and post-dose [at the end of the injection and 1, 2, 4, 8, and 12 hours post dose]). Blood samples were collected for the analysis of anti-mAb1 antibody levels on day 1 (pre-dose), and on days 29 and 64 (end of study).

For continuous variables, descriptive statistics included the following: the number of patients reflected in the calculation (n), mean, median, standard deviation, minimum, and maximum. For categorical or ordinal data, frequencies and percentages were displayed for each category.

### C. Results

Pain on injection and residual pain: Both injection modalities - fast and slow - were well tolerated and associated with relatively low levels of pain on injection. For both modalities, pain peaked approximately 15-30 seconds after the start of injection. Mean peak pain levels were below 15 mm on the 0-100 mm VAS. Mean pain scores, including the global assessment (overall pain recalled at 1 minute after the injection), as well as residual pain over time, were comparable between the fast and slow injection; differences observed were not clinically relevant (i.e., Δ<10 on the 0-100 VAS scale). More subjects on slow injection reported little to no pain (VAS<5 mm), compared to those who received the fast injection. Overall, injection pain profile seemed slightly more favorable for the slow injection, but did not clearly differentiate the two injection modalities.

Injection site reactions: ISR incidence overall was similar between the two study groups (89% for the fast injection, versus 94% for the slow injection). However, incidence of objective ISR findings (erythema and/or induration) was higher in the in the slow injection group (83%) compared with the fast injection group (44%), especially for injection site erythema (61% vs. 11%, respectively). Subjective ISRs included tenderness and pruritus at the injection site and their incidence was somewhat higher for the fast injection (72%) compared with the slow injection (56%), especially for injection site tenderness (72% vs. 39%, respectively). ISR onset was noted from 1 hour to several days after the injection. Time to ISR resolution was also reported from 1 hour to several days after onset. Overall, the ISR profile appeared somewhat more favorable to the fast injection group, but did not provide a clear differentiation between the two injection modalities.

Adverse events: The number and incidence of treatment emergent adverse events were higher in the slow-injection group (35 TEAEs reported in 15 subjects) compared with the fast-injection group (19 TEAEs reported in 11 subjects). Most TEAEs were ISRs that were reported as adverse events based on investigator's assessment of clinical relevance. No TEAE led to discontinuation. There were 3 serious TEAEs related to a single case which were not related to the study drug or injection modality. Overall, the adverse event profile appeared slightly more favorable to the fast-injection group, mostly on the account of ISRs reported as adverse events.

### D. Conclusion

The study achieved the primary and secondary objectives stated in the protocol. mAb1 was safe and well tolerated when administered by either fast or slow injection. The results of the study did not provide a clear differentiation between the two injection modalities.

### Example 6: Sequential Ascending repeat-dose clinical trial of subcutaneously administered anti-IL-4R antibody (mAb1) in patients with moderate-to-severe atopic dermatitis

### A. Study Design

This study was a phase 1b, randomized, double-blind, placebo-controlled, sequential ascending, repeat-dose study of mAb1 subcutaneously administered in patients with moderate-to-severe extrinsic atopic dermatitis (AD). Thirty patients were randomized into the study (6 in placebo, 8 each in of 75 mg, 150 mg and 300 mg groups). Twenty-eight patients received all the treatments. The treatment period was 4 weeks in duration; patients were followed for 8 weeks after end of the treatment period. Patients were randomized in a 4:1 ratio to receive mAb1 or placebo in each of 3 ascending dose cohorts (75, 150, or 300 mg mAb1). The primary objective of the trial was to access the safety and tolerability, with PK as a secondary objective. Exploratory objectives included efficacy and biomarker endpoints. The exploratory efficacy variables included: (i) proportion of patients who achieved an IGA score of 0 or 1 through week 4 and each study visit; (ii) change and percent change in BSA, EASI, and 5-D pruritus scale from baseline to each visit; and (iii) weekly change from baseline in NRS scale.

### B. Efficacy variables

The efficacy variables IGA, BSA, EASI, SCORAD, 5-D pruritus scale, and pruritus NRS rating have been described elsewhere herein (see, for example, Example 7).

The IGA, BSA, EASI and SCORAD scores were assessed at every clinic visit. Patients underwent 5-D pruritus assessment at the following visits: screening, day 1/baseline (pre-dose), and days 15, 29, 43, 57, 71, and 85 (end of study) or early termination. Patients used the IVRS to record their Pruritus NRS score twice daily through the last study visit.

Baseline for efficacy variable is defined as the last non-missing value on or before the date of randomization. For the patient who has no value on or before his/her randomization date the last non-missing value on or before the date of first dose injection will be used as baseline.

### C. Statistical methods

Summary of safety and exploratory efficacy variables was generated by dose group and overall. The summary of safety and tolerance were performed based on the safety analysis set (SAF). The safety analyses were based on the reported adverse events (AEs), clinical laboratory evaluations, vital signs, and 12-lead ECG.

All the categorical variables were analyzed using the Fisher's exact test with nominal p-value and confidence intervals reported.

All continuous variables were analyzed by the ANalysis of COVAriance (ANCOVA). Unless otherwise specified, assessments of changes from baseline and construction of confidence intervals for continuous measures were based on an ANCOVA model which included treatment as the main factor and baseline value as covariates. Point estimate and 95% Cl of the difference in adjusted mean change from baseline between two treatment groups were provided. Due to small sample size of this study, p-values from the tests of the exploratory efficacy variables were provided for descriptive purpose. Missing values were imputed by the last observation carried forward (LOCF).

### D. Patient Disposition

The patients in placebo group were the youngest, and 33% of patients in placebo group were Hispanic or Latino compared to the treatment groups where all the patients were non-Hispanic. Table 6 summarizes the demographic characteristics of the patient population.

**Table 6: Summary of Demographic Characteristics**

| | Placebo (N=6) | 75 mg (N=8) | 150mg (N=8) | 300mg (N=8) | All Doses (N=24) |
|---|---|---|---|---|---|
| Mean age, years (SD) | 23.0 (4.00) | 35.8 (12.51) | 45.6 (10.78) | 46.1 (11.36) | 42.5 (12.09) |

| Ethnicity, n (%) | | | | | |
|---|---|---|---|---|---|
| Hispanic or Latino | 2 (33.3%) | 0 | 0 | 0 | 0 |
| Non-Hispanic | 4 (66.7%) | 8 (100%) | 8 (100%) | 8 (100%) | 24 (100%) |

| Race, n (%) | | | | | |
|---|---|---|---|---|---|
| American-Indian/ Alaska Native | 0 | 0 | 0 | 1 (12.5%) | 1 (4.2%) |
| Asian | 0 | 0 | 0 | 1 (12.5%) | 1 (4.2%) |
| African-American | 2 (33.3%) | 4 (50.0%) | 2 (25.0%) | 2 (25.0%) | 8 (33.3%) |
| White | 4 (66.7%) | 4 (50.0%) | 5 (62.5%) | 4 (50.0%) | 13 (54.2%) |
| Other | 0 | 0 | 1 (12.5%) | 0 | 1 (4.2%) |

| Gender, n (%) | | | | | |
|---|---|---|---|---|---|
| Male | 3 (50.0%) | 6 (75.0%) | 5 (62.5%) | 2 (25.0%) | 13 (54.2%) |
| Female | 3 (50.0%) | 2 (25.0%) | 30 (37.5%) | 6 (75.0%) | 11 (45.8%) |
| Mean BMI, kg/m³ (SD) | 26.81 (8.191) | 26.41 (4.489) | 27.32 (5.285) | 31.60 (12.890) | 24.45 (8.399) |

Table 7 summarizes the baseline disease characteristics of the patient population.

**Table 7: Baseline Disease Characteristics**

| | Placebo (N=6) | 75 mg (N=8) | 150mg (N=8) | 300mg (N=8) | All Doses (N=24) |
|---|---|---|---|---|---|
| Duration of chronic AD, years | 17.8 (6.82) | 24.5 (16.95) | 27.5 (16.34) | 33.9 (17.92) | 28.6 (16.80) |
| EASI score | 18.1 (7.17) | 36.9 (11.75) | 25.6 (13.84) | 29.8 (6.44) | 30.8 (11.65) |
| IGA score | 3.2 (0.41) | 4.1 (0.35) | 3.9 (0.64) | 3.8 (0.46) | 3.9 (0.50) |
| %BSA of AD | 31.6 (24.27) | 64.4 (17.03) | 46.9 (28.20) | 47.0 (20.49) | 52.84 (22.99) |
| 5-D pruritus scale | 15.5 (3.83) | 21.5 (3.55) | 19.3 (2.92) | 19.6 (2.97) | 20.1 (3.18) |
| Pruritus NRS score | 5.8 (1.60) | 7.0 (1.78) | 6.7 (1.62) | 6.0 (1.18) | 6.6 (1.54) |

The mean baseline IGA, EASI, BSA, and pruritus NRS for the study participants was approximately 3.8, 28.2, 48.5, and 6.4 respectively.

### E. Results

Subcutaneous administration of mAb1 to patients with moderate-to-severe AD was safe and well tolerated in this study. A single serious adverse event was recorded for a patient in the 150 mg group, who was diagnosed with exercise-associated CPK increase. No deaths were reported. 25 of the treated patients or 83% reported at least one treatment emergent adverse event (TEAE). The most frequent TEAE from the treatment groups were infections and infestations (n=7 [29%] vs. 1 [17%] for placebo), and headaches in patients dosed with mAb1 (n=3 [13%] vs. 1 [17%] for placebo).

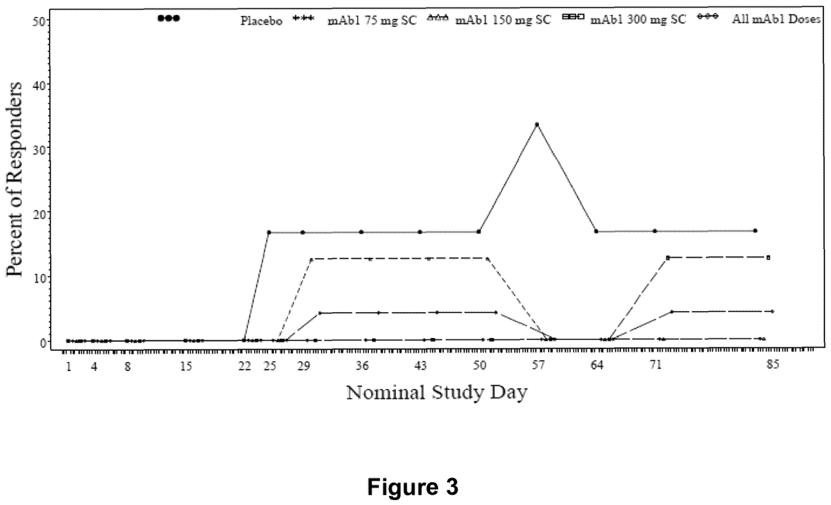

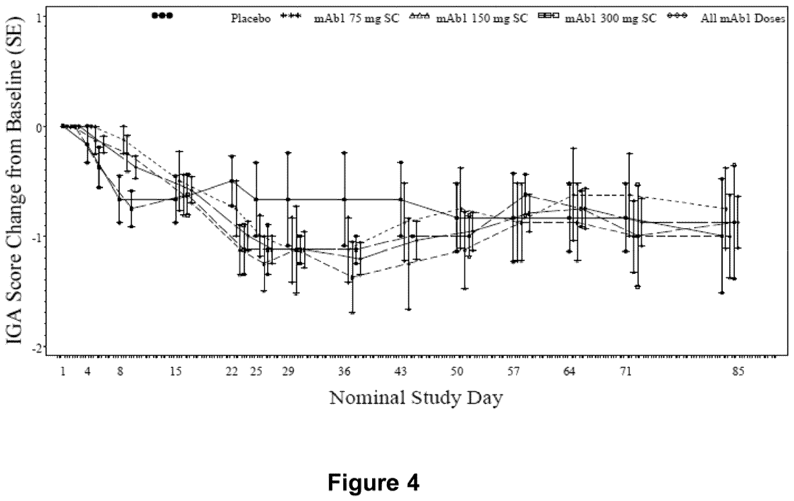

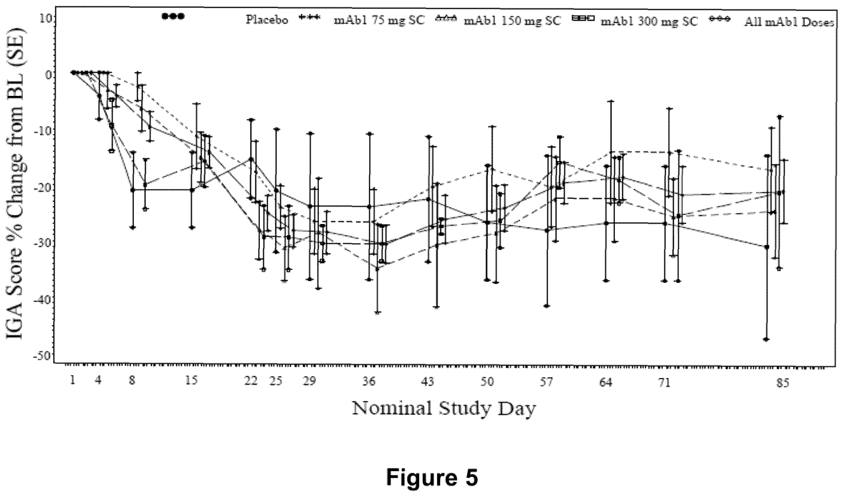

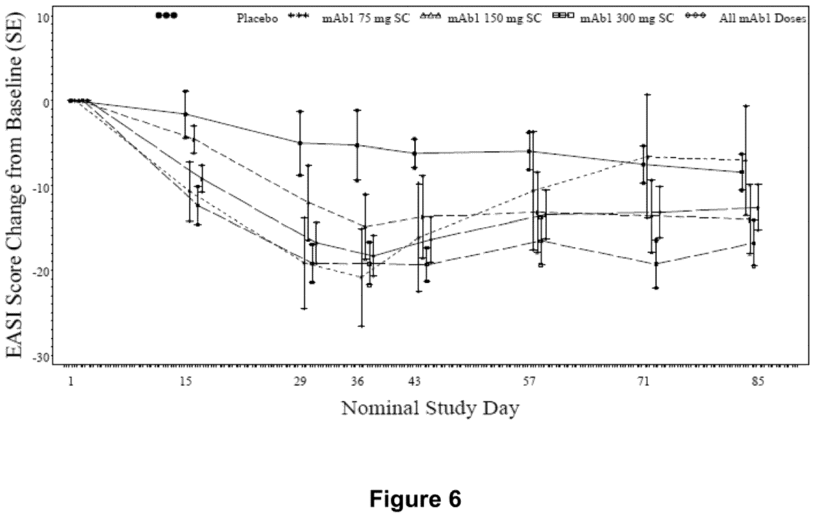

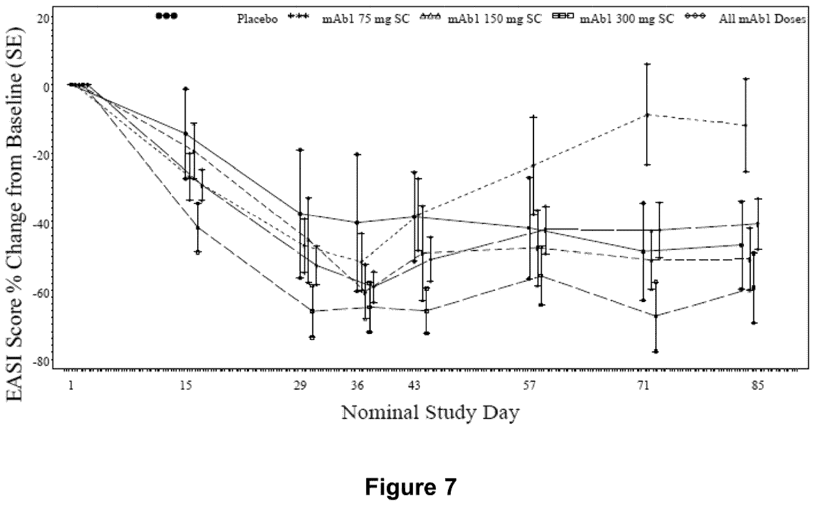

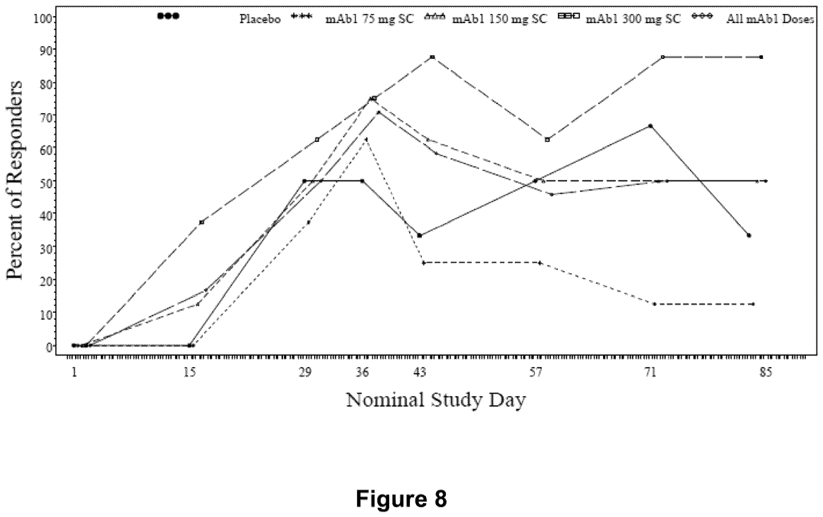

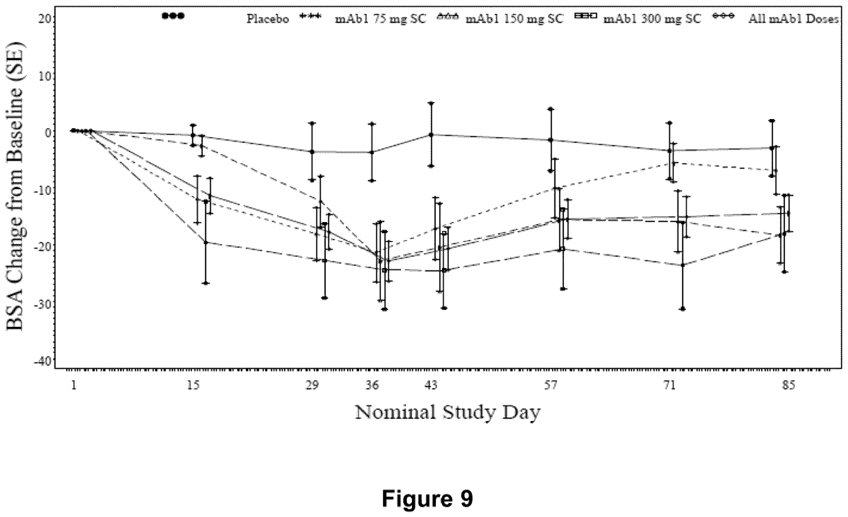

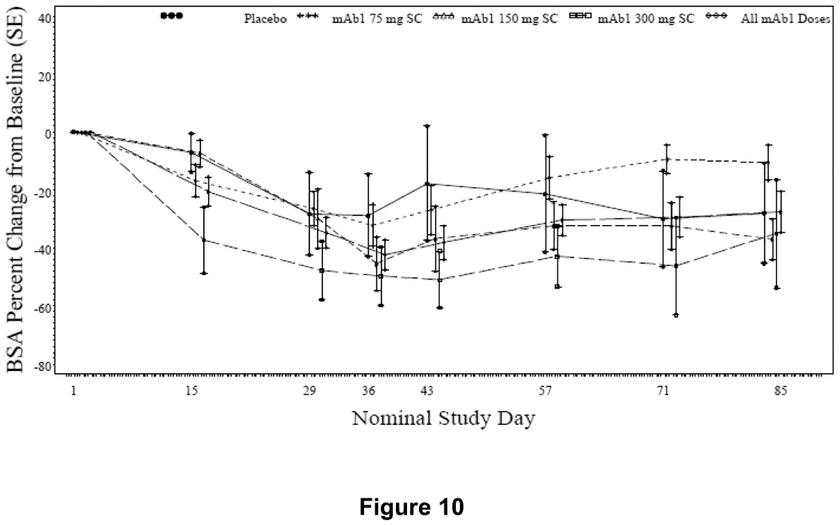

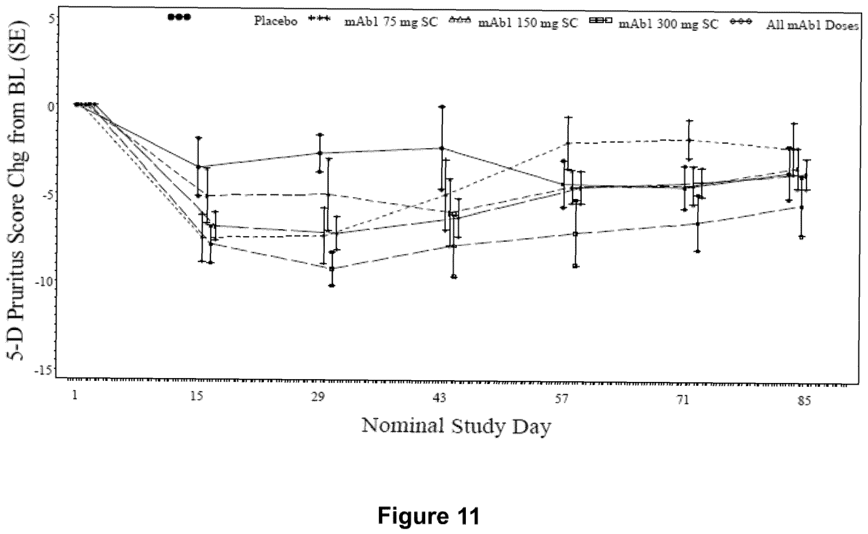

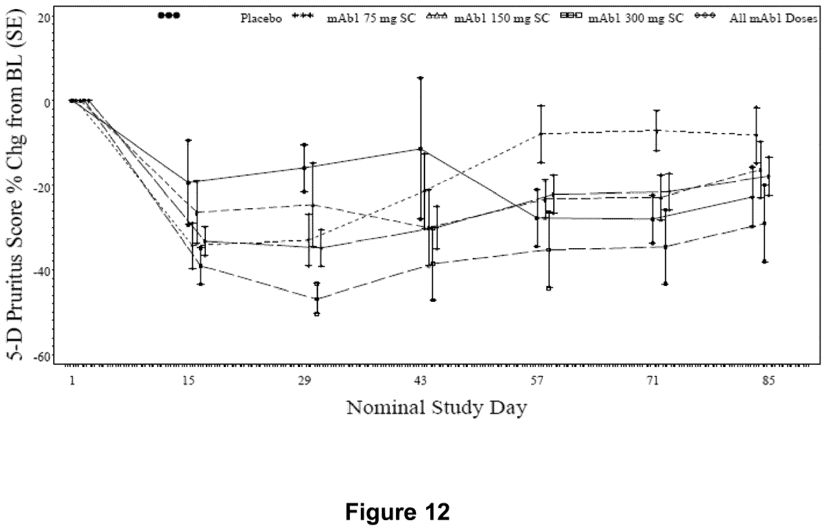

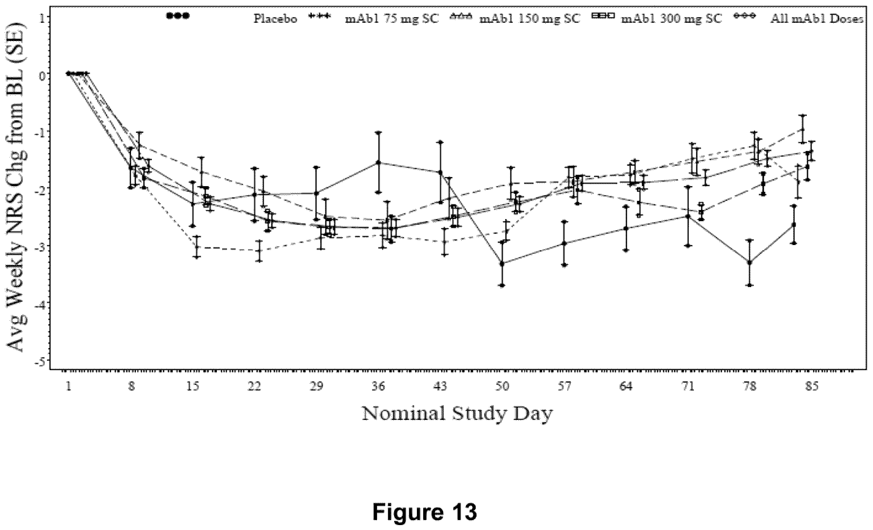

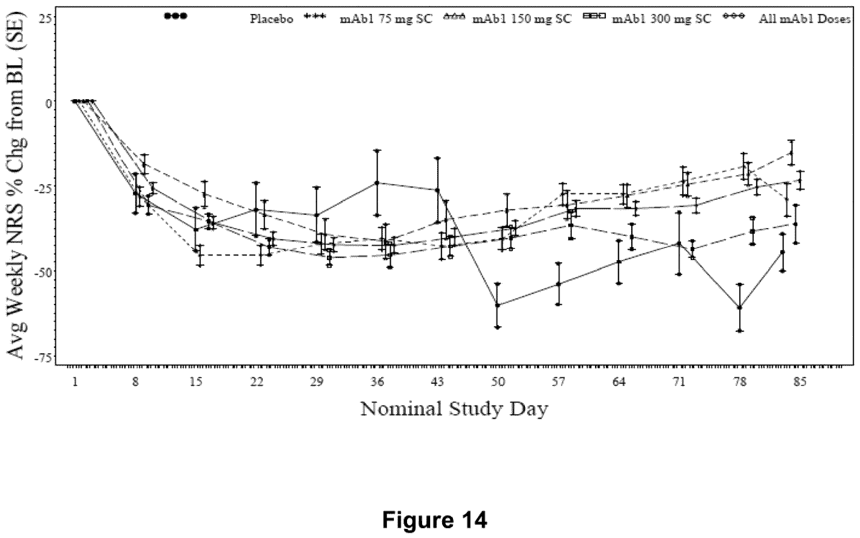

The baseline and exploratory efficacy results obtained from the study are summarized in Figures 3 - 14. mAb1 administration did not induce statistically significant improvement in any exploratory endpoints of AD. This may be due to the small sample size and the fact that the placebo patients were less severe and younger than active treatment groups.

### Example 7: Clinical Trial of Subcutaneously Administered Anti-IL-4R Antibody (mAb1) In Patients with Moderate-to-Severe Atopic Dermatitis

### A. Study Design

This study was a 12-week, double-blind, randomized, placebo-controlled, sequential ascending, repeated-dose study to assess the safety and pharmacokinetic profile of subcutaneous administration of the anti-IL-4R mAb, referred to herein as "mAb1," in adult patients with moderate-to-severe atopic dermatitis. Patients with moderate-to-severe AD had an Eczema Area and Severity Index (EASI) ≥ 12 and minimum 10% body surface area involvement. The treatment period was four weeks in duration, with patients being followed for 8 weeks after the end of the treatment period. Patients were withdrawn from topical agents (e.g., pimecrolimus, tacrolimus, and topical corticosteroids) for at least 1 week prior to baseline. Oral corticosteroids and immunosuppressives (e.g., cyclosporine, mycophenolate-mofetil, IFNγ) were also prohibited from ≥ 4 weeks prior to baseline.

Patients were randomized in a 3:1 ratio to receive mAb1 or placebo in each of two ascending dose cohorts (150 mg or 300 mg). The study consisted of a screening period (day - 14 through day -3), a treatment period (day 1 through day 29) (topical steroids were not allowed), and a follow-up period (day 29 through day 85) (topical steroids were allowed). During the treatment period, patients were seen in the clinic at least once weekly for safety, laboratory, and clinical effect assessments on days 1, 4, 8, 15, 22, 25 and 29 (week 4). Patients received a dose of study drug on days 1, 8, 15 and 22. Patients were monitored at the study site for 2 hours after each dose of study drug. The end of the treatment period study visit occurred on day 29 (week 4). During the follow-up period patients were seen in the clinic for follow-up assessments at days 36, 43, 50, 57, 64, 71 and 85 (end of study visit).

### B. Efficacy Variables

The exploratory efficacy variables measured in this study included: (1) proportion of patients who achieved an investigator's global assessment (IGA) score of 0 or 1 through week 4 and each study visit; (2) change and percent change in body surface area involvement of atopic dermatitis (BSA), eczema area and severity index (EASI), SCORAD, and 5-D pruritus scale from baseline to each visit; (3) weekly change from baseline in pruritus numeric rating scale (NRS); (4) change from baseline in circulating eosinophils, TARC, eotaxin-3, and total IgE through week 4; (5) change from baseline in circulating eosinophils, TARC, eotaxin-3, and total IgE through week 12; and (6) change from baseline in eosinophils, TARC, eotaxin-3, Phadiatop^{™} results, and total IgE associated with response through week 4.

Baseline for efficacy variable is defined as the last non-missing value on or before the date of randomization. For the patient who has no value on or before his/her randomization date the last non-missing value on or before the date of first dose injection will be used as baseline.

investigator's Global Assessment (IGA): The IGA is an assessment scale used in clinical studies to determine severity of AD and clinical response to treatment based on a 6-point scale ranging from 0 (clear) to 5 (very severe). The IGA score was assessed at every clinic visit.

Body Surface Area Involvement of Atopic Dermatitis (BSA): BSA affected by AD was assessed for each major section of the body (head, trunk, upper extremities, and lower extremities) and was reported as the total of percentage from each body sections. Patients were assessed for BSA at the following visits: screening, day1/baseline (pre-dose), and days 15, 29, 36, 43, 57, 71, and 85 (end of study) or early termination.

Eczema Area and Severity Index (EASI): The EASI is a validated measure used in clinical practice and clinical trials to assess the severity and extent of AD (Hanifin et al 2001, Exp. Dermetol. 10: 11-18). The EASI score calculation is based upon the Physician's Assessment of Individual Signs [erythema (E), induration/papulation (I), excoriation (X), and lichenification (L)], where each sign is scored as 0 = Absent, 1 = Mild, 2 = Moderate, or 3 = Severe, and also upon the Area Score [based on the % (BSA) affected] where 0 = 0% BSA, 1 = 1-9% BSA, 2 = 10-29% BSA, 3 = 30-49% BSA, 4 = 50-69% BSA, 5 = 70-89% BSA, 6 = 90-100% BSA.

For each of major section of the body (head, upper extremities, trunk and lower extremities), EASI score = (E+I+X+L) x Area Score. The total EASI score is the weighted total of the section EASI using the weights 10% = head, 20% = upper extremities, 30% = trunk, 40% = lower extremities. The minimum possible EASI score is 0 and the maximum possible EASI score is 72 where a higher score indicates increased severity of atopic dermatitis. Achieving an EASI 50 (50% or greater improvement in EASI score is considered by dermatology investigators to a clinically significant level of improvement to use as an endpoint.

Patients underwent EASI score assessment at the following visits: screening, day 1/baseline (pre-dose), and days 15, 29, 36, 43, 57, 71, and 85 (end of study) or early termination.

SCORAD: The SCORAD is a validated tool used in clinical research and clinical practice that was developed to standardize the evaluation of the extent and severity of AD (Dermatology 1993, 186: 23-31). The extent of AD is assessed as a percentage of each defined body area and reported as the sum of all areas, with a maximum score of 100% (assigned as "A" in the overall SCORAD calculation). The severity of 6 specific symptoms (erythema, oedema / papulation, excoriations, lichenification, oozing / crusts and dryness) of AD is assessed using the following scale: none (0), mild (1), moderate (2), or severe (3) (for a maximum of 18 total points, assigned as "B" in the overall SCORAD calculation). Subjective assessment of itch and sleeplessness is recorded for each symptom by the patient or relative on a visual analogue scale (VAS), where 0 is no itch (or sleeplessness) and 10 is the worst imaginable itch (or sleeplessness), with a maximum possible score of 20. This parameter is assigned as "C" in the overall SCORAD calculation. The SCORAD score is calculated as A/5 + 7B/2 + C. The maximum SCORAD score is 103.

Patients underwent SCORAD assessment at the following visits: screening, day 1/baseline (pre-dose), and days 15, 29, 36, 43, 57, 71, and 85 (end of study) or early termination.

5-D Pruritus Scale: The 5-D Pruritus Scale is a 5-question tool used in clinical trials to assess 5 dimensions of background itch: degree, duration, direction, disability, and distribution (Elman et. al.2010, Brit. J. Dermatol. 162: 587-593). Patients rate their symptoms over the preceding 2-week period as "present" or on a 1 to 5 scale, with 5 being the most affected for each question in degree, duration, direction and disability. Single-item domain scores (duration, degree and direction) are equal to the value indicated below the response choice (range 1-5).

The disability domain includes four items that assess the impact of itching on daily activities: sleep, leisure/social activities, housework/errands and work/school. The score for the disability domain is achieved by taking the highest score on any of the four items.

For the distribution domain, the number of affected body parts is tallied (potential sum 0-16) and the sum is sorted into five scoring bins: sum of 0-2 = score of 1, sum of 3-5 = score of 2, sum of 6-10 = score of 3, sum of 11-13 = score of 4, and sum of 14-16 = score of 5.

The scores of each of the five domains are achieved separately and then summed together to obtain a total 5-D score. 5-D scores can potentially range between 5 (no pruritus) and 25 (most severe pruritus).

Patients underwent 5-D pruritus assessment at the following visits: screening, day 1/baseline (pre-dose), and days 15, 29, 43, 57, 71, and 85 (end of study) or early termination.

Pruritus Numeric Rating Scale (NRS): The Pruritus NRS is a single-question assessment tool that was used to assess the patient's worst itch as a result of AD in the previous 12 hours. Patients call in to the IVRS twice daily from the evening of the screening visit and be asked the following question, "on a scale of 0 - 10, with 0 being 'no itch' and 10 being the 'worst itch imaginable', how would you rate your worst degree of itch experienced during the previous 12 hours?" Patients are instructed on using the IVRS to record their Pruritus NRS score at the screening visit and are queried for compliance at each following clinic visit. Patients complete the rating scale twice daily through the last study visit.

The baseline NRS is defined as the average of the reported NRSs during right after the screening visit and right before the baseline visit. For post-baseline NRS, The mean weekly NRS is calculated as the average of the reported daily NRS within the week (prorated mean).

### C. Safety Assessment

Safety was assessed throughout the study by monitoring Adverse Events and Serious Adverse Events.

An Adverse Event (AE) is any untoward medical occurrence in a subject or clinical investigation subject administered a pharmaceutical product. An AE can, therefore, be any unfavorable and unintended sign (including abnormal laboratory finding), symptom, or disease temporally associated with the use of a medicinal product, whether or not considered related to the medicinal (investigational) product. AEs also include: any worsening (i.e., any clinically significant change in frequency and/or intensity) of a pre-existing condition that is temporally associated with the use of the study drug; abnormal laboratory findings considered by the Investigator to be clinically significant; and any untoward medical occurrence.

A Serious Adverse Event (SAE) is any untoward medical occurrence that at any dose results in death; is life-threatening; requires in-patient hospitalization or prolongation of existing hospitalization; results in persistent or significant disability/ incapacity; is a congenital anomaly/ birth defect; or is an important medical event.

In addition, laboratory safety variables, vital sign variables, 12-lead electrocardiography (ECG) variables, and physical examination variables were measured throughout the study.

The clinical laboratory data consists of hematology, blood chemistry and urinalysis. Blood samples for hematology testing were collected at every study visit; blood samples for serum chemistry testing and urine samples for urinalysis were collected to measure overall patient health at screening, day 1/ baseline (pre-dose), day 8, day 15, day 29, day 36, day 57, day 85 (end-of-study) or early termination if subject is discontinued from the study.

Vital sign parameters include respiratory rate (bpm), pulse rate (bpm), systolic and diastolic blood pressure (mmHg) and body temperature (°C). Vital signs were collected (pre-dose, on dosing days) at screening and day 1/baseline, and days 4, 8, 15, 22, 25, 29, 36, and 85 (end of study) or early termination. Vital signs were taken at 1 and 2 hours post-injection following the study drug dose on days 1, 8, 15, and 22.

12-Lead ECG parameters include: Ventricular HR, PR interval, QRS interval, corrected QT interval (QTcF=QT/[RR^{0.33}] and QTcB=QT/[RR^{0.5}]) ECG status: normal, abnormal not clinical significant or abnormal clinical significant. A standard 12-lead ECG was performed at screening, day 29, and day 85 (end of study) or early termination.

A thorough and complete physical examination was performed at screening, day 29, and day 85 (end of study) or early termination.

### D. Data Analysis

### 1. Analyses of Exploratory Efficacy Variables

All categorical variables were analyzed using the Fisher's Exact test with nominal p-value and confidence intervals reported. All continuous variables were analyzed by the ANalysis of COVAriance (ANCOVA). Unless otherwise specified, assessments of changes from baseline and construction of confidence intervals for continuous measures were based on an ANCOVA model which includes treatment as the main factor and baseline value as covariates. Point estimate and 95% CI of the difference in adjusted mean change from baseline between two treatment groups are provided. Missing values were imputed by the last observation carried forward (LOCF) approach. In the event that the model assumptions were not warranted, the Rank-based analysis of covariates was used. Correlation analyses were performed using Spearman's correlation coefficient.

### 2. Analysis of Safety Data

The safety analysis is based on the reported AEs, clinical laboratory evaluations, vital signs, and 12-lead ECG. Thresholds for Potentially Clinically Significant Values (PCSV) in laboratory variables, vital signs and ECG are defined in SAP. The time interval to detect any event or abnormality is between the infusion of study medication and end of study. Data collected outside this interval are excluded from the calculation of descriptive statistics and identification of abnormalities for laboratory evaluations, vital sign and ECG.

### E. Results

As noted above, patients were treated either with 150 mg or 300 mg subcutaneous mAb1 once a week for four weeks, or with placebo. Except for a higher age at diagnosis in the 300mg treatment group, demographic and clinical characteristics were generally similar among treatments (Table 8). The study population was primarily male (62.2%), white (94.6%), with a mean age of 43.6 (15.4) years. Of the 37 patients, 31 (83.8%) completed treatments and 25 (67.6%) completed the whole study. The most frequent reason for withdrawal was lack of efficacy (4 placebo patients and 1 in each treatment group). There were no withdrawals due to adverse events with the administered mAb1.

The baseline and exploratory efficacy results obtained from the study are summarized in Tables 9 - 14.

**Table 8: Summary of Baseline Characteristics - all values represented as Mean (SD)**

| | | **mAb1** | | | |
|---|---|---|---|---|---|
| | **Placebo** | **150 mg** | **300 mg** | **All Doses Combined** | **All Subjects Combined** |
| No. Patients | 10 | 14 | 13 | 27 | 37 |
| Age, years, mean (SD) | 46.0 (16.2) | 40.6 (11.7) | 45.0 (18.6) | 42.7 (15.3) | 43.6 (15.4) |

| Gender, n (%) | | | | | |
|---|---|---|---|---|---|
| Male | 8 (80.0) | 7 (50.0) | 8 (61.5) | 15 (55.6) | 23 (62.2) |
| Female | 2 (20.0) | 7 (50.0) | 5 (38.5) | 12 (44.4) | 14 (37.8) |
| Race, n (%) | | | | | |
| White | 9 (90.0) | 14 (100) | 12 (92.3) | 26 (96.3) | 35 (94.6) |
| Black/African-American | 0 | 0 | 1 (7.7) | 1 (3.7) | 1 (2.7) |
| Other | 1 (10.0) | 0 | 0 | 0 | 1 (2.7) |
| BMI, kg/m² mean (SD) | 25.0 (4.6) | 24.7 (2.8) | 25.3 (3.5) | 25.0 (3.1) | 25.0 (3.5) |
| Chronic Atopic Dermatitis Diagnosis Age | 5.8 (10.50) | 5.9 (11.92) | 16.2 (23.79) | 10.9 (18.98) | 9.5 (17.11) |
| BSA | 45.5 (26.43) | 51.4 (29.98) | 49.0 (24.08) | 50.2 (26.80) | 48.9 (26.42) |
| EASI Score | 25.6 (13.73) | 32.6 (18.56) | 25.9 (13.38) | 29.4 (16.32) | 28.3 (15.56) |
| IGA Score | 3.9 (0.74) | 3.9 (0.73) | 3.3 (0.48) | 3.6 (0.69) | 3.7 (0.70) |
| NRS Score | 5.8 (1.92) | 5.6 (1.87) | 5.4 (1.69) | 5.5 (1.75) | 5.6 (1.78) |
| SCORAD Score | 65.3 (11.09) | 66.2 (17.07) | 58.6 (13.93) | 62.6 (15.81) | 63.3 (14.59) |
| Pruritus 5-D Score | 17.8 (3.94) | 18.9 (3.05) | 18.1 (3.99) | 18.5 (3.49) | 18.3 (3.58) |
| CCL17, ng/mL, mean (SD) | 7001 (2669.8) | 9162.2 (4851.7) | 4601.4 (1957.3) | 6881.8 (2280.4) | 6914.9 (2001.3) |

**Table 9: Summary of Percentage and Absolute Change in IGA Score from Baseline - all values represented as Mean (SD)**

| | | **mAb1** | | |
|---|---|---|---|---|
| | **Placebo** | **150 mg** | **300 mg** | **All Doses Combined** |
| No. Patients | 10 | 14 | 13 | 27 |
| Baseline IGA Score | 3.9 (0.74) | 3.9 (0.73) | 3.3 (0.48) | 3.6 (0.69) |
| Day **4** IGA Score | 3.9 (0.74) | 3.9 (0.73) | 3.3 (0.48) | 3.6 (0.69) |
| % Change from Baseline to Day **4** | 0.0 (0.00) | 0.0 (0.00) | 0.0 (0.00) | 0.0 (0.00) |
| Absolute change from Baseline to Day **4** | 0.0 (0.00) | 0.0 (0.00) | 0.0 (0.00) | 0.0 (0.00) |
| Day **8** IGA Score | 3.9 (0.60) | 3.6 (0.93) | 3.2 (0.55) | 3.4 (0.80) |
| % Change from Baseline to Day **8** | 4.6 (18.22) | -8.0 (13.46) | -4.5 (11.08) | -6.3 (12.27) |
| Absolute change from Baseline to Day **8** | 0.1 (0.60) | -0.3 (0.47) | -0.2 (0.38) | -0.2 (0.42) |
| Day **15** IGA Score | 4.0 (0.71) | 2.9 (1.19) | 2.8 (0.83) | 2.8 (1.01) |
| % Change from Baseline to Day **15** | 9.3 (29.89) | -27.2 (18.24) | -16.7 (21.25) | -21.9 (20.13) |
| Absolute change from Baseline to Day **15** | 0.2 (0.97) | -1.0 (0.71) | -0.5 (0.66) | -0.8 (0.71) |
| Day **22** IGA Score | 3.5 (0.55) | 2.7 (0.89) | 2.2 (0.90) | 2.4 (0.91) |
| % Change from Baseline to Day **22** | -2.8 (21.52) | -32.8 (11.68) | -34.6 (28.23) | -33.7 (21.49) |
| Absolute change from Baseline to Day **22** | -0.2 (0.75) | -1.3 (0.45) | -1.2 (0.90) | -1.2 (0.71) |
| Day **25** IGA Score | 3.5 (0.55) | 2.6 (1.00) | 2.0 (1.00) | 2.3 (1.02) |
| % Change from Baseline to Day **25** | -1.4 (28.59) | -34.4 (18.73) | -39.7 (29.30) | -37.2 (24.44) |
| Absolute change from Baseline to Day **25** | -0.2 (0.98) | -1.3 (0.78) | -1.3 (0.95) | -1.3 (0.85) |
| Day **29** IGA Score | 3.3 (0.52) | 2.2 (0.94) | 2.1 (0.95) | 2.1 (0.93) |
| % Change from Baseline to Day **29** | -8.3 (12.91) | -44.4 (19.86) | -37.8 (26.05) | -41.0 (23.05) |
| Absolute change from Baseline to Day **29** | -0.3 (0.52) | -1.8 (0.87) | -1.2 (0.83) | -1.5 (0.87) |
| Day **36** IGA Score | 3.5 (0.58) | 2.0 (0.74) | 2.1 (0.35) | 2.1 (0.60) |
| % Change from Baseline to Day **36** | -6.3 (12.50) | -48.9 (15.51) | -36.5 (8.84) | -43.9 (14.39) |
| Absolute change from Baseline to Day **36** | -0.3 (0.50) | -1.9 (0.67) | -1.3 (0.46) | -1.7 (0.67) |
| Day **43** IGA Score | 3.0 (1.10) | 2.2 (1.03) | 1.7 (0.79) | 2.0 (0.93) |
| % Change from Baseline to Day **43** | -20.0 (29.15) | -45.1 (23.40) | -47.7 (22.39) | -46.4 (22.44) |
| Absolute change from Baseline to Day **43** | -0.8 (1.17) | -1.8 (0.87) | -1.5 (0.69) | -1.7 (0.78) |
| Day **50** IGA Score | 3.2 (1.48) | 2.3 (1.14) | 1.6 (0.67) | 2.0 (0.98) |
| % Change from Baseline to Day **50** | -10.0 (37.91) | -42.1 (25.51) | -50.0 (21.08) | -45.9 (23.32) |
| Absolute change from Baseline to Day **50** | -0.4 (1.52) | -1.7 (1.07) | -1.6 (0.67) | -1.7 (0.88) |
| Day **57** IGA Score | 3.5 (0.58) | 2.2 (1.01) | 1.7 (0.79) | 2.0 (0.93) |
| % Change from Baseline to Day **57** | 2.1 (23.94) | -42.6 (24.94) | -47.0 (23.65) | -44.6 (23.93) |
| Absolute change from Baseline to Day **57** | 0.0 (0.82) | -1.7 (1.03) | -1.5 (0.82) | -1.6 (0.92) |
| Day **64** IGA Score | 3.2 (0.45) | 2.5 (1.13) | 1.7 (0.65) | 2.1 (0.97) |
| % Change from Baseline to Day **64** | -10.0 (13.69) | -36.8 (27.24) | -47.0 (18.36) | -41.9 (23..26) |
| Absolute change from Baseline to Day **64** | -0.4 (0.55) | -1.5 (1.04) | -1.5 (0.69) | -1.5 (0.86) |
| Day **71** IGA Score | 3.0 (.071) | 2.8 (0.87) | 1.6 (0.90) | 2.2 (1.07) |
| % Change from Baseline to Day **71** | -16.7 (15.59) | -25.6 (31.74) | -52.8 (26.67) | -39.8 (31.72) |
| Absolute change from Baseline to Day **71** | -0.6 (0.55) | -1.2 (1.33) | -1.8 (0.87) | -1.5 (1.12) |
| Day **85** IGA Score | 3.3 (0.96) | 2.8 (1.08) | 2.2 (0.94) | 2.5 (1.04) |
| % Change from Baseline to Day **85** | -6.3 (29.95) | -26.5 (35.84) | -33.3 (29.09) | -30.1 (31.92) |
| Absolute change from Baseline to Day **85** | -0.3 (0.96) | -1.2 (1.33) | -1.1 (0.90) | -1.1 (1.10) |

**Table 10: Summary of Percentage and Absolute Change in EASI Score from Baseline - all values represented as Mean (SD)**

| | | **mAb1** | | |
|---|---|---|---|---|
| | **Placebo** | **150 mg** | **300 mg** | **All Doses Combined** |
| No. Patients | 10 | 14 | 13 | 27 |
| Baseline EASI Score | 25.6 (13.73) | 32.6 (18.56) | 25.9 (13.38) | 29.4 (16.32) |
| Day **15** EASI Score | 31.4 (22.59) | 20.6 (16.87) | 14.1 (9.62) | 17.5 (14.00) |
| % Change from Baseline to Day **15** | 23.9 (79.60) | -35.6 (28.97) | -47.2 (20.13) | -41.2 (25.32) |
| Absolute change from Baseline to Day **15** | 5.7 (17.21) | -11.9 (14.03) | -11.8 (7.53) | -11.9 (11.16) |
| Day **29** EASI Score | 21.4 (15.36) | 12.7 (13.52) | 11.7 (14.25) | 12.2 (13.62) |
| % Change from Baseline to Day **29** | -13.1 (15.14) | -61.5 (26.54) | -63.1 (29.00) | -62.3 (27.27) |
| Absolute change from Baseline to Day **29** | -2.2 (5.14) | -19.6 (15.59) | -14.2 (8.00) | -16.8 (12.29) |
| Day **36** EASI Score | 13.9 (2.11) | 8.7 (10.94) | 10.6 (11.30) | 9.4 (10.83) |
| % Change from Baseline to Day **36** | -10.7 (19.60) | -74.7 (20.64) | -58.9 (25.75) | -68.3 (23.54) |
| Absolute change from Baseline to Day **36** | -1.9 (3.08) | -23.7 (16.13) | -12.9 (8.44) | -19.4 (14.36) |
| Day **43** EASI Score | 13.9 (5.85) | 9.5 (12.71) | 8.6 (9.96) | 9.0 (11.23) |
| % Change from Baseline to Day **43** | -29.1 (27.93) | -71.3 (25.68) | -66.7 (25.72) | -69.1 (25.22) |
| Absolute change from Baseline to Day **43** | -6.3 (5.61) | -22.8 (16.59) | -14.9 (8.88) | -19.0 (13.78) |
| Day **57** EASI Score | 14.1 (12.14) | 12.1 (14.93) | 8.2 (10.22) | 10.3 (12.87) |
| % Change from Baseline to Day **57** | -15.2 (78.20) | -63.3 (34.42) | -65.5 (27.26) | -64.3 (30.70) |
| Absolute change from Baseline to Day **57** | -4.7 (15.74) | -21.6 (19.92) | -15.3 (10.72) | -18.7 (16.36) |
| Day **71** EASI Score | 13.3 (8.00) | 13.7 (12.01) | 7.2 (8.61) | 10.5 (10.75) |
| % Change from Baseline to Day **71** | -42.3 (29.40) | -56.8 (36.08) | -73.7 (21.83) | -65.3 (30.41) |
| Absolute change from Baseline to Day **71** | -12.2 (10.91) | -18.6 (16.96) | -18.9 (11.33) | -18.8 (14.11) |
| Day **85** EASI Score | 10.1 (3.73) | 15.9 (17.57) | 7.9 (8.31) | 11.8 (13.85) |
| % Change from Baseline to Day **85** | -41.6 (33.96) | -51.2 (44.04) | -68.6 (25.00) | -60.3 (35.68) |
| Absolute change from Baseline to Day **85** | -10.5 (11.97) | -18.1 (20.78) | -15.5 (8.12) | -16.7 (15.20) |

**Table 11: Summary of Percentage and Absolute Change in BSA Score from Baseline - all values represented as Mean (SD)**

| | | **mAb1** | | |
|---|---|---|---|---|
| | **Placebo** | **150 mg** | **300 mg** | **All Doses Combined** |
| No. Patients | 10 | 14 | 13 | 27 |
| Baseline BSA Score | 45.5 (26.43) | 51.4 (29.98) | 49.0 (24.08) | 50.2 (26.80) |
| Day **15** BSA Score | 42.8 (26.62) | 42.3 (27.46) | 38.7 (24.88) | 40.6 (25.81) |
| % Change from Baseline to Day **15** | -3.0 (14.43) | -16.6 (24.86) | -26.4 (23.61) | -21.3 (24.30) |
| Absolute change from Baseline to Day **15** | -2.4 (6.25) | -9.1 (17.98) | -10.3 (9.10) | -9.7 (14.15) |
| Day **29** BSA Score | 34.3 (25.78) | 28.8 (31.01) | 36.0 (29.38) | 32.6 (29.76) |
| % Change from Baseline to Day **29** | -2.5 (22.46) | -44.7 (41.65) | -35.6 (37.02) | -40.0 (38.76) |
| Absolute change from Baseline to Day **29** | -0.5 (10.41) | -22.0 (30.09) | -13.0 (13.81) | -17.3 (23.05) |
| Day **36** BSA Score | 21.0 (5.48) | 25.2 (25.27) | 29.5 (29.51) | 26.9 (26.37) |
| % Change from Baseline to Day **36** | 10.1 (37.06) | -51.5 (35.29) | -38.6 (41.01) | -46.4 (37.18) |
| Absolute change from Baseline to Day **36** | 1.3 (7.23) | -25.7 (27.96) | -11.5 (14.60) | -20.0 (24.12) |
| Day **43** BSA Score | 29.0 (19.79) | 27.8 (31.69) | 28.9 (25.09) | 28.3 (28.08) |
| % Change from Baseline to Day **43** | -4.2 (31.32) | -46.4 (47.42) | -44.4 (32.77) | -45.4 (40.17) |
| Absolute change from Baseline to Day **43** | -3.2 (8.35) | -23.1 (32.26) | -16.4 (10.04) | -19.9 (24.04) |
| Day **57** BSA Score | 27.3 (28.19) | 31.2 (31.48) | 28.3 (22.40) | 29.8 (27.15) |
| % Change from Baseline to Day **57** | -31.6 (31.63) | -40.5 (52.06) | -42.7 (34.66) | -41.5 (44.01) |
| Absolute change from Baseline to Day **57** | -11.0 (20.58) | -22.6 (34.40) | -17.0 (13.33) | -20.0 (26.51) |
| Day **71** BSA Score | 26.6 (20.63) | 30.6 (24.59) | 23.2 (20.72) | 26.9 (22.56) |
| % Change from Baseline to Day **71** | -27.9 (35.30) | -38.3 (44.29) | -55.7 (26.21) | -47.0 (36.69) |
| Absolute change from Baseline to Day **71** | -12.2 (23.53) | -20.3 (26.80) | -26.2 (17.24) | -23.2 (22.24) |
| Day **85** BSA Score | 20.0 (14.00) | 32.2 (32.71) | 18.1 (14.85) | 24.8 (25.46) |
| % Change from Baseline to Day **85** | -41.5 (6.32) | -38.5 (54.33) | -62.6 (19.64) | -51.1 (41.06) |
| Absolute change from Baseline to Day **85** | -16.3 (15.88) | -22.2 (36.78) | -27.2 (12.77) | -24.8 (26.51) |

**Table 12: Summary of Percentage and Absolute Change in SCORAD Score from Baseline - all values represented as Mean (SD)**

| | | **mAb1** | | |
|---|---|---|---|---|
| | **Placebo** | **150 mg** | **300 mg** | **All Doses Combined** |
| No. Patients | 10 | 14 | 13 | 27 |
| Baseline SCORAD score | 65.3 (11.09) | 66.2 (17.07) | 58.6 (13.93) | 62.6 (15.81) |
| Day **15** SCORAD score | 68.9 (17.01) | 52.5 (18.61) | 41.4 (13.78) | 47.1 (17.01) |
| % Change from Baseline to Day **15** | 8.1 (22.20) | -21.0 (15.53) | -29.4 (14.80) | -25.1 (15.49) |
| Absolute change from Baseline to Day **15** | 5.0 (13.08) | -13.7 (10.94) | -17.3 (9.27) | -15.4 (10.14) |
| Day **29** SCORAD score | 56.2 (17.51) | 38.8 (17.39) | 32.9 (20.53) | 35.7 (18.94) |
| % Change from Baseline to Day **29** | -10.1 (17.58) | -38.9 (19.00) | -46.5 (26.30) | -42.9 (22.94) |
| Absolute change from Baseline to Day **29** | -5.5 (11.20) | -24.3 (13.06) | -25.8 (15.03) | -25.1 (13.85) |
| Day **36** SCORAD score | 53.8 (8.97) | 28.6 (14.79) | 34.0 (12.78) | 30.8 (13.93) |
| % Change from Baseline to Day **36** | -8.9 (8.74) | -54.7 (17.10) | -44.6 (15.77) | -50.7 (16.93) |
| Absolute change from Baseline to Day **36** | -5.4 (5.33) | -34.5 (14.35) | -27.2 (10.64) | -31.6 (13.20) |
| Day **43** SCORAD score | 46.4 (10.88) | 31.3 (17.18) | 25.9 (14.30) | 28.7 (15.75) |
| % Change from Baseline to Day **43** | -25.3 (15.86) | -50.3 (22.09) | -55.9 (18.41) | -53.0 (20.15) |
| Absolute change from Baseline to Day **43** | -16.6 (10.29) | -31.8 (16.14) | -30.8 (10.69) | -31.3 (13.15) |
| Day **57** SCORAD score | 49.3 (22.19) | 36.2 (21.67) | 27.6 (15.77) | 32.3 (19.29) |
| % Change from Baseline to Day **57** | -19.0 (33.04) | -44.4 (27.74) | -49.9 (24.92) | -46.9 (26.06) |
| Absolute change from Baseline to Day **57** | -11.2 (20.24) | -29.0 (19.89) | -29.1 (15.70) | -29.0 (17.71) |
| Day **71** SCORAD score | 46.4 (14.80) | 41.9 (17.40) | 28.6 (18.83) | 35.3 (18.98) |
| % Change from Baseline to Day **71** | -27.5 (18.86) | -34.0 (23.47) | -53.4 (23.21) | -43.7 (24.88) |
| Absolute change from Baseline to Day **71** | -17.2 (11.67) | -21.2 (14.43) | -29.9 (12.18) | -25.5 (13.78) |
| Day **85** SCORAD score | 48.8 (21.01) | 42.3 (21.07) | 31.2 (19.86) | 36.5 (20.77) |
| % Change from Baseline to Day **85** | -19.7 (37.22) | -33.4 (26.47) | -48.4 (27.53) | -41.2 (27.49) |
| Absolute change from Baseline to Day **85** | -12.8 (22.14) | -22.3 (20.81) | -25.8 (12.96) | -24.1 (16.85) |

**Table 13: Summary of Percentage and Absolute Change in 5-D Pruritus Scale from Baseline - all values represented as Mean (SD)**

| | | **mAb1** | | |
|---|---|---|---|---|
| | **Placebo** | **150 mg** | **300 mg** | **All Doses Combined** |
| No. Patients | 10 | 14 | 13 | 27 |
| Baseline 5-D Pruritus Scale | 17.8 (3.94) | 18.9 (3.05) | 18.1 (3.99) | 18.5 (3.49) |
| Day **15** 5-D Pruritus Scale | 17.0 (4.61) | 14.3 (4.68) | 13.0 (5.03) | 13.7 (4.8) |
| % Change from Baseline to Day **15** | 3.6 (30.77) | -24.8 (19.73) | -28.2 (19.49) | -26.5 (19.31) |
| Absolute change from Baseline to Day **15** | 0.0 (6.18) | -4.6 (3.97) | -5.1 (4.15) | -4.9 (3.99) |
| Day **29** 5-D Pruritus Scale | 16.7 (3.50) | 12.2 (5.02) | 11.4 (5.97) | 11.8 (5.45) |
| % Change from Baseline to Day **29** | 8.1 (18.85) | -35.4 (20.28) | -37.1 (26.40) | -36.3 (23.30) |
| Absolute change from Baseline to Day **29** | 1.2(3.19) | -6.5 (4.25) | -6.7 (5.57) | -6.6 (4.91) |
| Day **43** 5-D Pruritus Scale | 14.5 (2.17) | 11.8 (4.43) | 10.0 (5.44) | 11.0 (4.91) |
| % Change from Baseline to Day **43** | -9.4 (23.24) | -37.1 (19.61) | -42.5 (24.75) | -39.7 (21.86) |
| Absolute change from Baseline to Day **43** | -2.3 (5.20) | -7.0 (3.95) | -7.5 (5.26) | -7.2 (4.52) |
| Day **57** 5-D Pruritus Scale | 14.5 (2.74) | 13.4 (5.45) | 11.2 (5.40) | 12.4 (5.43) |
| % Change from Baseline to Day **57** | -7.8 (28.55) | -29.6 (25.38) | -36.2 (22.7) | -32.6 (23.90) |
| Absolute change from Baseline to Day **57** | -2.0 (5.14) | -5.5 (4.75) | -6.3 (4.71) | -5.9 (4.65) |
| Day **71** 5-D Pruritus Scale | 16.4 (2.88) | 16.2 (5.47) | 12.3 (5.66) | 14.3 (5.79) |
| % Change from Baseline to Day **71** | 8.1 (22.85) | -15.4 (21.50) | -31.6 (24.97) | -23.5 (24.25) |
| Absolute change from Baseline to Day **71** | 1.0 (3.24) | -2.7 (3.70) | -5.6 (4.87) | -4.1 (4.48) |
| Day **85** 5-D Pruritus Scale | 17.5 (3.00) | 15.3 (4.92) | 14.0 (5.83) | 14.6 (5.33) |
| % Change from Baseline to Day **85** | 20.6 (34.03) | -19.1 (23.10) | -21.4 (26.23) | -20.3 (24.25) |
| Absolute change from Baseline to Day **85** | 2.5 (5.00) | -3.7 (4.50) | -3.7 (4.66) | -3.7 (4.48) |

**Table 14: Summary of Percentage and Absolute Change in Average Weekly NRS Score from Baseline - all values represented as Mean (SD)**

| | | **mAb1** | | |
|---|---|---|---|---|
| | **Placebo** | **150 mg** | **300 mg** | **All Doses Combined** |
| No. Patients | 10 | 14 | 13 | 27 |
| Baseline NRS Score | 5.8 (1.92) | 5.6 (1.87) | 5.4 (1.69) | 5.5 (1.75) |
| Week **1** NRS Score | 5.5 (1.46) | 5.1 (1.88) | 4.3 (1.59) | 4.7 (1.75) |
| % Change from Baseline to Week **1** | -2.9 (14.99) | -9.1 (15.88) | -16.5 (28.61) | -12.8 (22.98) |
| Absolute change from Baseline to Week **1** | -0.3 (0.99) | -0.5 (0.93) | -1.1 (1.76) | -0.8 (1.42) |
| Week **2** NRS Score | 5.4 (1.42) | 4.2 (2.41) | 3.5 (1.83) | 3.9 (2.13) |
| % Change from Baseline to Week **2** | 1.3 (25.45) | -26.6 (31.37) | -31.9 (31.90) | -29.3 (31.11) |
| Absolute change from Baseline to Week **2** | -0.1 (1.50) | -1.3 (1.67) | -1.9 (2.01) | -1.6 (1.84) |
| Week **3** NRS Score | 5.5 (2.02) | 3.6 (1.80) | 3.2 (1.35) | 3.4 (1.57) |
| % Change from Baseline to Week **3** | 2.9 (16.38) | -35.7 (17.81) | -37.2 (30.15) | -36.4 (24.23) |
| Absolute change from Baseline to Week **3** | 0.2 (0.83) | -1.8 (0.92) | -2.2 (1.87) | -2.0 (1.45) |
| Week **4** NRS Score | 4.5 (1.33) | 3.7 (2.18) | 2.9 (2.18) | 3.3 (2.17) |
| % Change from Baseline to Week **4** | -1.1 (28.49) | -35.0 (25.50) | -45.4 (40.95) | -40.2 (33.78) |
| Absolute change from Baseline to Week **4** | -0.1 (1.35) | -1.8 (1.37) | -2.5 (2.18) | -2.1 (1.81) |
| Week **5** NRS Score | 4.4 (1.68) | 3.2 (2.08) | 2.8 (1.92) | 3.0 (1.96) |
| % Change from Baseline to Week **5** | -5.4 (34.34) | -44.6 (26.15) | -43.1 (37.67) | -43.9 (31.42) |
| Absolute change from Baseline to Week **5** | -0.3 (1.60) | -2.3 (1.54) | -2.3 (2.10) | -2.3 (1.79) |
| Week **6** NRS Score | 3.8 (1.89) | 3.2 (1.98) | 2.7 (2.08) | 3.0 (2.00) |
| % Change from Baseline to Week **6** | -19.0 (35.26) | -43.6 (23.27) | -49.7 (33.29) | -46.3 (27.72) |
| Absolute change from Baseline to Week **6** | -0.9 (1.72) | -2.3 (1.37) | -2.6 (1.98) | -2.4 (1.64) |
| Week **7** NRS Score | 3.8 (2.18) | 3.2 (2.52) | 2.3 (1.84) | 2.7 (2.21) |
| % Change from Baseline to Week **7** | -17.3 (42.69) | -45.0 (33.78) | -55.2 (35.03) | -49.9 (33.99) |
| Absolute change from Baseline to Week **7** | -0.8 (2.06) | -2.3 (1.90) | -3.1 (2.13) | -2.7 (2.00) |
| Week **8** NRS Score | 3.5 (2.01) | 3.2 (2.10) | 2.5 (1.93) | 2.9 (2.00) |
| % Change from Baseline to Week **8** | -22.1 (38.80) | -43.0 (35.08) | -50.7 (34.39) | -46.7 (34.18) |
| Absolute change from Baseline to Week **8** | -1.0 (1.90) | -2.3 (1.84) | -2.8 (2.18) | -2.6 (1.98) |
| Week **9** NRS Score | 3.7 (1.93) | 3.6 (2.12) | 2.5 (2.34) | 3.1 (2.25) |
| % Change from Baseline to Week **9** | -20.7 (34.06) | -34.2 (36.64) | -52.9 (39.71) | -43.1 (38.47) |
| Absolute change from Baseline to Week **9** | -0.9 (1.65) | -1.8 (1.74) | -2.8 (2.25) | -2.3 (2.02) |
| Week **10** NRS Score | 3.7 (1.86) | 4.3 (2.39) | 3.1 (2.38) | 3.7 (2.40) |
| % Change from Baseline to Week **10** | -19.7 (31.94) | -25.1 (32.66) | -43.6 (38.72) | -34.4 (36.22) |
| Absolute change from Baseline to Week **10** | -0.8 (1.54) | -1.2 (1.30) | -2.3 (2.10) | -1.7 (1.80) |
| Week **11** NRS Score | 3.9 (2.18) | 4.9 (2.45) | 2.9 (1.92) | 3.9 (2.39) |
| % Change from Baseline to Week **11** | -17.1 (37.62) | -10.1 (43.91) | -43.4 (37.56) | -26.0 (43.46) |
| Absolute change from Baseline to Week **11** | -0.7 (1.77) | -0.5 (1.85) | -2.2 (1.93) | -1.4 (2.03) |
| Week **12** NRS Score | 3.9 (1.88) | 4.3 (2.68) | 3.4 (2.59) | 3.9 (2.60) |
| % Change from Baseline to Week **12** | -13.6 (29.53) | -21.1 (39.41) | -38.0 (35.65) | -29.1 (37.65) |
| Absolute change from Baseline to Week **12** | -0.5 (1.35) | -1.1 (2.00) | -1.9 (1.93) | -1.5 (1.96) |

### F. Conclusions

Subcutaneous administration of an anti-IL-4R antibody (mAb1) to adult patients with moderate-to-severe atopic dermatitis was generally safe and well tolerated after 4 weekly doses of 150 or 300 mg with an adverse event (AE) rate similar to placebo and no dose limiting toxicities or serious AEs. The most common AEs with mAb1 were nasopharyngitis and headache. The mAb1 rapidly (by Day 8) reduced pruritus and improved skin disease in a dose-dependent fashion. Administration of mAb1 at 150 and 300 mg resulted in significant improvement in IGA, EASI, BSA, SCORAD and NRS pruritus as early as Day 8 through day 85 in both mean and absolute and percent change, as compared to baseline (see Tables 9 - 14). In the 300mg arm at day 29, proportion of patients who achieved an EASI50 response was 71.4% vs 18.8% for placebo (p=0.0025) and NRS pruritus score decreased by 45.4% vs 18.6% for placebo (p=0.0016).The effect was sustained through day 85 for EASI50 and day 75 for NRS pruritus. For the 300mg treatment group, the difference from placebo was significant for an additional 6 weeks after end of treatment period. The mAb significantly improved other clinical outcomes at day 29, mean % change IGA (p=0.0002), EASI (p<0.0001), BSA (p=0.0037), and 5D pruritus (p<0.0001). These improvements were generally observed by Day 8 and persisted after end of treatment. No rebound phenomena were observed after end of treatment.

The results shown in this Example therefore demonstrate that mAb1 is safe and effective for the treatment of atopic dermatitis.

### Example 8: Treatment of patients with moderate-to-severe atopic dermatitis with anti-IL-4R antibody: analysis of pooled phase 1b studies

AD efficacy parameters were measured and pooled for analysis from two separate clinical trials in patients with moderate-to-severe AD. "Study A" was a 12-week, double-blind, randomized, placebo-controlled, sequential ascending dose study to assess the safety and tolerability of administered anti-IL-4R antibody (mAb1) in patients with atopic dermatitis. The treatment period was 4 weeks with patients being followed for 8 weeks after the end of the treatment period. Patients were randomized in a 4:1 ratio to receive mAb1 or placebo in each of the three ascending dose cohorts (75 mg, 150 mg, or 300 mg). The study consisted of a screening period (day -14 to day -3), a treatment period (day 1 through day 29), and a follow-up period (day 29 through day 85). During the treatment period, patients were seen in the clinic once weekly for safety, laboratory and clinical effect assessments on days 1, 4, 8, 15, 22, 25 and 29 (week 4). Patients received a dose of mAb1 or placebo on days 1, 8, 15 and 22. The end of the treatment period study was on day 29 (week 4). Patients were monitored at the study site for 6 hours after the injection (of mAb1 or placebo) on day 1, and for 3 hours after the injection on days 8, 15 and 22. During the follow-up period, patients were seen in the clinic for follow-up assessments at days 36, 43, 50, 57, 64, 71, and 85 (end of study visit).

"Study B" was a 12-week, double-blind, randomized, placebo-controlled, sequential ascending, repeated-dose study in patients with moderate-to-severe AD. AD subjects were administered 150mg or 300 mg of mAb1, or placebo on days 1, 8, 15 and 22 of the study (four weekly doses) (See Example 3 herein). All administrations for both studies were subcutaneous.

The patient inclusion criteria for the studies were: (1) should be male or female ≥ 18 years; (2) have chronic atopic dermatitis for 3 years; (3) have EASI ≥ 12; (4) IGA ≥ 3; (5) ≥ 15% BSA of AD involvement (in the US) or ≥ 10% BSA of AD involvement (ex-US); and (6) history of inadequate response to stable regimen of topical corticosteroids (TCS) or calcineurin inhibitors.

The patient exclusion criteria for the study were: (1) WBC < 3.5 × 10³/µl; (2) platelets < 125 × 10³/µl; (3) neutrophils < 1.75 × 10³/µl; (4) AST/ALT > 1.5x ULN; (5) positive for hepatitis B or hepatitis C; and (6) treatment with TCS or calcineurin inhibitors within 1 week of baseline.

The primary endpoint of the studies was to monitor incidence of treatment-emergent adverse events (TEAEs) from baseline through week 12. The exploratory endpoints for efficacy variables were: (i) % achieving an IGA of 0 or 1 through week 4; (ii) % improvement in BSA and EASI from baseline; and (iii) change from baseline in NRS scale.

The efficacy variables IGA, BSA, EASI, SCORAD, 5-D Pruritus scale, and Pruritus NRS rating have been described elsewhere herein (see, e.g., Example 4).

The IGA, BSA, EASI and SCORAD scores were assessed at every clinic visit. Patients underwent 5-D pruritus assessment at the following visits: screening, day 1/baseline (pre-dose), and days 15, 29, 43, 57, 71, and 85 (end of study) or early termination. Patients used the IVRS to record their Pruritus NRS score twice daily through the last study visit.

Baseline for efficacy variable is defined as the last non-missing value on or before the date of randomization. For the patient who has no value on or before his/her randomization date the last non-missing value on or before the date of first dose injection will be used as baseline.

The baseline demographics for the patient population are presented below in Table 15.

**Table 15: Baseline Demographics**

| | Placebo (N=16) | 75 mg (N=8) | 150mg (N=22) | 300mg (N=21) | All Doses (N=51) |
|---|---|---|---|---|---|
| Mean age, years (SD) | 37.4 (17.16) | 35.8 (12.51) | 42.5 (11.37) | 45.4 (15.92) | 42.6 (13.73) |

| Race, n (%) | | | | | |
|---|---|---|---|---|---|
| Caucasian | 13 (81.3%) | 4 (50.0%) | 19 (86.4%) | 16 (76.2%) | 39 (76.5%) |
| Non-Caucasian | 3 (18.7%) | 4 (50.0%) | 3 (13.6%) | 5 (23.8%) | 12 (23.5%) |

| Gender, n (%) | | | | | |
|---|---|---|---|---|---|
| Male | 11 (68.8%) | 6 (75.0%) | 12 (54.5%) | 10 (47.6%) | 28 (54.9%) |
| Female | 5 (31.3%) | 2 (25.0%) | 10 (45.5%) | 11 (52.4%) | 23 (45.1%) |
| Mean BMI, kg/m³ (SD) | 25.69 (5.993) | 26.41 (4.489) | 25.68 (3.991) | 27.71 (8.667) | 26.63 (6.361) |

The mean baseline disease characteristics are given in Table 16.

**Table 16: Mean Baseline Disease Characteristics**

| | Placebo (N=16) | 75 mg (N=8) | 150mg (N=22) | 300mg (N=21) | All Doses (N=51) |
|---|---|---|---|---|---|
| Duration of chronic AD, years | 31.8 (18.67) | 24.5 (16.95) | 32.1 (15.44) | 30.7 (16.95) | 30.4 (16.19) |
| EASI score | 22.8 (12.02) | 36.9 911.75) | 30.0 (17.00) | 27.4 (11.21) | 30.0 (14.19) |
| IGA score | 3.6 (0.72) | 4.1 (0.35) | 3.9 (0.68) | 3.5 (0.51) | 3.8 (0.62) |
| %BSA of AD | 40.3 (25.77) | 64.4 917.03) | 49.8 (28.75) | 48.2 (22.26) | 51.4 (24.87) |
| 5-D pruritus scale | 16.9 (3.94) | 21.5 (3.55) | 19.0 (2.94) | 18.7 (3.64) | 19.3 (3.41) |
| Pruritus NRS score | 5.8 (1.75) | 7.0 (1.78) | 6.0 (1.82) | 5.7 (1.51) | 6.0 (1.72) |

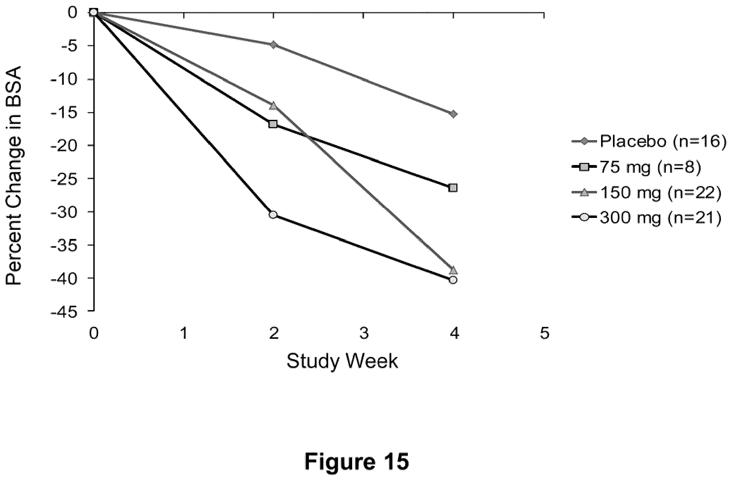

The exploratory efficacy results obtained from the pooled studies are summarized in Tables 17 - 25 and in Figures 15 - 22.

**Table 17: Summary of subjects achieving IGA ≤ 1 at Day 29 and all study visits**

| Number and % subjects with IGA≤ 1 | Placebo (N=16) | 75 mg (N=8) | 150 mg (N=22) | 300 mg (N=21) | All Doses Combined (N=51) |
|---|---|---|---|---|---|
| Week 4, Day 29 | 1 (6.3%) | 0 | 4 (18.2%) | 2 (9.5%) | 6 (11.8%) |
| Day 4 | 0 | 0 | 0 | 0 | 0 |
| Week 1, Day 8 | 0 | 0 | 0 | 0 | 0 |
| Week 2, Day 15 | 0 | 0 | 0 | 1 (4.8%) | 1 (2.0%) |
| Week 3, Day 22 | 0 | 0 | 0 | 2 (9.5%) | 2 (3.9%) |
| Week 3, Daγ 25 | 1 (6.3%) | 0 | 1 (4.5%) | 4 (19.0%) | 5 (9.8%) |
| Week 5, Day 36 | 1 | 0 | 4 (18.2%) | 2 (9.5%) | 6 (11.8%) |
| Week 6, Day 43 | 2 (12.5%) | 0 | 5 (22.7%) | 3 (14.3%) | 8 (15.7%) |
| Week 7, Day 50 | 2 (12.5%) | 0 | 4 (18.2%) | 3 (14.3%) | 7 (13.7%) |
| Week 8, Day 57 | 2 (12.5%0 | 0 | 3 (13.6%) | 5 (23.8%) | 8 (15.7%) |
| Week 9, Day 64 | 1 (6.3%) | 0 | 3 (13.6%) | 4 (19.0%) | 7 (13.7%) |
| Week 10, Day 71 | 1 (6.3%) | 0 | 1 (4.5%) | 5 (23.8%) | 6 (11.8%) |
| Week 12, Day 85 | 1 (6.3%) | 0 | 0 | 3 (14.3%) | 3 (5.9%) |

**Table 18: Summary of Percentage and Absolute Change in BSA Score from Baseline - all values represented as Mean (SD)**

| | | **mAb1** | | | |
|---|---|---|---|---|---|
| | **Placeb o** | **75mg** | **150mg** | **300mg** | **All Doses Combined** |
| No. Patients | 16 | 8 | 22 | 21 | 51 |
| Baseline BSA Score | 40.3 (25.77) | 64.4 (17.03) | 49.8 (28.75) | 48.2 (22.26) | 51.4 (24.87) |
| Day **15** BSA Score | 37.6 (26.61) | 52.3 (12.54) | 40.9 (25.66) | 34.4 (22.66) | 40.0 (23.23) |
| % Change from Baseline to Day **15** | -4.8 (14.80) | -16.8 (15.17) | -13.9 (21.77) | -30.5 (27.09) | -21.4 (24.27) |
| Absolute change from Baseline to Day **15** | -1.7 (5.37) | -12.1 (11.58) | -7.0 (15.07) | -13.9 (14.73) | -10.7 (14.51) |
| Day **29** BSA Score | 31.1 (29.69) | 46.3 (12.42) | 31.1 (28.78) | 31.5 925.33) | 33.8 (25.47) |
| % Change from Baseline to Day **29** | -15.3 (31.02) | -26.4 (16.41) | -38.8 (37.00) | -40.3 (33.78) | -37.4 (32.88) |
| Absolute change from Baseline to Day **29** | -2.1 (10.93) | -18.1 (13.14) | -18.2 (24.61) | -16.7 (16.05) | -17.5 (19.31) |
| Day **36** BSA Score | 25.1 (26.81) | 41.2 (15.59) | 24.9 (24.15) | 26.0 (22.67) | 28.0 (22.70) |
| % Change from Baseline to Day **36** | -13.3 (39.22) | -33.7 (21.53) | -48.6 (32.13) | -44.2 (34.61) | -44.4 (31.41) |
| Absolute change from Baseline to Day **36** | -1.8 (10.33) | -22.4 (15.26) | -24.3 (25.07) | -18.0 (17.82) | -21.6 (20.85) |
| Day **43** BSA Score | 29.9 (27.04) | 48.4 (21.56) | 24.8 (26.36) | 26.2 (21.03) | 29.1 (24.42) |
| % Change from Baseline to Day **43** | -11.0 (39.52) | -29.2 (24.87) | -43.3 (42.81) | -47.2 (30.07) | -42.7 (35.05) |
| Absolute change from Baseline to Day **43** | -2.0 (10.74) | -19.0 (15.63) | -22.2 (29.35) | -19.8 (14.41) | -20.7 (21.52) |
| Day **57** BSA Score | 27.2 (31.12) | 57.5 (23.40) | 31.2 (28.60) | 28.3 (20.11) | 33.7 (26.24) |
| % Change from Baseline to Day **57** | -33.6 (32.95) | -18.7 (23.06) | -37.4 (42.74) | -41.9 (29.38) | -36.6 (35.90) |
| Absolute change from Baseline to Day **57** | -8.3 (16.62) | -12.4 (16.36) | -20.0 (28.38) | -17.6 (13.86) | -18.0 (21.99) |
| Day **71** BSA Score | 27.4 (28.13) | 58.4 (19.79) | 30.7 (24.56) | 23.2 (19.85) | 31.1 (24.32) |
| % Change from Baseline to Day **71** | -29.0 (36.38) | -13.2 (11.92) | -35.7 (37.54) | -52.0 (35.43) | -39.9 (36.13) |
| Absolute change from Baseline to Day **71** | -7.5 (17.71) | -8.5 (8.10) | -18.4 (23.12) | -25.2 (18.53) | -20.1 (20.14) |
| Day **85** BSA Score | 25.1 (27.73) | 58.0 (19.52) | 30.7 (28.38) | 23.6 (17.95) | 30.7 (25.04) |
| % Change from Baseline to Day **85** | -33.4 (32.68) | -16.9 (16.63) | -37.8 (43.59) | -49.0 (37.34) | -40.4 (39.14) |
| Absolute change from Baseline to Day **85** | -8.4 (14.45) | -11.9 (11.45) | -20.6 (29.67) | -22.7 (15.74) | -20.5 (22.31) |

**Table 19: Summary of Percentage and Absolute Change in EASI Score from Baseline - all values represented as Mean (SD)**

| | | **mAb1** | | | |
|---|---|---|---|---|---|
| | **Placeb o** | **75mg** | **150mg** | **300mg** | **All Doses Combined** |
| No. Patients | 16 | 8 | 22 | 21 | 51 |
| Baseline EASI Score | 22.8 (12.02) | 36.9 (11.75) | 30.0 (17.00) | 27.4 (11.21) | 30.0 (14.19) |
| Day **15** EASI Score | 25.4 (20.13) | 26.2 (7.72) | 19.8 (15.05) | 15.4 (8.57) | 19.0 (12.06) |
| % Change from Baseline to Day **15** | 8.7 (66.05) | -26.9 (19.29) | -31.1 (27.24) | -45.1 (19.90) | -36.3 (24.02) |
| Absolute change from Baseline to Day **15** | 2.8 (14.11) | -10.7 (9.83) | -9.7 (12.02) | -12.0 (6.93) | -10.8 (9.67) |
| Day **29** EASI Score | 17.2 (15.11) | 17.7 (6.05) | 13.1 (11.89) | 11.3 (11.84) | 13.1 (11.17) |
| % Change from Baseline to Day **29** | -25.4 (34.98) | -47.0 (21.93) | -55.0 (30.36) | -64.3 (25.83) | -57.7 (27.45) |
| Absolute change from Baseline to Day **29** | -3.6 (7.25) | -19.2 (15.11) | -16.6 (14.58) | -16.1 (7.69) | -16.8 (11.97) |
| Day **36** EASI Score | 13.2 (11.97) | 16.3 (7.74) | 9.4 (10.27) | 10.5 (8.69) | 11.0 (9.42) |
| % Change from Baseline to Day **36** | -28.4 (41.10) | -51.5 (25.53) | -69.6 (22.46) | -61.9 (22.69) | -63.6 (23.41) |
| Absolute change from Baseline to Day **36** | -3.9 (7.94) | -21.5 (17.30) | -20.5 (14.98) | -16.1 (8.23) | -19.0 (13.13) |
| Day **43** EASI Score | 12.9 97.13) | 19.8 (10.41) | 9.6 (11.01) | 9.3 (8.29) | 11.1 (10.32) |
| % Change from Baseline to Day **43** | -33.8 (28.94) | -39.4 (31.87) | -64.2 (33.89) | -66.4 (22.39) | -61.2 (29.98) |
| Absolute change from Baseline to Day **43** | -6.2 (4.71) | -17.0 (19.33) | -19.7 (16.63) | -16.8 (7.84) | -18.0 (13.76) |
| Day **57** EASI Score | 13.0 (11.95) | 27.0 (16.46) | 12.2 (12.88) | 10.4 (9.40) | 13.5 (13.11) |
| % Change from Baseline to Day **57** | -28.7 (62.63) | -24.5 (47.21) | -57.3 (33.38) | -61.1 (24.91) | -54.3 (33.99) |
| Absolute change from Baseline to Day **57** | -5.4 (11.79) | -11.9 (22.95) | -18.4 (17.88) | -15.8 (9.69) | -16.5 (15.81) |
| Day **71** EASI Score | 11.8 (9.22) | 28.3 (13.06) | 13.0 (10.86) | 8.5 (9.21) | 13.1 (12.06) |
| % Change from Baseline to Day **71** | -45.8 (31.06) | -14.5 (41.14) | -54.9 (32.01) | -71.3 (24.14) | -56.8 (34.68) |
| Absolute change from Baseline to Day **71** | -9.6 (8.23) | -9.4 (22.16) | -16.9 (15.41) | -19.1 (9.88) | -16.9 (14.32) |
| Day **85** EASI Score | 9.8 (4.87) | 27.1 (11.99) | 14.2 (14.30) | 10.5 (9.26) | 14.0 (12.77) |
| % Change from Baseline to Day **85** | -44.8 (30.60) | -28.3 (29.69) | -51.3 (37.58) | -63.0 (25.55) | -53.9 (32.86) |
| Absolute change from Baseline to Day **85** | -9.3 (8.01) | -13.4 (18.94) | -16.6 (17.67) | -15.4 (7.57) | -15.7 (13.81) |

**Table 20: Summary of Percentage and Absolute Change in 5-D Pruritus Scale from Baseline - all values represented as Mean (SD)**

| | **mAb1** | | | | |
|---|---|---|---|---|---|
| | **Placeb o** | **75mg** | **150mg** | **300mg** | **All Doses Combined** |
| No. Patients | 16 | 8 | 22 | 21 | 51 |
| Baseline 5-D Pruritus Scale | 16.9 (3.94) | 21.5 (3.55) | 19.0 (2.94) | 18.7 (3.64) | 19.3 (3.41) |
| Day **15** 5-D Pruritus Scale | 15.0 (4.66) | 14.0 (3.55) | 14.0 (4.46) | 12.5 (4.08) | 13.4 (4.15) |
| % Change from Baseline to Day **15** | -5.6 (29.83) | -34.3 (15.43) | -26.6 (19.26) | -32.4 (17.60) | -30.3 (17.95) |
| Absolute change from Baseline to Day **15** | -1.4 (5.55) | -7.5 (3.82) | -5.0 (3.97) | -6.1 (3.93) | -5.9 (3.94) |
| Day **29** 5-D Pruritus Scale | 14.8 93.77) | 14.1 (3.31) | 13.1 (5.03) | 11.0 (4.86) | 12.3 (4.79) |
| % Change from Baseline to Day **29** | -3.9 (20.07) | -33.0 (17.25) | -30.8 (23.71) | -40.8 (21.83) | -35.6 (22.02) |
| Absolute change from Baseline to Day **29** | -0.8 (3.41) | -7.4 (4.47) | -5.9 (4.84) | -7.7 (4.78) | -6.9 (4.73) |
| Day **43** 5-D Pruritus Scale | 13.8 (3.71) | 16.5 (4.54) | 12.1 (4.64) | 10.7 (4.83) | 12.3 (5.04) |
| % Change from Baseline to Day **43** | -10.4 (31.60) | -21.4 (25.01) | -35.0 (22.07) | -40.8 (23.87) | -35.0 (23.86) |
| Absolute change from Baseline to Day **43** | -2.3 (5.25) | -5.0 (5.66) | -6.6 (4.45) | -7.6 (5.04) | -6.8 (4.90) |
| Day **57** 5-D Pruritus Scale | 12.3 (3.35) | 19.9 (3.98) | 13.9 94.75) | 11.6 (5.18) | 14.0 (5.46) |
| % Change from Baseline to Day **57** | -19.0 (25.37) | -9.0 (20.15) | -27.2 (21.28) | -37.2 (21.68) | -28.1 (22.85) |
| Absolute change from Baseline to Day **57** | -3.4 (4.43) | -2.3 (4.46) | -5.1 (4.03) | -6.8 (4.61) | -5.3 (4.49) |
| Day **71** 5-D Pruritus Scale | 13.5 (4.03) | 19.4 (3.51) | 15.3 (4.78) | 12.9 (5.61) | 14.7 (5.36) |
| % Change from Baseline to Day **71** | -11.6 (25.71) | -8.3 (14.91) | -18.9 (19.50) | -31.7 (24.53) | -23.3 (22.58) |
| Absolute change from Baseline to Day **71** | -2.0 (4.12) | -2.0 (3.39) | -3.4 (3.56) | -5.8 (4.70) | -4.3 (4.24) |
| Day **85** 5-D Pruritus Scale | 14.1 (4.48) | 18.6 (1.34) | 15.2 (3.99) | 14.6 (5.26) | 15.3 (4.53) |
| % Change from Baseline to Day **85** | -5.4 (32.44) | -10.0 (22.58) | -18.5 (21.29) | -21.9 (23.41) | -19.0 (22.18) |
| Absolute change from Baseline to Day **85** | -1.2 (5.09) | -2.8 (4.92) | -3.7 (4.04) | -4.1 (4.52) | -3.7 (4.27) |

**Table 21: Summary of Percentage and Absolute Change in Average Weekly NRS Score from Baseline - all values represented as Mean (SD)**

| | | **mAb1** | | | |
|---|---|---|---|---|---|
| | **Placeb o** | **75mg** | **150mg** | **300mg** | **All Doses Combined** |
| No. Patients | 10 | 8 | 22 | 21 | 51 |
| Baseline NRS Score | 5.8 (1.75) | 7.0 (1.78) | 6.0 (1.82) | 5.7 (1.51) | 6.0 (1.72) |
| Week **1** NRS Score | 5.1 (1.73) | 5.2 (2.50) | 5.2 (1.91) | 4.3 (1.52) | 4.8 (1.88) |
| % Change from Baseline to Week **1** | -11.9 (23.13) | -27.3 (20.25) | -12.7 (18.26) | -21.6 (26.03) | -18.8 (22.42) |
| Absolute change from Baseline to Week **1** | -0.8 (1.40) | -1.7 (1.22) | -0.8 (1.30) | -1.4 (1.59) | -1.2 (1.44) |
| Week **2** NRS Score | 4.7 (2.00) | 4.0 (2.36) | 4.5 (2.38) | 3.7 (1.59) | 4.1 (2.07) |
| % Change from Baseline to Week **2** | -14.8 (36.13) | -44.6 (21.90) | -26.9 (29.96) | -33.3 (26.69) | -32.4 (27.63) |
| Absolute change from Baseline to Week **2** | -1.0 (2.16) | -3.0 (1.350 | -1.5 (1.76) | -2.0 (1.71) | -1.9 (1.73) |
| Week **3** NRS Score | 5.0 (2.29) | 3.9 (2.12) | 4.0 (2.12) | 3.3 (1.30) | 3.7 (1.81) |
| % Change from Baseline to Week **3** | -10.2 (33.75) | -45.6 (21.67) | -35.4 (23.84) | -39.4 (25.92) | -38.8 (24.17) |
| Absolute change from Baseline to Week **3** | -0.7 (2.01) | -3.1 (1.30) | -2.0 (1.49) | -2.4 (1.65) | -2.3 (1.55) |
| Week **4** NRS Score | 4.1 (2.03) | 4.1 (1.95) | 3.9 (2.38) | 3.1 (1.84) | 3.6 (2.10) |
| % Change from Baseline to Week **4** | -18.6 (40.12) | -42.3 (22.62) | -36.7 (29.33) | -45.4 (32.89) | -41.3 (29.63) |
| Absolute change from Baseline to Week **4** | -1.2 (2.29) | -2.9 (1.38) | -2.1 (1.85) | -2.6 (1.77) | -2.4 (1.74) |
| Week **5** NRS Score | 4.2 (2.29) | 4.1 (2.03) | 3.5 (2.36) | 3.0 (1.80) | 3.4 (2.09) |
| % Change from Baseline to Week **5** | -18.9 (43.93) | -41.9 (24.53) | -43.4 (30.89) | -44.2 (32.74) | -43.5 (30.09) |
| Absolute change from Baseline to Week **5** | -1.2 (2.43) | -2.9 (1.55) | -2.5 (1.97) | -2.5 (1.92) | -2.6 (1.85) |
| Week **6** NRS Score | 4.0 (2.40) | 4.1 (2.22) | 3.7 (2.38) | 3.0 (1.84) | 3.5 (2.14) |
| % Change from Baseline to Week **6** | -24.9 (42.63) | -42.7 (24.23) | -40.0 (30.52) | -46.9 (28.41) | -43.3 (28.31) |
| Absolute change from Baseline to Week **6** | -1.4 (2.36) | -2.8 (1.44) | -2.2 (1.86) | -2.6 (1.68) | -2.5 (1.71) |
| Week **7** NRS Score | 3.4 (2.59) | 4.4 (2.39) | 3.7 (2.56) | 2.8 (1.78) | 3.4 (2.26) |
| % Change from Baseline to Week **7** | -35.5 (42.70) | -41.3 (21.96) | -40.3 (33.56) | -49.9 (30.73) | -44.5 (30.73) |
| Absolute change from Baseline to Week **7** | -1.9 (2.33) | -2.8 (1.10) | -2.2 (1.90) | -2.8 (1.83) | -2.5 (1.77) |
| Week **8** NRS Score | 3.5 (2.61) | 5.4 (2.40) | 3.7 (2.24) | 3.0 (1.98) | 3.7 (2.24) |
| % Change from Baseline to Week **8** | -33.9 (38.63) | -27.8 (21.17) | -38.2 (33.09) | -45.6 (32.23) | -39.8 (31.29) |
| Absolute change from Baseline to Week **8** | -1.8 (2.19) | -1.9 (1.19) | -2.2 (1.80) | -2.6 (1.99) | -2.3 (1.80) |
| Week **9** NRS Score | 3.6 (2.26) | 5.5 (2.44) | 4.1 (2.10) | 3.0 (2.27) | 3.9 (2.32) |
| % Change from Baseline to Week **9** | -32.8 (35.28) | -26.1 (17.08) | -31.5 (32.14) | -46.2 (36.56) | -36.9 (32.95) |
| Absolute change from Baseline to Week **9** | -1.7 (2.01) | -1.7 (1.02) | -1.8 (1.59) | -2.5 (2.10) | -2.1 (1.77) |
| Week **10** NRS Score | 3.7 (2.51) | 5.3 (2.33) | 4.6 (2.18) | 3.2 (1.99) | 4.1 (2.21) |
| % Change from Baseline to Week **10** | -30.3 (41.78) | -21.7 (24.33) | -24.6 (28.77) | -43.4 (31.24) | -32.5 (30.36) |
| Absolute change from Baseline to Week **10** | -1.6 (2.31) | -1.4 (1.51) | -1.3 (1.37) | -2.4 (1.70) | -1.8 (1.59) |
| Week **11** NRS Score | 2.8 (2.03) | 5.8 (2.11) | 5.0 (2.19) | 3.2 (1.81) | 4.4 (2.23) |
| % Change from Baseline to Week **11** | -40.2 (40.04) | -13.1 (26.33) | -14.2 (36.88) | -41.2 (31.87) | -25.1 (35.60) |
| Absolute change from Baseline to Week **11** | -2.0 (2.26) | -0.9 (1.61) | -0.8 (1.72) | -2.2 (1.64) | -1.4 (1.76) |
| Week **12** NRS Score | 3.5 (1.48) | 5.2 (2.37) | 4.8 (2.47) | 3.5 (2.37) | 4.4 (2.44) |
| % Change from Baseline to Week **12** | -28.9 (29.54) | -25.4 (25.39) | -17.9 (33.42) | -35.5 (33.02) | -25.4 (32.53) |
| Absolute change from Baseline to Week **12** | -1.5 (1.66) | -1.7 (1.50) | -1.0 (1.77) | -1.7 (1.73) | -1.3 (1.71) |

**Table 22: Summary of Percentage and Absolute Change in IGA Score from Baseline - all values represented as Mean (SD)**

| | | **mAb1** | | | |
|---|---|---|---|---|---|
| | **Placeb o** | **75mg** | **150mg** | **300mg** | **All Doses Combined** |
| No. Patients | 16 | 8 | 22 | 21 | 51 |
| Baseline IGA Score | 3.6 (0.72) | 4.1 (0.35) | 3.9 (0.68) | 3.5 (0.51) | 3.8 (0.62) |
| Day **4** IGA Score | 3.6 (0.73) | 4.1 (0.35) | 3.9 (0.71) | 3.3 (0.48) | 3.7 (0.65) |
| % Change from Baseline to Day **4** | -1.6 (6.25) | 0.0 (0.00) | -1.1 (5.33) | -3.6 (8.96) | -2.0 (6.79) |
| Absolute change from Baseline to Day **4** | -0.1 (0.25) | 0.0 (0.00) | 0.0 (0.21) | -0.1 (0.36) | -0.1 (0.27) |
| Day **8** IGA Score | 3.3 (0.90) | 4.0 (0.00) | 3.6 (0.85) | 3.1 (0.54) | 3.5 (0.73) |
| % Change from Baseline to Day **8** | -5.6 (21.28) | -2.5 (7.07) | -7.3 (12.55) | -10.3 (13.67) | -7.8 (12.46) |
| Absolute change from Baseline to Day **8** | -0.2 | -0.1 | -0.3 | -0.4 | -0.3 (0.46) |
| | (0.68) | (0.35) | (0.46) | (0.50) | |
| Day **15** IGA Score | 3.4 (0.99) | 3.6 (0.52) | 3.0 (0.97) | 2.9 (0.70) | 3.1 (0.83) |
| % Change from Baseline to Day **15** | -2.8 (28.98) | -11.3 (16.20) | -23.7 (16.69) | -16.3 (18.16) | -18.5 (17.55) |
| Absolute change from Baseline to Day **15** | -0.1 (0.92) | -0.5 (0.76) | -0.9 (0.64) | -0.6 (0.60) | -0.7 (0.65) |
| Day **22** IGA Score | 3.1 (0.67) | 3.4 (0.52) | 2.7 (0.73) | 2.3 (0.80) | 2.7 (0.80) |
| % Change from Baseline to Day **22** | -9.0 (19.61) | -17.5 (15.35) | -30.8 (12.76) | -32.5 (23.99) | -29.4 (19.17) |
| Absolute change from Baseline to Day **22** | -0.3 (0.65) | -0.8 (0.710 | -1.2 (0.52) | -1.1 (0.79) | -1.1 (0.68) |
| Day **25** IGA Score | 3.0 (0.89) | 3.1 (0.35) | 2.5 (0.87) | 2.2 (0.89) | 2.5 (0.86) |
| % Change from Baseline to Day **25** | -12.1 (29.43) | -23.8 (10.94) | -34.5 (18.64) | -35.7 (25.16) | -33.2 (21.05) |
| Absolute change from Baseline to Day **25** | -0.5 (0.93) | -1.0 (0.53) | -1.4 (0.790 | -1.2 (0.83) | -1.2 (0.77) |
| Day **29** IGA Score | 2.9 (1.08) | 3.0 (0.53) | 2.4 (0.99) | 2.3 (0.85) | 2.4 (0.89) |
| % Change from Baseline to Day **29** | -16.0 (24.48) | -26.3 (16.20) | -38.0 (24.02) | -34.9 (21.18) | -34.8 (21.68) |
| Absolute change from Baseline to Day **29** | -0.5 (0.80) | -1.1 (0.83) | -1.5 (1.00) | -1.2 (0.68) | -1.3 (0.85) |
| Day **36** IGA Score | 2.9 (1.20) | 3.0 (0.58) | 2.2 (0.76) | 2.4 (0.50) | 2.4 (0.70) |
| % Change from Baseline to Day **36** | -16.7 (26.35) | -26.4 (17.49) | -44.1 (19.38) | -33.3 (9.13) | -37.1 (16.97) |
| Absolute change from Baseline to Day **36** | -0.5 (0.85) | -1.1 (0.90) | -1.7 (0.81) | -1.2 (0.40) | -1.4 (0.74) |
| Day **43** IGA Score | 2.8 (1.06) | 3.3 (0.76) | 2.3 (1.02) | 2.2 (0.83) | 2.4 (0.97) |
| % Change from Baseline to Day **43** | -21.1 (26.91) | -19.3 (21.88) | -40.8 (28.04) | -39.0 (19.85) | -36.6 (24.53) |
| Absolute change from Baseline to Day **43** | -0.8 (0.97) | -0.9 (1.07) | -1.6 (1.04) | -1.3 (0.58) | 1.4 (0.89) |
| Day **50** IGA Score | 2.7 (1.19) | 3.3 (0.82) | 2.4 (1.07) | 2.1 (0.80) | 2.4 (1.00) |
| % Change from Baseline to Day **50** | -18.9 (30.98) | -18.3 (23.80) | -37. 2 (25.87) | -40.7 (21.93) | -36.0 (24.57) |
| Absolute change from Baseline to Day **50** | -0.6 (1.12) | -0.8 (1.17) | -1.5 (1.07) | -1.4 (0.70) | -1.3 (0.95) |
| Day **57** IGA Score | 2.8 (1.20) | 3.2 (0.75) | 2.5 (1.03) | 2.2 (0.97) | 2.5 (1.00) |
| % Change from Baseline to Day **57** | -17.6 (33.45) | -22.5 (22.08) | -34.8 (25.21) | -36.3 (24.99) | -33.7 (24.60) |
| Absolute change from Baseline to Day **57** | -0.6 (1.01) | -1.0 (1.10) | -1.4 (1.07) | -1.2 (0.83) | -1.3 (0.97) |
| Day **64** IGA Score | 2.7 (0.79) | 3.5 (1.05) | 2.7 (1.08) | 2.1 (0.81) | 2.6 (1.06) |
| % Change from Baseline to Day **64** | -18.9 (21.44) | -14.2 (29.23) | -30.9 (26.08) | -38.5 (20.61) | -31.5 (25.22) |
| Absolute change from Baseline to Day **64** | -0.6 (0.67) | -0.7 (1.37) | -1.2 (1.06) | -1.3 (0.70) | -1.2 (0.98) |
| Day **71** IGA Score | 2.6 (0.81) | 3.4 (0.89) | 2.8 (0.86) | 2.1 (1.15) | 2.5 (1.10) |
| % Change from Baseline to Day **71** | -22.0 (20.84) | -17.0 (26.36) | -25.5 (27.32) | -41.7 (31.65) | -32.0 (30.18) |
| Absolute change from Baseline to Day **71** | -0.7 (0.65) | -0.8 (1.300 | -1.1 (1.18) | -1.5 (1.10) | -1.2 (1.15) |
| Day **85** IGA Score | 2.6 (1.17) | 3.2 (0.84) | 2.8 (0.99) | 2.6 (0.96) | 2.8 (0.96) |
| % Change from Baseline to Day **85** | -20.8 (36.69) | -22.0 (24.65) | -25.6 (31.31) | -24.6 (28.66) | -24.7 (28.77) |
| Absolute change from Baseline to Day **85** | -0.7 (1.16) | -1.0 (1.22) | -1.1 (1.23) | -0.8 (0.90) | -1.0 (1.07) |

**Table 23: Number (%) of subjects achieving EASI-50 at Day 29 and every study visit - LOCF**

| Number and % subjects with EASI50 | Placebo (N=16) | 75 mg (N=8) | 150 mg (N=22) | 300 mg (N=21) | All Doses Combined (N=51) |
|---|---|---|---|---|---|
| Week 4, Day 29 | 3 (18.8%) | 3 (37.5%) | 12 (54.5%) | 15 (71.4%) | 30 (58.8%) |
| Week 2, Day 15 | 0 | 0 | 6 (27.3%) | 11 (52.4%) | 17 (33.3%) |
| Week 5, Day 36 | 3 (18.8%) | 5 (62.5%) | 16 (72.7%) | 15 (71.4%) | 36 (70.6%) |
| Week 6, Day 43 | 3 (18.8%) | 2 (25.0%) | 14 (63.6%) | 16 (76.2%) | 32 (62.7%) |
| Week 8, Day 57 | 5 (31.3%) | 2 (25.0%) | 12 (54.5%) | 13 (61.9%) | 27 (52.9%) |
| Week 10, Day 71 | 6 (37.5%) | 1 (12.5%) | 13 (59.1%) | 16 (76.2%) | 30 (58.8%) |
| Week 12, Day 85 | 3 (18.8%) | 1 (12.5%) | 12 (54.5%) | 17 (81.0%) | 30 (58.8%) |

**Table 24: Number (%) of subjects achieving EASI-25 at Day 29 and every study visit - LOCF**

| Number and % subjects with EASI25 | Placebo (N=16) | 75 mg (N=8) | 150 mg (N=22) | 300 mg (N=21) | All Doses Combined (N=51) |
|---|---|---|---|---|---|
| Week 4, Day 29 | 4 (25.0%) | 7 (87.5%) | 16 (72.7%) | 18 (85.7%) | 41 (80.4%) |
| Week 2, Day 15 | 3 (18.8%) | 5 (62.5%) | 13 (59.1%) | 16 (76.2%) | 34 (66.7%) |
| Week 5, Day 36 | 6 (37.5%) | 7 (87.5%) | 19 (86.4%) | 18 (85.7%) | 44 (86.3%) |
| Week 6, Day 43 | 7 (43.8%) | 5 (62.5%) | 19 (86.4%) | 18 (85.7%) | 42 (82.4%) |
| Week 8, Day 57 | 8 (50.0%) | 4 (40.0%) | 16 (72.7%) | 17 (81.0%) | 37 (72.5%) |
| Week 10, Day 71 | 8 (50.0%) | 3 (37.5%) | 17 (77.3%) | 19 (90.5%) | 39 (76.5%) |
| Week 12, Day 85 | 9 (56.3%) | 3 (37.5%) | 16 (72.7%) | 20 (95.2%) | 39 (76.5%) |

**Table 25: Number (%) of subjects achieving EASI-75 at Day 29 and every study visit - LOCF**

| Number and % subjects with EASI75 | Placebo (N=16) | 75 mg (N=8) | 150 mg (N=22) | 300 mg (N=21) | All Doses Combined (N=51) |
|---|---|---|---|---|---|
| Week 4, Day 29 | 1 (6.3%) | 1 (12.5%) | 6 (27.3%) | 8 (38.1%) | 15 (29.4%) |
| Week 2, Day 15 | 0 | 0 | 1 (4.5%) | 1 (4.8%) | 2 (3.9%) |
| Week 5, Day 36 | 1 (6.3%) | 1 (12.5%) | 9 (40.9%) | 7 (33.3%) | 17 (33.3%) |
| Week 6, Day 43 | 1 (6.3%) | 1 (12.5%) | 8 (36.4%) | 6 (28.6%) | 15 (29.4%) |
| Week 8, Day 57 | 2 (12.5%) | 1 (12.5%) | 9 (40.9%) | 6 (28.6%) | 16 (31.4%) |
| Week 10, Day 71 | 2 (12.5%) | 1 (12.5%) | 6 (27.3%) | 11 (52.4%) | 18 (35.5%) |
| Week 12, Day 85 | 2 (12.5%) | 1 (12.5%) | 6 (27.3%) | 7 (33.3%) | 14 (27.5%) |

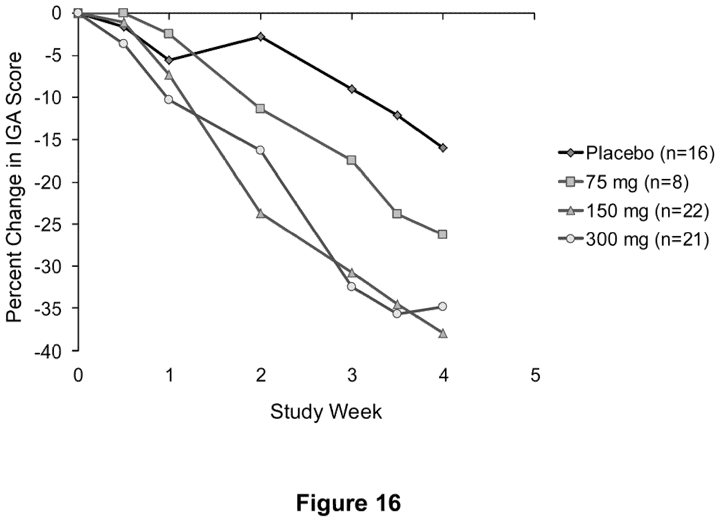

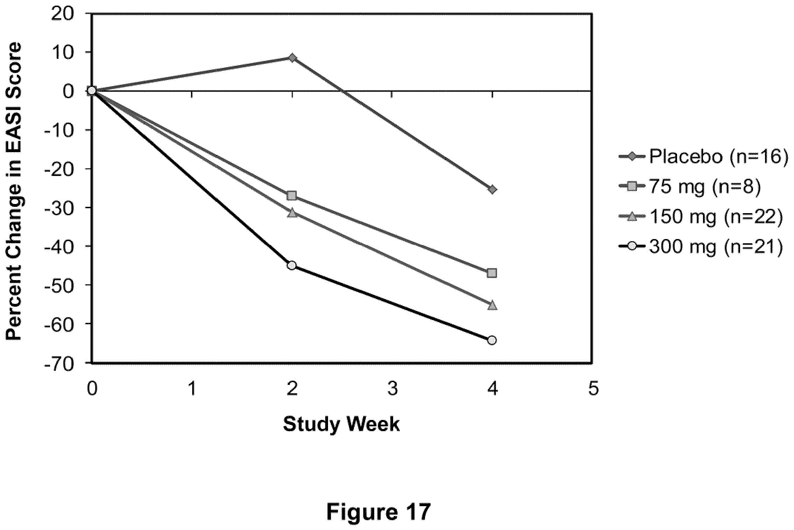

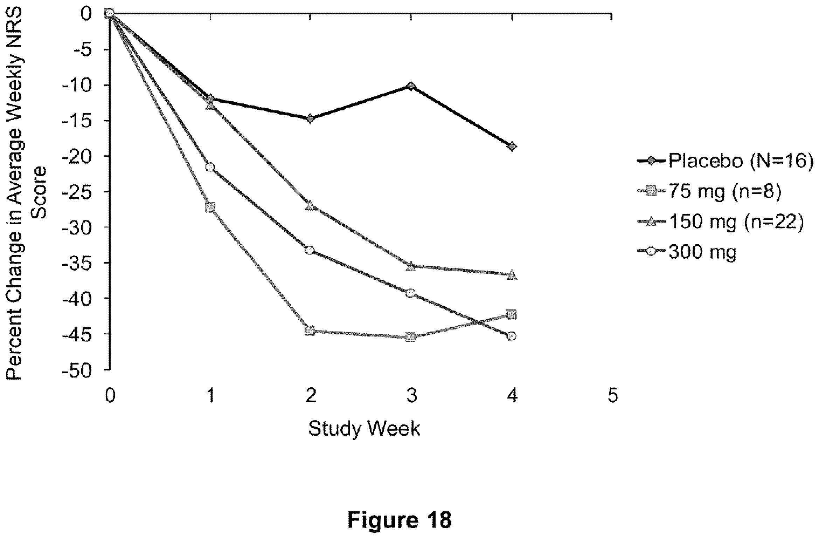

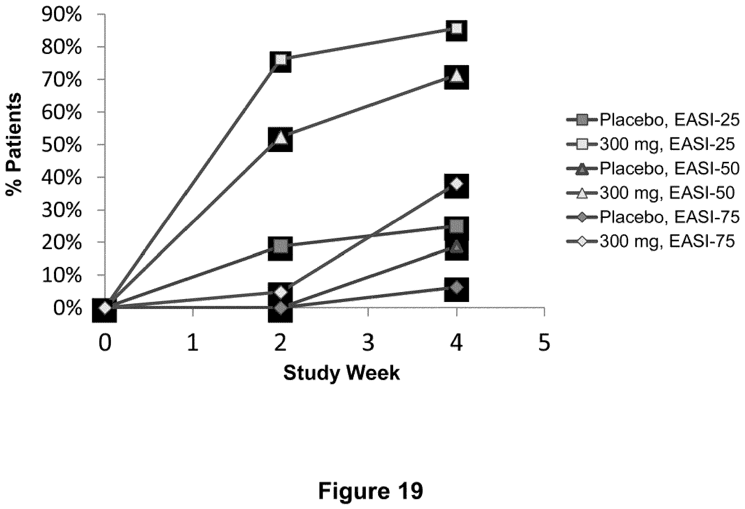

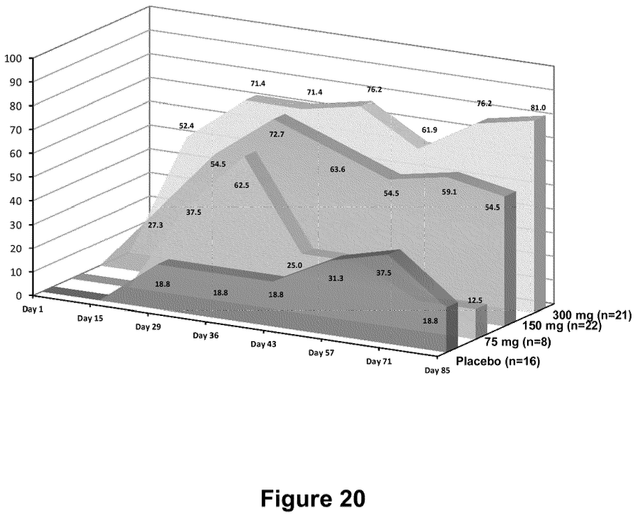

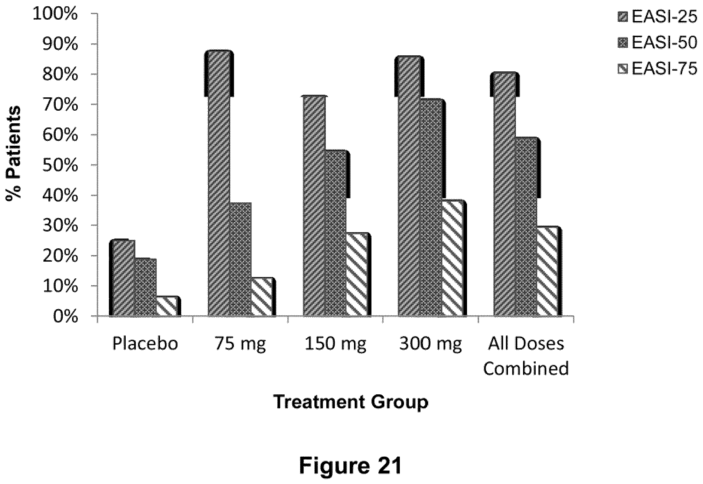

mAb1 was well-tolerated and effective in adults with moderate-to-severe AD. mAb1 administration significantly improved AD disease activity and severity. At 4 weeks, 150mg and 300mg mAb1 achieved significant improvements vs. placebo for change from baseline in %BSA (p<0.05) (Figure 15), IGA (p<0.001) (Figure 16), EASI (p<0.001) (Figure 17), and pruritus NRS (p<0.01, 300 mg) (Figure 18). More patients had ≥ 50% reduction in EASI score with 150mg mAb1 (54.5%) and with 300mg (71.4%) vs. placebo (18.8%; p<0.05 for both) (Figures 19 and 20). More patients achieved EASI-25, EASI-50, and EASI-75 with mAb1 over placebo at week 4 (Figure 21).

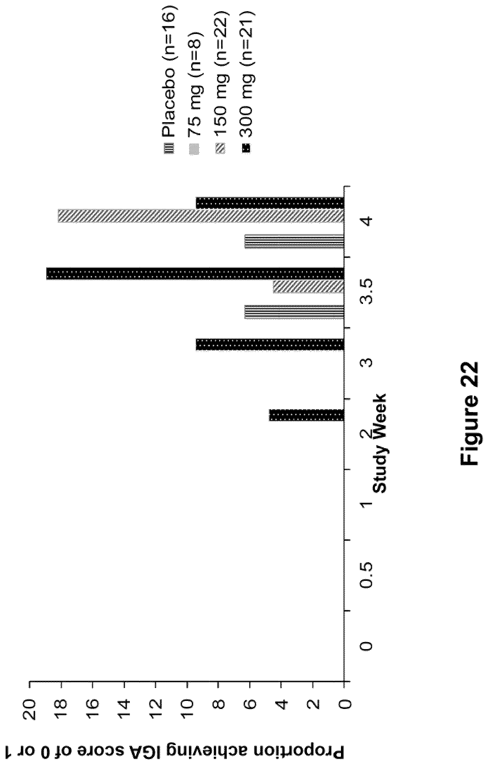

For 300 mg mAb1, significant improvement was seen within 2 weeks in %BSA (p<0.02), IGA (p<0.05), and EASI (p<0.0001). Improvements for BSA, IGA and EASI (p<0.05 vs. placebo) were maintained for 8 weeks. The proportion of patients with IGA 0 or 1 at week 4 was higher than placebo, but not statistically significant (Figure 22).

The most common treatment-emergent adverse events (AEs) with mAb1 administration were nasopharyngitis (19.6% vs. 12.5% for placebo) and headache (11.8% vs. 6.3% for placebo).

### Example 9: Parallel-Group, Dose-ranging Clinical Trial of Subcutaneously Administered anti-IL-4R Antibody (mAb1) in adult patients with moderate-to-severe atopic dermatitis

### A. Study Design

This study was a 32-week, randomized, double-blind, placebo-controlled, parallel group study to assess the dose response profile of weekly doses of mAb1 in adults with moderate-to-severe AD. The primary objective of the study was to assess the efficacy of multiple mAb1 dose regimens, compared to placebo, in adult patients with moderate-to-severe AD. The secondary objectives were: (1) to assess the safety of multiple mAb1 dose regimens, compared to placebo, in adult patients with moderate-to-severe AD; (2) to assess the pharmacokinetics (PK) of multiple mAb1 dose regimens in adult patients with moderate-to-severe AD; and (3) to assess the potential immune response across multiple mAb1 dose regimens, and to compare to placebo, in adult patients with moderate-to-severe AD.

The target population included adults with moderate-to-severe AD which could not be adequately controlled with topical medications or for whom topical treatment is otherwise inadvisable (e.g., side effects or safety risks). Approximately 240 to 288 patients were enrolled. Eligible patients were randomized in a 1:1:1:1:1:1 ratio to receive 1 of 6 weekly treatment regimens (5 active, 1 placebo). Randomization was stratified by disease severity (moderate vs. severe AD) and region (Japan vs. rest of world). The dosing schedule followed is given in Table 26.

**Table 26**

| All loading doses = 2 injections of 2 mL each; | | | | |
|---|---|---|---|---|
| All week 1 - week 15 doses = 1 injection of 2 mL | | | | |
| N | Day 1 (loading dose) | | Subsequent dosing through week 15 | |
| | 1 injection of each | Total dose | Dose regimen | Cumulative monthly dose |
| 40 | 300mg | 600 mg | 300 mg qw | 1200 mg |
| | 300 mg | | | |
| 40 | 300 mg | 600 mg | 300 mg q2w | 600 mg |
| | 300 mg | | | |
| 40 | 300 mg | 600 mg | 300 mg q4w | 300 mg |
| | 300 mg | | | |
| 40 | 200 mg | 400 mg | 200 mg q2w | 400 mg |
| | 200 mg | | | |
| 40 | 200 mg | 400 mg | 100 mg q4w | 100 mg |
| | 200 mg | | | |
| 40 | Placebo | 0 mg | PBO qw | 0 mg |
| | Placebo | | | |

All patients received 2 injections (a loading dose) on day 1, followed by weekly injections. For every 2 weeks (q2w) and every 4 weeks (q4w) dose-regimens, the next dose of study drug was administered at week 2 and week 4, respectively. Patients assigned to q2w and q4w dose-regimens received volume-matched placebo every week when mAb1 was not administered. After providing informed consent, patients were assessed for study eligibility at the screening visit. Patients who met eligibility criteria underwent day 1/baseline assessments, randomization, and then received a weekly injection of study drug from day 1 through week 15. During this time, patients returned for weekly clinic visits with some weeks requiring only a telephone contact. Patients (and/or caregivers) were trained on injecting study drug at visits 2, 3, 4, 5, and 6, and self-injected study drug at later study visits that required only a telephone contact. Patients were closely monitored at the study site for a minimum of 1 hour after each of the first 5 weekly injections. Safety, laboratory, and clinical effect assessments were performed at specified clinic visits. The end of treatment period visit occurred at week 16, 1 week after the last dose of study drug, when the primary endpoint was assessed. Follow-up visits occurred every 2 weeks from week 18 through week 32. The end of study visit occurred at week 32. Rescue treatment for AD (medication and/or phototherapy) was provided to study patients, if necessary, at the discretion of the investigator. Patients who needed rescue treatment were immediately discontinued from study drug, but were asked to continue to follow the schedule of study assessments. Efficacy measurements were obtained (Investigator's Global Assessment [IGA], Eczema Area and Severity Index [EASI], etc.) immediately before administering any rescue treatment. One sample for DNA analysis and multiple samples for RNA analysis were collected from patients who consent to participate in the optional genomic sub-study.

Study treatment: mAb1 administered subcutaneously: 300 mg weekly (qw), 300 mg q2w, 300 mg q4w, 200 mg q2w, or 100 mg q4w, from day 1 through week 15 OR once weekly subcutaneous dose of placebo from day 1 through week 15. A basic bland topical emollient was applied twice daily from day -7 through day 8.

Endpoints of the study: The primary endpoint of the study was the percent change in EASI score from baseline to week 16. The secondary endpoints included: (1) proportion of patients achieving IGA 0 (clear) or 1 (almost clear) at week 16; (2) proportion of patients achieving IGA score reduction of ≥ 2 at week 16; (3) absolute change in EASI scores from baseline to week 16; (4) proportion of patients achieving EASI-50, EASI-75, and EASI-90 (50, 75 and 90% reduction from baseline in EASI score) at week 16; (6) proportion of patients achieving SCORAD-50, SCORAD-75, and SCORAD-90 (50, 75 and 90% reduction from baseline in SCORAD score) at week 16; (7) absolute and percent change from baseline in pruritus scores (NRS and 4-point categorical scale); (8) absolute and percent change from baseline in POEM scores; (9) changes from baseline in GISS components (erythema, infiltration/population, excoriations, and lichenification); 910) changes from baseline in GISS cumulative score; (11) incidence of treatment-emergent adverse events (TEAEs) from baseline through week 32; and (12) pharmacokinetic profile of multiple mAb1 dose regimens.

The other exploratory endpoints included: (1) distribution of disease severity scores (eg, IGA, EASI, SCORAD) and change from baseline to various time points through week 16; (2) changes in Pruritus NRS, Pruritus categorical scale, SCORAD (pruritus VAS and sleep disturbance VAS), patient global assessment of disease status, patient global assessment of treatment effect, DLQI, POEM, EQ-5D, Itchy QOL, and HADS from baseline to various time points through week 16; (3) absolute and percent change in % BSA, SCORAD score, EASI and Pruritus NRS, from baseline to various time points through week 16; (4) proportion of patients who achieve reduction of IGA score by ≥2 from baseline to various time points through week 16; (5) proportion of patients who achieve reduction of IGA score by ≥3 from baseline to various time points through week 16; (6) changes in efficacy parameters from week 16 to week 32; (7) Incidence and profile (titers over time) of mAb1 ADAs; (8) effect of mAb1 plasma concentration on ADA formation and persistence; (9) effect of ADA on mAb1 plasma concentration; (14) effect of ADA on clinical outcomes (safety and efficacy); (10) effect of PK parameters (Cmax and AUC) on clinical outcomes; and (11) effect of body weight on drug exposure and clinical outcomes.

Rationale for study design: The purpose of this study was to find an optimal dose-regimen, which will be further investigated in confirmatory phase 3 studies. The design of this phase 2b study was informed by results from a previous mAb1 study, which investigated the safety and efficacy of a 300 mg dose of mAb1 administered weekly (qw) for 12 consecutive weeks in patients with moderate-to-severe AD. The selection of phase 2b dose-regimens was also supported by observed and simulated correlations between pharmacokinetic (PK) and pharmacodynamics (PD) parameters (PK/PD models) from earlier clinical trials. Using 300 mg qw (ie, the dose-regimen studied in phase 2a) as the high anchor of the dose range, the goal was to identify the lowest dose-regimen with maximal or near-maximal efficacy and/or, depending on mAb1's emerging safety profile, find the dose-regimen with the best benefit/risk ratio. Accordingly, 5 mAb1 dose-regimens were selected to reasonably cover the spectrum between a potentially supra-therapeutic dose-regimen (ie, the high anchor) and a dose-regimen with clearly sub-optimal efficacy (ie, the low anchor). The protocol also included a placebo arm to allow comparison of each active dose-regimen to a control.

Use of loading doses: Most patients received a loading dose on day 1, consisting of a doubling of the nominal dose that was administered at subsequent visits. This allowed systemic concentrations of mAb1 to reach steady state and the targeted systemic concentration faster, and potentially reducing the time to clinical benefit. Study treatment was administered for 16 weeks so that systemic concentrations of functional mAb1 could stabilize for all dose-regimens investigated. Pharmacokinetic modeling suggested that 'every-4-weeks' (q4w) dose-regimens could result in declining trough concentrations following an initial loading dose. Consequently, the immunogenic potential of these regimens may not be fully expressed within a shorter treatment course. After the last dose of study drug, all patients were followed for 16 additional weeks, which ensured that mAb1 clearance was virtually complete (plasma concentrations below the lower limit of quantification) before the end of study visit.

Rationale for Dose Selection: The highest mAb1 dose-regimen administered in this study was 300 mg qw. When given as a short (4-week) treatment course, this dose-regimen was safe and appeared to be the most efficacious in earlier phase 1b clinical trials, in which it was investigated alongside lower dose-regimens (150 mg qw and 75 mg qw). Pharmacokinetic modeling suggested that 300 mg qw may be supra-therapeutic in the long run: mAb1 plasma concentrations did not reach steady state by week 4, and were projected to stabilize at levels considerably above those required to saturate the target, ie, the membrane-bound alpha subunit of the IL-4 receptor. However, this needed to be confirmed by comparing 300 mg qw with lower dose-regimens in the context of a longer study treatment (12 weeks or longer), so that plasma concentrations could reach steady state for all dose-regimens investigated. Although 300 mg qw was administered for 12 weeks in an earlier study (e.g., phase 2a proof-of-concept), this dose-regimen was repeated in phase 2b to confirm phase 2a results and enabled a direct comparison with lower dose-regimens within the same study. Therefore, 300 mg qw was the high anchor of the dose range in the present study.

The low anchor of the dose-regimen range was 100 mg administered q4w. Based on PK/PD modeling, the resulting mAb1 plasma concentrations at steady state were expected to be consistently below target mediated clearance (ie, at levels low enough such that mAb1 elimination was achieved primarily via its binding to the IL-4 receptor), suggesting that the clinical response associated with this dose-regimen was incomplete. Three other dose-regimens were selected between the high and low anchors. A summary of these dose-regimens and the main rationale for their selection is provided below:
- 300 mg qw: High anchor. Same dose-regimen studied in phase 2a.
- 300 mg every 2 weeks (q2w): High probability of success based on PK/PD data and models. Could be sufficient to maintain therapeutic drug levels over multiple dosing intervals.
- 300 mg q4w: PK modeling indicated that mAb1 plasma levels climbed rapidly to >60 mg/L after the administration of a loading dose, which was associated with a fast onset of action, q4w dosing could be sufficient to maintain the therapeutic effect over time. Since the 300 mg dose is the highest available, this regimen had the best chance to demonstrate efficacy for q4w administration.
- 200 mg q2w: Some efficacy expected without reaching the maximum therapeutic effect. Useful for dose-response assessment and further PK/PD modeling. Helped evaluate a full spectrum of q2w regimens.
- 100 mg q4w: Low anchor. Likely non-optimally efficacious dose.
- Placebo: Provided a reliable reference for any apparent drug effects.

Inclusion and Exclusion Criteria: A patient had to meet the following criteria to be eligible for inclusion in the study: (1) Male or female, 18 years or older; (2) Chronic AD, (according to the AAD Consensus Criteria, [Eichenfeld 2004]), that has been present for at least 3 years before the screening visit; (3) EASI score ≥16 at the screening and baseline visits; (4) IGA score ≥3 (on the 0-4 IGA scale) at the screening and baseline visits; (5) ≥10% body surface area (BSA) of AD involvement at the screening and baseline visits; (6) Patients with documented recent history (within 3 months before the screening visit) of inadequate response to outpatient treatment with topical medications, or for whom topical treatments are otherwise inadvisable (eg, because of important side effects or safety risks)*; (7) Patients must have applied a stable dose of an additive-free, basic bland emollient twice daily for at least 7 days before the baseline visit; (8) Willing and able to comply with all clinic visits and study-related procedures; (9) Able to understand and complete study-related questionnaires; and (10) Provide signed informed consent. *NOTE: For the purpose of this protocol, inadequate response represented failure to achieve and maintain remission or a low disease activity state (eg, IGA 0=clear to 2=mild) despite treatment with topical corticosteroids of medium to high potency (± topical calcineurin inhibitors as appropriate), applied daily for at least 28 days or for the maximum duration recommended by the product prescribing information (eg, 14 days for super-potent topical corticosteroids), whichever is shorter. Important side effects or safety risks are those that outweigh the potential treatment benefits (eg, hypersensitivity reactions, significant skin atrophy, systemic effects, etc., or imminence thereof), as assessed by the investigator or by patient's treating physician.

A patient who met any of the following criteria was ineligible to participate in this study: (1) Prior treatment with mAb1; (2) Treatment with an investigational drug within 8 weeks or within 5 half-lives (if known), whichever is longer, before the baseline visit; (3) The following treatments within 4 weeks before the baseline visit, or any condition that will likely require such treatment(s) during the first 4 weeks of study treatment: systemic corticosteroids, immunosuppressive/immunomodulating drugs (eg, cyclosporine, mycophenolate-mofetil, IFN-y, azathioprine or methotrexate), or phototherapy for AD; (4) Treatment with topical corticosteroids, tacrolimus, and/or pimecrolimus within 1 week before the baseline visit; (5) Treatment with biologics as follows: any cell-depleting agents including but not limited to rituximab: within 6 months before the baseline visit, or until lymphocyte and CD 19+ lymphocyte count returns to normal, whichever is longer, infliximab, adalimumab, golimumab, certolizumab pegol, abatacept, etanercept, anakinra: within 16 weeks before the baseline visit for any indication, or within 5 years for dermatological indications, or other biologics: within 5 half-lives (if known) or 16 weeks, whichever is longer; (6) Treatment of AD with prescription moisturizers classified as medical device (eg, Atopiclair^{®}, MimyX^{®}, Epicerum^{®}, Cerave^{®}, etc.) within 1 week before the baseline visit; (7) Regular use (more than 2 visits per week) of a tanning booth/parlor within 4 weeks before the baseline visit; (8) Planned or anticipated use of any prohibited medications and procedures (including, but not limited to, topical tacrolimus and pimecrolimus; corticosteroids; prescription moisturizers classified as medical devices such as Atopiclair^{®}, MimyX^{®}, Epicerum^{®},Cerave^{®}, etc; allergen immunotherapy; systemic treatment for AD with an immunosuppressive/immunomodulating substance; treatment with a live (attenuated) vaccine or with an investigational drug (other than mAb1); major elective surgeries) during study treatment; (9) Treatment with a live (attenuated) vaccine within 12 weeks before the baseline visit; (10) Chronic or acute infection requiring treatment with antibiotics, antivirals, antiparasitics, antiprotozoals, or antifungals within 4 weeks before the screening visit, or superficial skin infections within 1 week before the screening visit; (11) Known or suspected immunosuppression, including history of invasive opportunistic infections (eg, histoplasmosis, listeriosis, coccidioidomycosis, pneumocystosis, aspergillosis) despite infection resolution, or otherwise recurrent infections of abnormal frequency, or prolonged infections suggesting an immune-compromised status, as judged by the investigator; (12) Known history of human immunodeficiency virus (HIV) infection or HIV seropositivity at the screening visit; (13) Positive or indeterminate hepatitis B surface antigen (HBsAg), hepatitis B core antibody (HBcAb), or hepatitis C antibody at the screening visit; (14) Elevated transaminases (ALT and/or AST) more than 3 times the upper limit of normal (>3 xULN) at the screening visit; (15) History of clinical endoparasitosis within 12 months before the baseline visit, other than treated vaginal trichomoniasis; (16) Presence of skin comorbidities that may interfere with study assessments; (17) History of malignancy within 5 years before the baseline visit, except completely treated in situ carcinoma of the cervix, completely excised non-metastatic squamous or basal cell carcinoma of the skin; (18) History of non-malignant lymphoproliferative disorders; (19) High risk of parasite infection, such as residence within or recent travel (within 12 months before the baseline visit) to areas endemic for endoparasitoses, where the circumstances are consistent with parasite exposure (eg, extended stay, rural or slum areas, lack of running water, consumption of uncooked, undercooked, or otherwise potentially contaminated food, close contact with carriers and vectors, etc.), unless subsequent medical assessments (eg, stool exam, blood tests, etc.) have ruled out the possibility of parasite infection/infestation; (20) History of alcohol or drug abuse within 2 years before the screening visit; (21) Severe concomitant illness(es) that, in the investigator's judgment, would adversely affect the patient's participation in the study. Examples include, but are not limited to patients with short life expectancy, patients with uncontrolled diabetes (HbA1c ≥9%), patients with cardiovascular conditions (eg, stage III or IV cardiac failure according to the New York Heart Association classification), severe renal conditions (eg, patients on dialysis) hepato-biliary conditions (eg, Child-Puig class B or C), neurological conditions (eg, demyelinating diseases), active major autoimmune diseases (eg, lupus, inflammatory bowel disease, rheumatoid arthritis, etc.), other severe endocrinological, gastrointestinal, metabolic, pulmonary, or lymphatic diseases. The specific justification for patients excluded under this criterion will be noted in study documents (chart notes, case report forms [CRF], etc); (22) Any other medical or psychological condition including relevant laboratory abnormalities at screening that, in the opinion of the investigator, suggest a new and/or insufficiently understood disease, may present an unreasonable risk to the study patient as a result of his/her participation in this clinical trial, may make patient's participation unreliable, or may interfere with study assessments. The specific justification for patients excluded under this criterion will be noted in study documents (chart notes, CRF, etc.); (23) Planned major surgical procedure during the patient's participation in this study; (24) Patient is a member of the investigational team or his/her immediate family; (25) Pregnant or breast-feeding women; and (26) Unwilling to use adequate birth control, if of reproductive potential and sexually active. Adequate birth control is defined as agreement to consistently practice an effective and accepted method of contraception throughout the duration of the study and for 16 weeks after last dose of study drug.

### B. Safety

Safety was assessed throughout the study by monitoring Adverse Events and Serious Adverse Events.

An Adverse Event (AE) is any untoward medical occurrence in a subject or clinical investigation subject administered a pharmaceutical product. An AE can, therefore, be any unfavorable and unintended sign (including abnormal laboratory finding), symptom, or disease temporally associated with the use of a medicinal product, whether or not considered related to the medicinal (investigational) product. AEs also include: any worsening (i.e., any clinically significant change in frequency and/or intensity) of a pre-existing condition that is temporally associated with the use of the study drug; abnormal laboratory findings considered by the Investigator to be clinically significant; and any untoward medical occurrence.

A Serious Adverse Event (SAE) is any untoward medical occurrence that at any dose results in death; is life-threatening; requires in-patient hospitalization or prolongation of existing hospitalization; results in persistent or significant disability/ incapacity; is a congenital anomaly/ birth defect; or is an important medical event.

In addition, laboratory safety variables, vital sign variables, 12-lead electrocardiography (ECG) variables, and physical examination variables were measured throughout the study.

The clinical laboratory data consists of hematology, blood chemistry and urinalysis. Blood samples for hematology testing were collected at every study visit; blood samples for serum chemistry testing and urine samples for urinalysis were collected to measure overall patient health at screening, day 1/ baseline (pre-dose), day 15, day 29, day 43, day 57, day 71, day 85, day 99, day 113, day 141, day 169, and day 197 (end-of-study) or early termination if subject is discontinued from the study.

Vital sign parameters include respiratory rate (bpm), pulse rate (bpm), systolic and diastolic blood pressure (mmHg) and body temperature (°C). Vital signs were collected (pre-dose, on dosing days) at screening and day 1/baseline, and days 4, 8, 15, 22, 25, 29, 43, 64, 71, 85, 99, 113, 127, 141, 155, 169, 183, 197 and 211 (end of study) or early termination. Vital signs were taken at 1 and 2 hours post-injection following the study drug dose on days 1, 8, 15, and 22.

12-Lead ECG parameters include: Ventricular HR, PR interval, QRS interval, corrected QT interval (QTcF=QT/[RR^{0.33}] and QTcB=QT/[RR^{0.5}]) ECG status: normal, abnormal not clinical significant or abnormal clinical significant. A standard 12-lead ECG was performed at screening, day 29, and day 113 (end of treatment) or early termination.

A thorough and complete physical examination was performed at screening, day 29, and day 113 (end of treatment) or early termination.

### C. Efficacy variables

The efficacy variables IGA, BSA, EASI, SCORAD, 5-D Pruritus scale, and Pruritus NRS rating have been described elsewhere herein (see, e.g., Example 7).

The IGA, BSA, EASI and SCORAD scores were assessed at every clinic visit. Patients underwent 5-D pruritus assessment at the following visits: screening, day 1/baseline (pre-dose), day 113 (end of treatment), and day 211 (end of study) or early termination. Patients used the IVRS to record their Pruritus NRS score twice daily through the last study visit.

In addition, other variables such as Global Individual Signs Score (GISS), Pruritus Categorical Scale, Patient Oriented Eczema Measure (POEM), Dermatology Life Quality Index (DLQI), Itchy QOL, EQ-50, HADS, and Patient Global Assessment of Disease Status and Treatment Effect were also assessed.

Baseline for efficacy variable was defined as the last non-missing value on or before the date of randomization. For the patient who had no value on or before his/her randomization date, the last non-missing value on or before the date of first dose injection was used as baseline.

### Example 10: Repeat-dose Clinical Trial of Subcutaneously Administered anti-IL-4R Antibody (mAb1) in adult patients with moderate-to-severe atopic dermatitis

### A. Study Design

This study was a 28-week randomized, double-blind, placebo-controlled study of the anti-IL-4R mAb, referred herein as "mAb1", administered subcutaneously in patients with moderate-to-severe atopic dermatitis. The treatment period was 12 weeks in duration with the patients followed for a further 16 weeks after end of the treatment.

109 patients were included and randomized in the ratio of 1:1 for the study (54 in placebo and 55 for 300mg of the antibody). 43 patients (30 in placebo and 13 in 300 mg group) withdrew from the study. Randomization was stratified according to IgE levels (IgE < 150 kU/L vs. ≥ 150 kU/L at the screening visit) to test the efficacy of mAb1 in patients with extrinsic or intrinsic form of AD. Patients who met eligibility criteria underwent day 1/baseline assessments, randomization, and then received 300 mg of mAb1 or placebo SC. Each weekly dose of study drug was given as one 2-mL injection, or was split into two 1-mL injections. Patients returned for weekly clinic visits and received an injection of study drug on days 8, 15, 22, 29, 36, 43, 50, 57, 64, 71, and 78. Patients were closely monitored at the study site for a minimum of 2 hours after each dose of study drug. The end of the treatment period was day 85. Follow-up visits occurred on days 92, 99, 106, 113, 120, 127, 134, 141, 148, 155, 162, 169, 176, 183, 190, and the end of study visit on day 197.

Inclusion criteria for the study were as follows: (1) Male or female 18 years or older; (2) Chronic AD, diagnosed by the Eichenfield revised criteria of Hannifin and Rajka, that has been present for at least 3 years before the screening visit; (3) EASI score ≥ 16 at the screening and baseline visits; (4) IGA score ≥ 3 at the screening and baseline visits; (5) ≥ 10% BSA of AD involvement at the screening and baseline visits; (6) history of inadequate response to a stable (≥ 1 month) regimen of topical corticosteroids or calcineurin inhibitors as treatment for AD within the last 3 months before the screening visit; (7) Patients must have applied a stable dose of an additive-free, basic bland emollient twice-daily for at least 7 days before the baseline visit; and (8) Willingness, commitment, and ability to return for all clinic visits and complete all study-related procedures and willing and able to sign the informed consent form (ICF).

Exclusion criteria for the study were as follows: (1) Prior treatment with mAb1; (2) Presence of any of the following laboratory abnormalities at the screening visit: white blood cell count < 3.5 x 10³/µL; platelet count <125 x 10³/µL; neutrophil count <1.75 x 10³/µL; aspartate aminotransferase (AST)/alanine aminotransferase (ALT) >1.5x the ULN; and CPK > 2x the ULN; (3) Positive or indeterminate results at the screening visit for hepatitis B surface antigen, hepatitis B core antibody or hepatitis C antibody; (4) Onset of a new exercise routine or major change to a previous exercise routine within 4 weeks prior to screening (visit 1). Subjects had to be willing to maintain a similar level of exercise for the duration of the study and to refrain from unusually strenuous exercise for the duration of the trial; (5) Treatment with an investigational drug within 8 weeks or within 5 half-lives, if known, whichever is longer, before the baseline visit; (6) Treatment with a live (attenuated) vaccine within 12 weeks before the baseline visit; (7) Treatment with allergen immunotherapy within 6 months before the baseline visit; (8) Treatment with leukotriene inhibitors within 4 weeks before the baseline visit; (9) Treatment with systemic corticosteroids within 4 weeks before the baseline visit; (10) Treatment with topical corticosteroids, tacrolimus, and/or pimecrolimus within 1 week before the baseline visit; (11) Systemic treatment for AD with an immunosuppressive/immunomodulating substance, eg. Cyclosporine, mycophenolate-mofetil, IFN-y, phototherapy, (narrow band uvB, uvB, uvA1, psoralen + uvA), azathioprine, methotrexate, or biologics, within 4 weeks before the baseline visit; (12) three or more bleach baths during any week within the 4 weeks before the baseline visit; (13) Treatment of AD with a medical device (eg. Atopiclair^{®}, MimyX^{®}, Epicerum^{®}, Cerave^{®}, etc) within 1 week before the baseline visit; (14) Chronic or acute infection requiring treatment with oral or IV antibiotics, antivirals, anti-parasitics, anti-protozoals, or anti-fungals within 4 weeks before the screening visit, or superficial skin infections within 1 week before the screening visit; (15) Known history of HIV infection; (16) History of hypersensitivity reaction to doxycycline or related compounds; (17) History of clinical parasite infection, other than vaginal trichomoniasis; (18) History of malignancy within 5 years before the baseline visit, with the following exceptions; patients with a history of completely treated carcinoma in situ of cervix, and non-metastatic squamous or basal cell carcinoma of the skin are allowed; (19) Planned surgical procedure during the length of the patient's participation in the study; (20) Use of a tanning booth/parlor within 4 weeks before the screening visit; (21) Significant concomitant illness or history of significant illness such as psychiatric, cardiac, renal, neurological, endocrinological, metabolic or lymphatic disease, or any other illness or condition that would have adversely affected the subject's participation in this study; (22) Pregnant or breast-feeding women; and/or (23) Unwilling to use adequate birth control. Adequate birth control is defined as agreement to consistently practice an effective and accepted method of contraception throughout the duration of the study and for 16 weeks after last dose of study drug. For females, adequate birth control methods are defined as: hormonal contraceptives, intrauterine device (IUD), or double barrier contraception (ie, condom + diaphragm, condom or diaphragm + spermicidal gel or foam). For males, adequate birth control methods are defined as: double barrier contraception (ie, condom + diaphragm, condom or diaphragm + spermicidal gel or foam). For females, menopause is defined as 24 months without menses; if in question, a follicle-stimulating hormone of ≥25 U/mL must be documented. Hysterectomy, bilateral oophorectomy, or bilateral tubal ligation must be documented, as applicable.

### B. Efficacy Variables

The primary endpoint was the percent change in EASI score from baseline to week 12. The secondary endpoints measured in this study included: (1) proportion of patients who achieved an investigator's global assessment (IGA) score of 0 or 1 at week 12; (2) proportion of patients who achieved ≥ 50% overall improvement in EASI score (also called EASI 50) from baseline to week 12; (3) change in EASI score from baseline to week 12; (4) change and percent change in IGA score, body surface area involvement of atopic dermatitis (BSA), eczema area and severity index (EASI), SCORAD, Pruritus NRS and 5-D pruritus scale from baseline to week 12; (5) Incidence of TEAEs from baseline through week 28; (6) change from baseline in eosinophils, TARC, Phadiatop^{™} results, and total IgE associated with response; (7) change in QoLIAD from baseline to week 12; (8) proportion of patients who achieve reduction of IGA score of ≥ 2 from baseline to week 12; (9) proportion of patients who achieve reduction of IGA score of ≥ 3 from baseline to week 12; and (10) PD response of circulating eosinophils, TARC and total IgE.

Baseline for efficacy variable is defined as the last non-missing value on or before the date of randomization. For the patient who has no value on or before his/her randomization date the last non-missing value on or before the date of first dose injection will be used as baseline.

### Investigation Procedures

The efficacy variables IGA, BSA, EASI, SCORAD, 5-D Pruritus scale, and Pruritus NRS rating have been described elsewhere herein (see, e.g., Example 7).

The IGA, BSA, EASI and SCORAD scores were assessed at every clinic visit. Patients underwent 5-D pruritus assessment at the following visits: screening, day 1/baseline (pre-dose), and days 15, 29, 43, 57, 71, 85, 99, 113, 127, 141, 155, 169, 183 and 197 (end of study) or early termination. Patients used the IVRS to record their Pruritus NRS score twice daily through the last study visit.

Quality of Life Index for Atopic Dermatitis (QoLIAD): The QoLIAD is a 25-item, validated questionnaire used in clinical practice and clinical trials to assess the impact of AD disease symptoms and treatment on QoL. The format is a simple yes/no response to 25 items with a scoring system of 0 to 25; a high score is indicative of a poor QoL. The questionnaire was administered at screening and day 1/baseline (pre-dose), and days 29, 57, 85, 99, 113, 127, 141, 155, 169, 183, and 197 (end of study) or early termination.

### C. Investigational Treatment

mAb1 drug product was supplied as a lyophilized powder in a 5 ml glass vial for SC administration. When delivered SC, the mAb1 drug product was reconstituted with 2.5 ml of sterile water for injection, yielding a solution containing 150 mg/mL of mAb1. The dose level of mAb1 tested was 300 mg for SC administration. mAb1 or placebo was administered as 1 (2 mL) or 2 (1 mL) SC injections in the clinic on day 1/baseline and days 8, 15, 22, 29, 36, 43, 50, 57, 64, 71, and 78. Although it was preferred that each weekly dose of study drug be given as one 2-mL injection, each weekly dose could be split into two 1-mL injections. Subcutaneous injection sites were alternated between the following sites: back of arms, abdomen (except the navel or waist area), and upper thighs. Administration to the extremities was not allowed due to the possibility of different absorption and bioavailability. If administration of multiple injections were required on the same day, each injection was delivered at a different injection site (e.g., 1 injection administered in the right lower quadrant of the abdomen and the other in the left lower quadrant of the abdomen). Subcutaneous injection sites were alternated so that the same sites were not injected for 2 consecutive weeks.

Placebo matching mAb1 was prepared in the same formulation as mAb1, but without addition of antibody.

Patients were monitored at the study site for a minimum of 2 hours after each dose of study drug.

In addition, patients were required to apply stable doses of an additive-free, basic bland emollient twice daily for at least 7 days before the baseline visit and throughout study participation. Patients reported compliance with background treatment during the study using the IVRS or IWRS. The system prompted patients to answer the following question about emollient use: "Did you use a moisturizer approved by the study doctor on the affected areas of your skin?"

### D. Safety Assessment

Safety was assessed throughout the study by monitoring Adverse Events and Serious Adverse Events.

An Adverse Event (AE) is any untoward medical occurrence in a subject or clinical investigation subject administered a pharmaceutical product. An AE can, therefore, be any unfavorable and unintended sign (including abnormal laboratory finding), symptom, or disease temporally associated with the use of a medicinal product, whether or not considered related to the medicinal (investigational) product. AEs also include: any worsening (i.e., any clinically significant change in frequency and/or intensity) of a pre-existing condition that is temporally associated with the use of the study drug; abnormal laboratory findings considered by the Investigator to be clinically significant; and any untoward medical occurrence.

A Serious Adverse Event (SAE) is any untoward medical occurrence that at any dose results in death; is life-threatening; requires in-patient hospitalization or prolongation of existing hospitalization; results in persistent or significant disability/ incapacity; is a congenital anomaly/ birth defect; or is an important medical event.

In addition, laboratory safety variables, vital sign variables, 12-lead electrocardiography (ECG) variables, and physical examination variables were measured throughout the study.

The clinical laboratory data consists of hematology, blood chemistry and urinalysis. Blood samples for hematology testing were collected at every study visit; blood samples for serum chemistry testing and urine samples for urinalysis were collected to measure overall patient health at screening, day 1/ baseline (pre-dose), day 15, day 29, day 43, day 57, day 71, day 85, day 99, day 113, day 141, day 169, and day 197 (end-of study) or early termination if subject is discontinued from the study.

Vital sign parameters include respiratory rate (bpm), pulse rate (bpm), systolic and diastolic blood pressure (mmHg) and body temperature (°C). Vital signs were collected (pre-dose, on dosing days) at screening and day 1/baseline, and days 8, 15, 22, 29, 36, 43, 50, 57, 64, 71, 78, 85, 99, 113, 141, 169 and 197 (end of study) or early termination. Vital signs were taken at 1 and 2 hours post-injection following the study drug dose on days 1, 8, 15, 22, 29, 36, 43, 50, 57, 64, 71 and 78.

12-Lead ECG parameters include: Ventricular HR, PR interval, QRS interval, corrected QT interval (QTcF=QT/[RR^{0.33}] and QTcB=QT/[RR^{0.5}]) ECG status: normal, abnormal not clinical significant or abnormal clinical significant. A standard 12-lead ECG was performed at screening, day 141, and day 197 (end of study) or early termination.

Research samples (serum/RNA/plasma) were collected at screening and day1/baseline (pre-dose), and days 8, 15, 22, 29, 57, 85, and 197 (end of study) or early termination, and at unscheduled visits.

A thorough and complete physical examination was performed at screening, day 85, and day 197 (end of study) or early termination.

### E. Data Analysis

### 1. Analyses of Exploratory Efficacy Variables

All categorical variables were analyzed using the Fisher's Exact test with nominal p-value and confidence intervals reported. All continuous variables were analyzed by the ANalysis of COVAriance (ANCOVA) using baseline IgE stratum (<150 kU/L vs. ≥150 kU/L at the screening visit). Unless otherwise specified, assessments of changes from baseline and construction of confidence intervals for continuous measures were based on an ANCOVA model which includes treatment as the main factor and baseline value as covariates. Point estimate and 95% Cl of the difference in adjusted mean change from baseline between two treatment groups are provided. Missing values will be imputed by the last observation carried forward (LOCF) approach. In the event that the model assumptions are not warranted, the Rank-based analysis of covariates will be used.

### 2. Analysis of Safety Data

The safety analysis is based on the reported AEs, clinical laboratory evaluations, vital signs, and 12-lead ECG. Thresholds for Potentially Clinically Significant Values (PCSV) in laboratory variables, vital signs and ECG are defined in SAP. The time interval to detect any event or abnormality is between the infusion of study medication and end of study. Data collected outside this interval are excluded from the calculation of descriptive statistics and identification of abnormalities for laboratory evaluations, vital signs and ECG.

### F. Safety: Results

mAb1 was generally well-tolerated with a favorable safety profile. The overall adverse event (AE) profile was characteristic of a healthy population. No deaths were reported. There were 8 patients with SAEs, of which 1 was in mAb1 group (facial bones fracture) and 7 were in the placebo group (angina pectoris, cellulitis, eczema herpeticum, skin bacterial infection, renal failure, asthmatic crisis, lung disorder and atopic dermatitis). There were 8 patients with TEAE resulting in discontinuation from study drug, of which 1 was in the mAb1 group and 7 in the placebo group. There were 87 patients with at least one TEAE (n=43 [78.2%] in mAb1 vs. 44 [81.5%] in placebo group). The most frequent TEAEs were nasopharyngitis infections in subjects dosed with mAb1 (n=22 [40%] vs. 10 [18.5%] for placebo). Other TEAEs in the treatment group included eye infections, nervous system disorders, and general disorders and administration site conditions. No other clinically significant laboratory test results (blood chemistry, hematology, or urinalysis) were reported during the study. No trends were seen in mean/median baseline in any laboratory parameter. There were no significant trends in mean or median changes from baseline in temperature or pulse throughout the study. No clinically significant abnormalities were seen on physical examination results, ECGs or vital signs.

Subcutaneous administration of mAb1 to adult patients with moderate-to-severe AD was generally safe and well-tolerated.

### G. Efficacy: Results

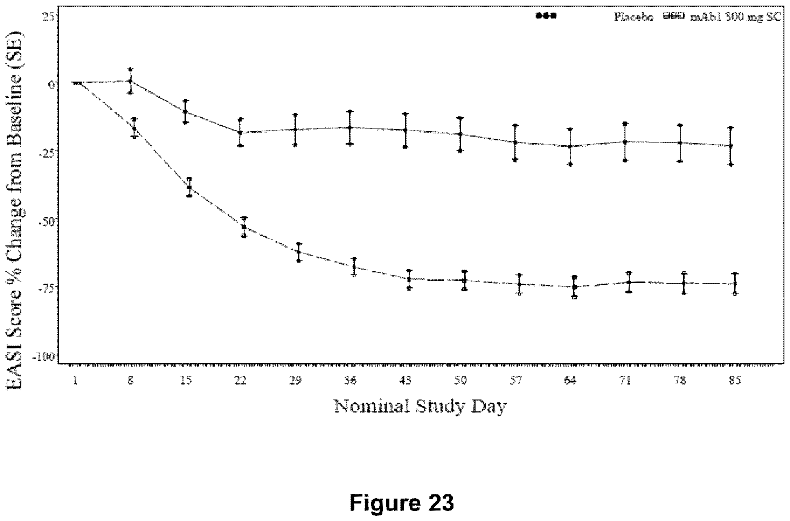

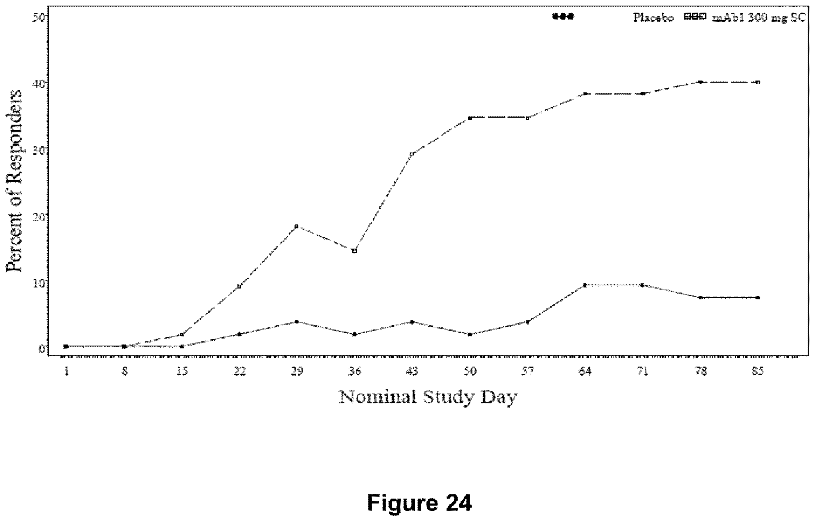

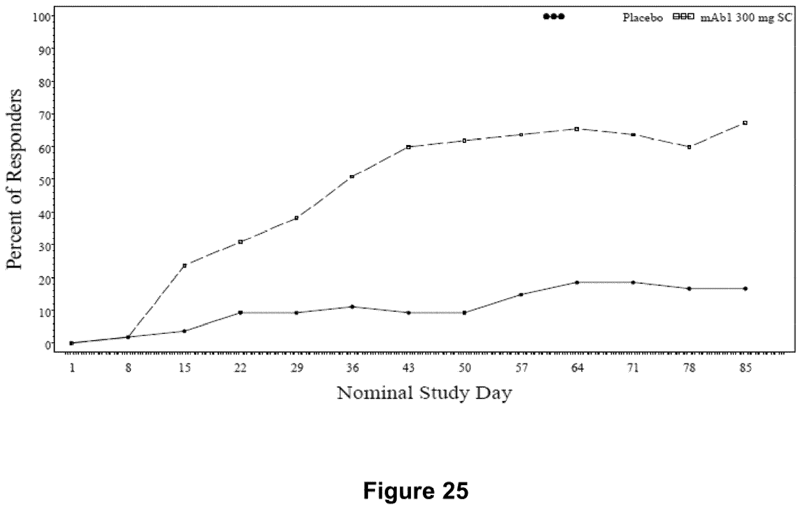

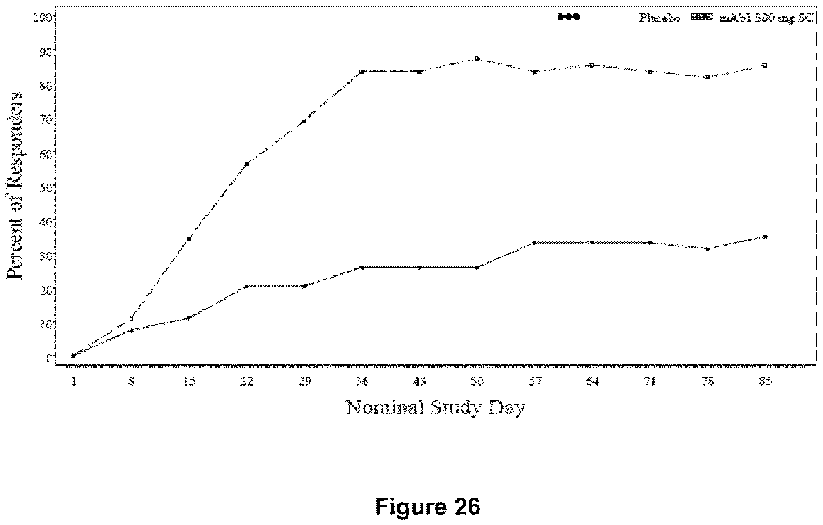

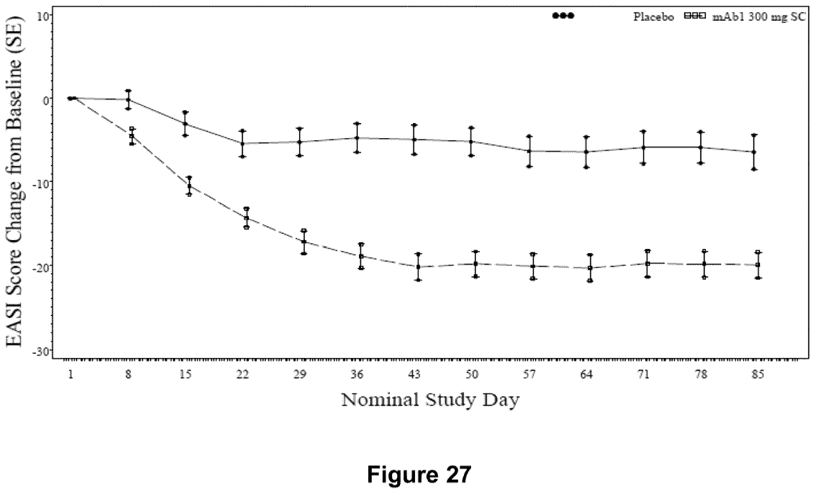

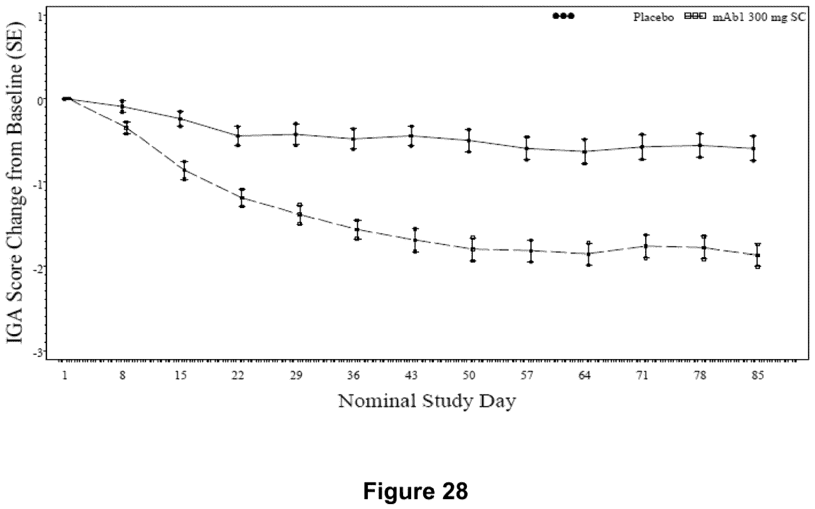

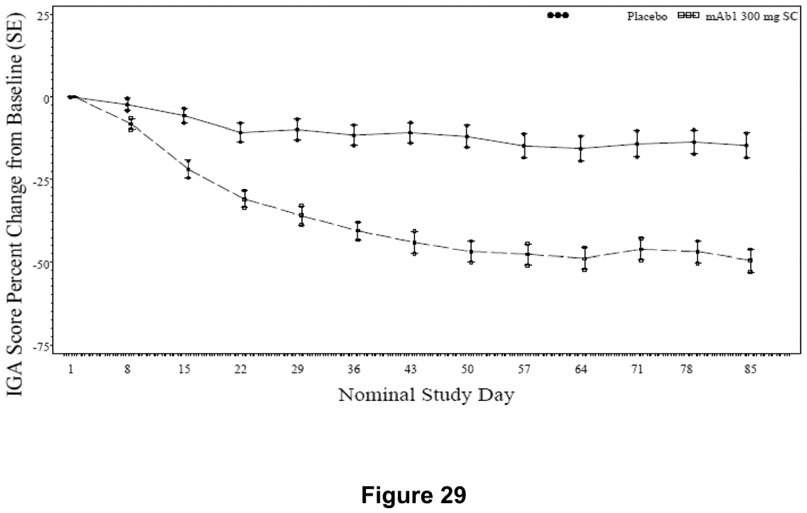

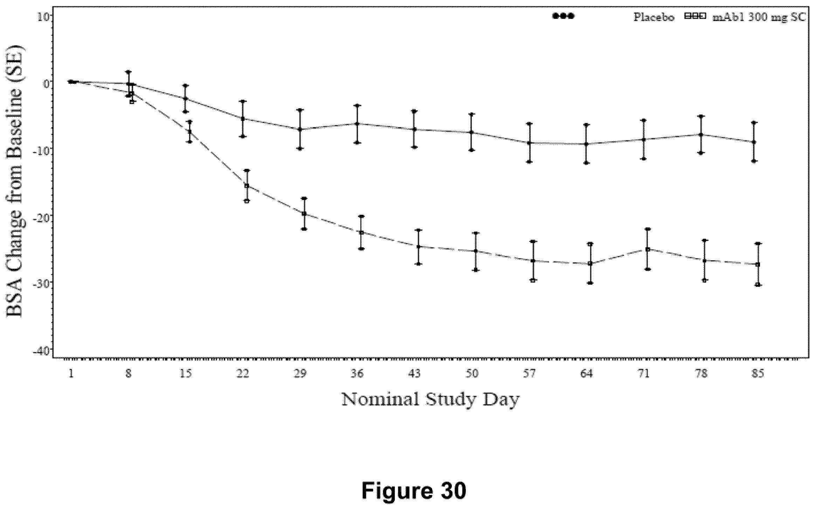

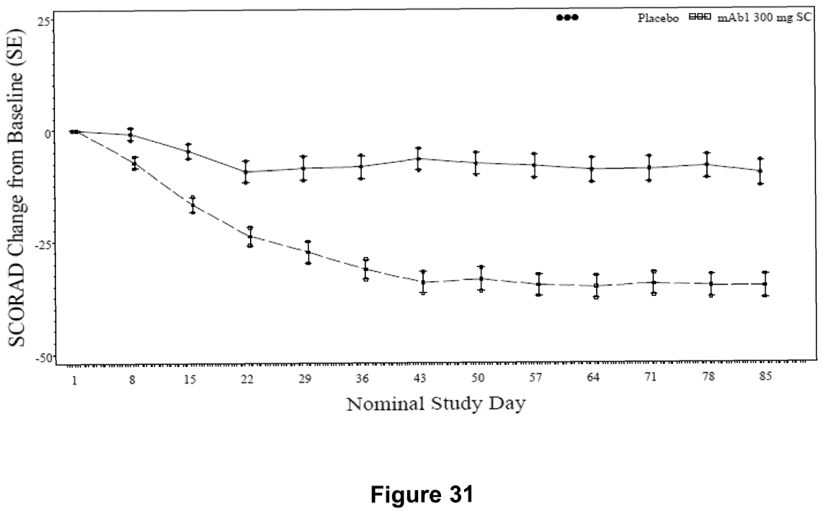

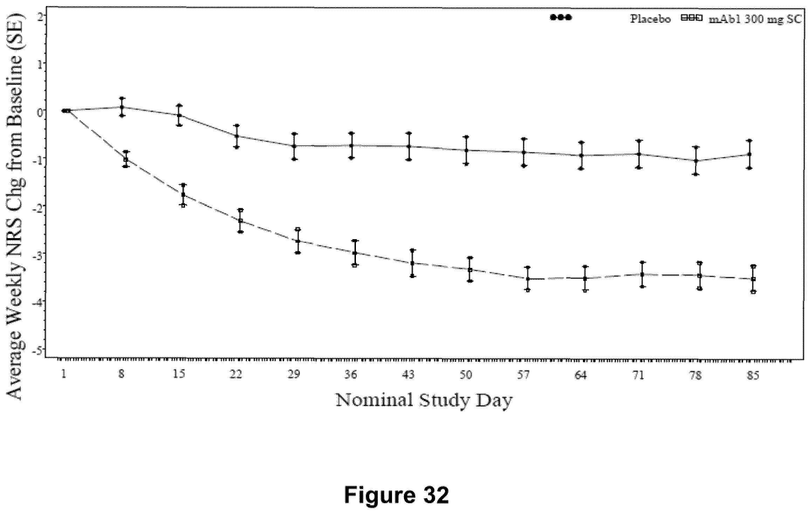

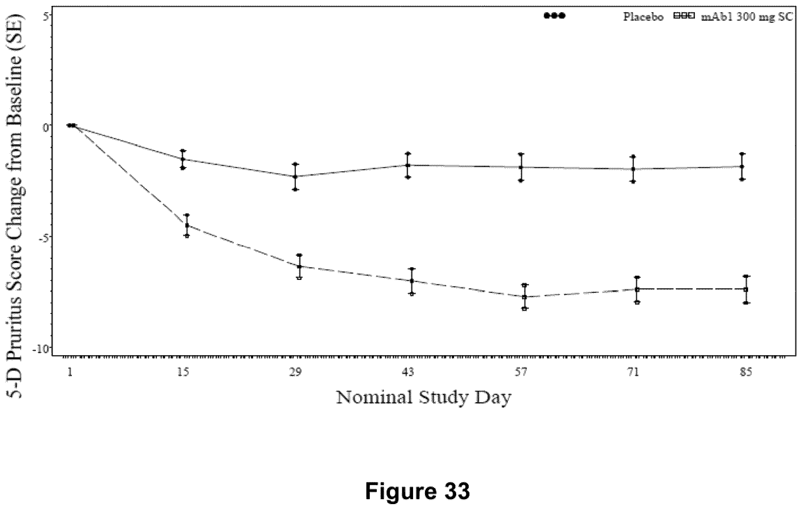

The baseline and exploratory efficacy results obtained from the study are summarized in Figures 23 - 33 and Tables 27 - 35. As noted above, patients were treated with 300 mg subcutaneous mAb1 once a week for 12 weeks, or with placebo.

**Table 27: Summary of Baseline Characteristics - all values represented as Mean (SD)**

| | **Placebo** | **mAb1 300 mg** | **All Subjects Combined** |
|---|---|---|---|
| No. Patients | 54 | 55 | 109 |
| Age (years) Mean (SD) | 39.4 (12.29) | 33.7 (10.41) | 36.5 (11.69) |
| Ethnicity n (%) | | | |
| Hispanic or Latino | 1 (1.9%) | 3 (5.5%) | 4 (3.7%) |
| Not Hispanic or Latino | 53 (98.1%) | 52 (94.5%) | 105 (96.3%) |
| Gender n (%) | | | |
| Male | 27 (50.0%) | 31 (56.4%) | 58 (53.2%) |
| Female | 27 (50.0%) | 24 (43.6%) | 51 (46.8%) |
| Height (cm) Mean (SD) | 171.2 (9.89) | 173.4 (9.88) | 172.3 (9.90) |
| Weight (kg) Mean (SD) | 72.41 (17.539) | 78.13 (17.416) | 75.30 (17.632) |
| BMI (kg/m²) Mean (SD) | 24.51 (4.639) | 25.89 (4.837) | 25.20 (4.768) |
| Chronic Atopic Dermatitis Diagnosis Age | 14.4 (18.35) | 6.6 (10.53) | 10.5 (15.37) |
| BSA | 50.8 (24.14) | 46.8 (24.55) | 48.8 (24.32) |
| EASI Score | 30.8 (13.63) | 28.4 (13.57) | 29.6 (13.59) |
| IGA Score | 4.0 (0.69) | 3.9 (0.67) | 3.9 (0.68) |
| NRS Score | 5.8 (1.93) | 6.1 (1.34) | 5.9 (1.66) |
| SCORAD Score | 69.1 (13.38) | 66.7 (13.82) | 67.9 (13.59) |
| Pruritus 5-D Scale | 18.7 (3.50) | 18.4 (3.04) | 18.5 (3.26) |

**Table 28: Summary of Percentage and Absolute Change in EASI Score from Baseline to Week 12 and Each Visit during Follow-up period - all values represented as Mean (SD)**

| | **Placebo** | **300mg mAb1** |
|---|---|---|
| No. Patients | 54 | 55 |
| Baseline EASI Score | 30.8 (13.63) | 28.4 (13.57) |
| Day **85** EASI Score | 24.4 (19.01) | 8.5 (12.15) |
| % Change from Baseline to Day **85** | -23.3 (49.26) | -74.0 (26.94) |
| Absolute change from Baseline to Day **85** | -6.4 (14.85) | -19.9 (11.52) |
| Day **99** EASI Score | 24.2 (19.15) | 8.4 (11.86) |
| % Change from Baseline to Day **99** | -23.2 (49.42) | -73.5 (27.21) |
| Absolute change from Baseline to Day **99** | -6.6 (15.20) | -20.0 (12.24) |
| Day **113** EASI Score | 24.1 (18.80) | 9.1 (12.13) |
| % Change from Baseline to Day **113** | -23.4 (47.75) | -71.4 (27.03) |
| Absolute change from Baseline to Day **113** | -6.7 (14.96) | -19.4 (11.42) |
| Day **127** EASI Score | 24.5 (18.91) | 9.2 (12.41) |
| % Change from Baseline to Day **127** | -22.1 (47.11) | -71.2 (27.39) |
| Absolute change from Baseline to Day **127** | -6.3 (14.98) | -19.2 (11.15) |
| Day **141** EASI Score | 23.8 (18.47) | 9.4 (12.18) |
| % Change from Baseline to Day **141** | -23.9 (47.01) | -70.8 (26.91) |
| Absolute change from Baseline to Day **141** | -7.0 (14.77) | -19.0 (10.86) |
| Day **155** EASI Score | 24.0 (18.27) | 9.9 (12.40) |
| % Change from Baseline to Day **155** | -23.0 (46.22) | -68.8 (27.35) |
| Absolute change from Baseline to Day **155** | -6.7 (14.49) | -18.5 (10.74) |
| Day **169** EASI Score | 23.5 (18.22) | 11.0 (12.76) |
| % Change from Baseline to Day **169** | -24.2 (46.66) | -64.4 (29.19) |
| Absolute change from Baseline to Day **169** | -7.3 (14.93) | -17.5 (10.82) |
| Day **183** EASI Score | 23.5 (18.57) | 10.8 (13.00) |
| % Change from Baseline to Day **183** | -24.6 (47.35) | -65.0 (29.21) |
| Absolute change from Baseline to Day **183** | -7.3 (15.12) | -17.6 (10.93) |
| Day **197 EASI** Score | 23.4 (18.59) | 11.0 (13.13) |
| % Change from Baseline to Day **197** | -25.0 (48.57) | -64.0 (30.80) |
| Absolute change from Baseline to Day **197** | -7.4 (15.23) | -17.4 (11.88) |

**Table 29: Summary of Percentage and Absolute Change in IGA Score from Baseline to Week 12 and Each Visit during Follow-up period - all values represented as Mean (SD)**

| | **Placebo** | **300mg mAb1** |
|---|---|---|
| No. Patients | 54 | 55 |
| Baseline IGA Score | 4.0 (0.69) | 3.9 (0.67) |
| Day **85** IGA Score | 3.4 (1.19) | 2.0 (1.15) |
| % Change from Baseline to Day **85** | -14.7 (27.37) | -49.5 (25.94) |
| Absolute change from Baseline to Day **85** | -0.6 (1.07) | -1.9 (0.98) |
| Day **99** IGA Score | 3.4 (1.16) | 2.1 (1.17) |
| % Change from Baseline to Day **99** | -14.0 (27.03) | -45.8(26.98) |
| Absolute change from Baseline to Day **99** | -0.6 (1.06) | -1.7 (1.06) |
| Day **113** IGA Score | 3.3 (1.20) | 2.2 (1.08) |
| % Change from Baseline to Day **113** | -15.9 (27.82) | -43.1 (25.53) |
| Absolute change from Baseline to Day **113** | -0.6 (1.12) | -1.7 (1.06) |
| Day **127** IGA Score | 3.4 (1.16) | 2.2 (1.16) |
| % Change from Baseline to Day **127** | -14.5 (26.66) | -44.1 (27.06) |
| Absolute change from Baseline to Day **127** | -0.6 (1.07) | -1.7 (1.07) |
| Day **141** IGA Score | 3.4 (1.15) | 2.2 (1.12) |
| % Change from Baseline to Day **141** | -15.0 (26.52) | -42.8 (26.01) |
| Absolute change from Baseline to Day **141** | -0.6 (1.05) | -1.6 (1.01) |
| Day **155** IGA Score | 3.4 (1.14) | 2.3 (1.08) |
| % Change from Baseline to Day **155** | -14.2 (25.89) | -41.5 (25.20) |
| Absolute change from Baseline to Day **155** | -0.6 (1.02) | -1.6 (1.01) |
| Day **169** IGA Score | 3.3 (1.17) | 2.5 (1.07) |
| % Change from Baseline to Day **169** | -15.9 (26.96) | -36.0 (25.87) |
| Absolute change from Baseline to Day **169** | -0.6 (1.08) | -1.4 (1.03) |
| Day **183** IGA Score | 3.3 (1.18) | 2.4 (1.10) |
| % Change from Baseline to Day **183** | -16.3 (27.33) | -37.2 (26.93) |
| Absolute change from Baseline to Day **183** | -0.7 (1.10) | -1.5 (1.09) |
| Day **197** IGA Score | 3.3 (1.29) | 2.3 (1.09) |
| % Change from Baseline to Day **197** | -16.5 (30.18) | -39.0 (27.42) |
| Absolute change from Baseline to Day **197** | -0.7 (1.20) | -1.5 (1.10) |

**Table 30: Summary of Absolute Change in BSA Score from Baseline to Week 12 and Each Visit during Follow-up period - all values represented as Mean (SD)**

| | **Placebo** | **300mg mAb1** |
|---|---|---|
| No. Patients | 54 | 55 |
| Baseline BSA Score | 50.8 (24.13) | 46.8 (24.55) |
| Day **85** BSA Score | 41.8 (30.44) | 19.4 (23.43) |
| Absolute change from Baseline to Day **85** | -9.0 (21.07) | -27.4 (22.81) |
| Day **99** BSA Score | 41.7 (30.85) | 19.9 (22.85) |
| Absolute change from Baseline to Day **99** | -9.2 (21.85) | -26.9 (22.74) |
| Day **113** BSA Score | 41.3 (30.52) | 20.8 (23.16) |
| Absolute change from Baseline to Day **113** | -9.5 (21.34) | -26.0 (21.90) |
| Day **127** BSA Score | 42.1 (30.41) | 21.4 (23.48) |
| Absolute change from Baseline to Day **127** | -8.7 (20.72) | -25.4 (21.29) |
| Day **141** BSA Score | 41.5 (29.85) | 21.3 (22.88) |
| Absolute change from Baseline to Day **141** | -9.4 (20.57) | -25.5 (21.50) |
| Day **155** BSA Score | 41.5 (29.61) | 22.1 (23.05) |
| Absolute change from Baseline to Day **155** | -9.3 (20.26) | -24.6 (21.55) |
| Day **169** BSA Score | 41.2 (29.28) | 24.6 (24.15) |
| Absolute change from Baseline to Day **169** | -9.6 (20.35) | -22.2 (21.50) |
| Day **183** BSA Score | 41.0 (30.28) | 24.1 (24.15) |
| Absolute change from Baseline to Day **183** | -9.9 (21.35) | -22.7 (22.86) |
| Day **197** BSA Score | 40.5 (29.95) | 24.9 (25.70) |
| Absolute change from Baseline to Day **197** | -10.4 (21.40) | -21.9 (24.11) |

**Table 31: Summary of Absolute Change in SCORAD Score from Baseline to Week 12 and Each Visit during Follow-up period - all values represented as Mean (SD)**

| | **Placebo** | **300mg mAb1** |
|---|---|---|
| No. Patients | 54 | 55 |
| Baseline SCORAD Score | 69.1 (13.38) | 66.7 (13.82) |
| Day **85** SCORAD Score | 59.3 (23.44) | 31.7 (22.08) |
| Absolute change from Baseline to Day **85** | -9.8 (20.53) | -35.0 (19.43) |
| Day **99** SCORAD Score | 58.8 (23.35) | 32.5 (20.99) |
| Absolute change from Baseline to Day **99** | -10.3 (21.33) | -34.3 (18.94) |
| Day **113** SCORAD Score | 59.1 (22.30) | 34.0 (2051) |
| Absolute change from Baseline to Day **113** | -10.0 (20.89) | -32.7 (18.48) |
| Day **127** SCORAD Score | 59.9 (22.36) | 34.0 (21.25) |
| Absolute change from Baseline to Day **127** | 09.2 (20.59) | -32.7 (18.23) |
| Day **141** SCORAD Score | 59.0 (21.85) | 33.9 (20.51) |
| Absolute change from Baseline to Day **141** | -10.1 (20.12) | -32.8 (17.97) |
| Day **155** SCORAD Score | 59.0 (22.50) | 35.1 (20.16) |
| Absolute change from Baseline to Day **155** | -10.0 (20.17) | -31.6 (17.99) |
| Day **169** SCORAD Score | 58.5 (22.33) | 37.1 (20.82) |
| Absolute change from Baseline to Day **169** | -10.6 (20.90) | -29.6 (19.15) |
| Day **183** SCORAD Score | 58.7 (22.47) | 37.5 (20.89) |
| Absolute change from Baseline to Day **183** | -10.4 (20.86) | -29.2 (19.50) |
| Day **197** SCORAD Score | 57.8 (23.82) | 38.8 (22.04) |
| Absolute change from Baseline to Day **197** | -11.3 (22.05) | -27.9 (21.70) |

**Table 32: Summary of Absolute Change in 5-D Pruritus Scale from Baseline to Week 12 and Each Week during Follow-up period- all values represented as Mean (SD)**

| | **Placebo** | **300mg mAb1** |
|---|---|---|
| No. Patients | 54 | 55 |
| Baseline 5-D Pruritus Score | 18.7 (3.50) | 18.4 (3.04) |
| Day **85** 5-D Pruritus Score | 16.9 (5.33) | 11.0 (4.22) |
| Absolute change from Baseline to Day **85** | -1.9 (4.28) | -7.4 (4.33) |
| Day **99** 5-D Pruritus Score | 16.7 (5.28) | 11.3 (3.96) |
| Absolute change from Baseline to Day **99** | -2.0 (4.63) | -7.0 (4.41) |
| Day **113** 5-D Pruritus Score | 16.5 (5.57) | 11.7 (4.05) |
| Absolute change from Baseline to Day **113** | -2.2 (4.91) | -6.7 (4.21) |
| Day **127** 5-D Pruritus Score | 16.7 (5.44) | 11.5 (4.07) |
| Absolute change from Baseline to Day **127** | -2.0 (4.72) | -6.9 (4.24) |
| Day **141** 5-D Pruritus Score | 16.4 (5.67) | 11.8 (4.19) |
| Absolute change from Baseline to Day **141** | -2.3 (5.12) | -6.6 (4.56) |
| Day **155** 5-D Pruritus Score | 16.6 (5.53) | 12.0 (4.21) |
| Absolute change from Baseline to Day **155** | -2.1 (4.90) | -6.4 (4.49) |
| Day **169** 5-D Pruritus Score | 16.8 (5.35) | 12.7 (4.20) |
| Absolute change from Baseline to Day **169** | -1.9 (4.78) | -5.7 (4.58) |
| Day **183** 5-D Pruritus Score | 16.6 (5.59) | 12.8 (4.56) |
| Absolute change from Baseline to Day **183** | -2.1 (5.02) | -5.6 (4.90) |
| Day **197** 5-D Pruritus Score | 16.6 (5.50) | 13.1 (4.85) |
| Absolute change from Baseline to Day **197** | -2.1 (5.12) | -5.3 (5.06) |

**Table 33: Summary of Absolute Change in Average NRS Score from Baseline to Week 12 and Each Week during Follow-up period - all values represented as Mean (SD)**

| | **Placebo** | **300mg mAb1** |
|---|---|---|
| No. Patients | 54 | 55 |
| Baseline NRS Score | 5.8 (1.93) | 6.1 (1.34) |
| Day **85** NRS Score | 4.9 (2.53) | 2.6 (1.67) |
| Absolute change from Baseline to Day **85** | -0.9 (2.07) | -3.5 (2.00) |
| Day **92** NRS Score | 4.8 (2.57) | 2.8 (1.68) |
| Absolute change from Baseline to Day **92** | -1.0 (2.07) | -3.4 (2.12) |
| Day **99** NRS Score | 4.7 (2.54) | 2.7 (1.72) |
| Absolute change from Baseline to Day **99** | -1.0 (2.06) | -3.4 (2.17) |
| Day **106** NRS Score | 4.8 (2.59) | 2.7 (1.63) |
| Absolute change from Baseline to Day **106** | -1.0 (2.15) | -3.4 (2.08) |
| Day **113** NRS Score | 4.9 (2.69) | 2.7 (1.63) |
| Absolute change from Baseline to Day **113** | -0.9 (2.21) | -3.4 (2.00) |
| Day **120** NRS Score | 4.8 (2.61) | 2.7 (1.68) |
| Absolute change from Baseline to Day **120** | -1.0 (2.18) | -3.4 (2.07) |
| Day **127** NRS Score | 4.8 (2.68) | 2.8 (1.79) |
| Absolute change from Baseline to Day **127** | -1.0 (2.24) | -3.3 (2.20) |
| Day **134** NRS Score | 4.7 (2.75) | 2.8 (1.78) |
| Absolute change from Baseline to Day **134** | -1.1 (2.24) | -3.3 (2.18) |
| Day **141** NRS Score | 4.7 (2.73) | 2.9 (1.89) |
| Absolute change from Baseline to Day **141** | -1.1 (2.26) | -3.2 (2.28) |
| Day **148** NRS Score | 4.7 (2.75) | 2.9 (1.89) |
| Absolute change from Baseline to Day **148** | -1.1 (2.28) | -3.2 (2.28) |
| Day **155** NRS Score | 4.7 (2.75) | 2.9 (1.86) |
| Absolute change from Baseline to Day **155** | -1.1 (2.30) | -3.2 (2.19) |
| Day **162** NRS Score | 4.7 (2.75) | 3.0 (1.93) |
| Absolute change from Baseline to Day **162** | -1.1 (2.29) | -3.1 (2.28) |
| Day **169** NRS Score | 4.7 (2.75) | 3.2 (1.99) |
| Absolute change from Baseline to Day **169** | -1.1 (2.28) | -3.0 (2.43) |
| Day **176** NRS Score | 4.7 (2.74) | 3.2 (2.01) |
| Absolute change from Baseline to Day **176** | -1.1 (2.27) | -3.0 (2.49) |
| Day **183** NRS Score | 4.7 (2.75) | 3.1 (1.97) |
| Absolute change from Baseline to Day **183** | -1.1 (2.28) | -3.0 (2.41) |
| Day **190** NRS Score | 4.7 (2.78) | 3.1 (1.91) |
| Absolute change from Baseline to Day **190** | -1.1 (2.31) | -3.1 (2.25) |
| Day **197** NRS Score | 4.7 (2.75) | 3.1 (1.95) |
| Absolute change from Baseline to Day **197** | -1.1 (2.28) | -3.0 (2.28) |

**Table 34: Summary of Subjects achieving an IGA score of 0 or 1 to Week 12 and each visit during Follow-up period**

| Number and proportion of subjects achieving an IGA score of 0 or 1 | Placebo (N=54) | 300 mg mAb1 (N=55) |
|---|---|---|
| Week 12, Day 85 | 4 (7.4%) | 22 (40.0%) |
| Week 14, Day 99 | 4 (7.4%) | 22 (40.0%) |
| Week 16, Day 113 | 5 (9.3%) | 18 (32.7%) |
| Week 18, Day 127 | 3 (5.6%) | 20 (36.4%) |
| Week 20, Day 141 | 4 (7.4%) | 17 (30.9%) |
| Week 22, Day 155 | 3 (5.6%) | 17 (30.9%) |
| Week 24, Day 169 | 3 (5.6%) | 13 (23.6%) |
| Week 26, Day 183 | 3 (5.6%) | 15 (27.3%) |
| Week 28, Day 197 | 6 (11.1%) | 16 (29.1%) |

**Table 35: Summary of Subjects achieving an EASI 50 Week 12 and each visit during Follow-up period**

| Number and proportion of subjects achieving an EASI score percent decrease of 50% | Placebo (N=54) | 300 mg mAb1 (N=55) |
|---|---|---|
| Week 12, Day 85 | 19 (35.2%) | 47 (85.5%) |
| Week 14, Day 99 | 19 (35.2%) | 46 (83.6%) |
| Week 16, Day 113 | 18 (33.3%) | 46 (83.6%) |
| Week 18, Day 127 | 18 (33.3%) | 45 (81.8%) |
| Week 20, Day 141 | 18 (33.3%) | 46 (83.6%) |
| Week 22, Day 155 | 16 (29.6%) | 43 (78.2%) |
| Week 24, Day 169 | 18 (33.3%) | 40 (72.7%) |
| Week 26, Day 183 | 19 (35.2%) | 41 (74.5%) |
| Week 28, Day 197 | 23 (42.6%) | 40 (72.7%) |

### H. Conclusions

Subcutaneous administration of an anti-IL-4R antibody (mAb1) to adult patients with moderate-to-severe atopic dermatitis was generally safe and well tolerated after 12 weekly doses of 300 mg. Administration of mAb1 at 300 mg resulted in significant improvement in IGA, EASI, BSA, SCORAD and NRS pruritus through day 85 in both mean and absolute and percent change, as compared to baseline (see Tables 27 - 33). The proportion of patients achieving an IGA score of 0 or 1 at Day 85 for the 300mg group was 40.0%, while the same number for placebo was 7.4% (Table 34). At Day 85, the proportion of patients who achieved an EASI score percent decrease of 50% ("EASI-50") was 85.5% for the 300 mg group, whereas the EASI-50 for placebo-treated patients at Day 85 was 35.2% (Table 35). The percent change in EASI score from baseline to week 12 of mAb1 was statistically significant from placebo group (-74.0% vs. -23.0%, p-value<0.0001). The treatment group was statistically significantly different from placebo group in all of the secondary efficacy endpoints. The following were the p-values for: IGA responder (0 or 1) (<0.0001), EASI responder (<0.0001), EASI absolute change from baseline (<0.0001), absolute change of IGA from baseline (<0.0001), percent change of IGA from baseline (<0.0001), absolute change in BSA (<0.0001), absolute change in SCORAD (<0.0001), absolute change in Pruritus NRS (<0.0001), and absolute change in 5-D pruritus scale from baseline to week 12 (<0.0001) respectively.

### Example 11: Clinical Trial to Assess the Safety of mAb1 Administered Concomitantly with Topical Corticosteroids to Patients with Moderate-to-severe AD

### A. Study Design

This study was a randomized, double-blind, parallel-group, placebo-controlled study to assess the safety and explore the efficacy of repeated subcutaneous doses of mAb1 administered concomitantly with topical corticosteroids (TCS) to treat AD in patients with moderate-to-severe AD. Patients were randomized 2:1 to receive 300 mg mAb1 or placebo once weekly for 4 consecutive weeks via subcutaneous injections (on days 1, 8, 15, and 22). All patients received concomitant open-label, daily treatment for up to 28 days with a potent TCS product (50-100 times as potent as hydrocortisone), such as methylprednisolone aceponate 0.1%, mometasone furoate 0.1%, or betamethasone valerate 0.1%. Other topical medications, such as a lower potency TCS or topical calcineurin inhibitors (TCI), were used to treat AD lesions located on the face, flexural and genital areas.

Starting with the screening visit, patients began applying an additive-free, basic bland emollient twice daily for at least 7 days before the baseline visit and continued its use throughout the study (once daily on treatment days in areas of TCS application). Environmental control measures and non-pharmacologic treatment modalities such as allergen avoidance and bleach baths were allowed at the discretion of the investigator.

Screening occurred between day -21 and -1. Patients received their first drug injection (300 mg mAb1, or placebo) on day 1 and returned to the clinic for additional drug injections on days 8, 15 and 22 (+/- 1 day) for a total of 4 weekly doses. Starting on day 1, patients applied the topical medication(s) described above once daily in the evening, and continued the applications to all AD-affected areas (i.e., areas with active AD lesions) until control was achieved for up to 28 days. After control was achieved, TCS application to AD-prone areas without active lesions (i.e., areas from which lesions have cleared) was limited to 2 days every week until study day 28. After day 28, topical treatment of any residual AD lesions continued as needed. Throughout the study, patients continued to apply an additive-free, basic bland emollient, twice daily (once daily on topical treatment days in areas treated with topical medication). Patients returned for clinic visits on days 29, 36, 50, 64 and 78 (end of study).

The inclusion criteria for the study were: (1) male/female patients aged 18 years or older; (2) chronic AD as diagnosed by the Eichenfield revised criteria of Hannifin and Rajka, that had been present for at least 2 years before screening; (3) AD activity as assessed by IGA score ≥ 3 and SCORAD > 20 at the screening and baseline visits, with one or more active AD lesions for which treatment with potent TCS is indicated; (4) at least 10% BSA affected by AD at the screening and baseline visits; (5) patients must be applying an additive-free, basic bland emollient twice daily for at least 7 days before the baseline visit; (6) willing and able to comply with clinic visits and study-related procedures; and (7) able to read and understand, and willing to sign the consent form.

The exclusion criteria for the study were: (1) prior treatment with mAb1; (2) hypersensitivity to corticosteroids or to any other ingredients contained in the TCS product used during the study; (3) AD lesions located predominantly (≥ 50% of the cumulative lesional area) on face, flexural and genital areas; (4) presence of skin comorbidities that may interfere with study assessments; (5) the following treatments within 4 weeks before the baseline visit or any conditions that may require such treatment(s)during the study: systemic corticosteroids, immunosuppressive or immunomodulating drugs, eg, cyclosporine, mycophenolate-mofetil, IFN-gamma, azathioprine or methotrexate; (6) treatment with biologics as follows: (a) any cell-depleting agents, including but not limited to, rituximab; within 6 months prior to the baseline visit, or until lymphocyte and CD19+ lymphocyte count return to normal, whichever is larger, (b) infliximab, adalimumab, golimumab, certolizumab pegol, abatacept, etanercept, anakinra: within 8 weeks prior to the baseline visit, and 9c) other biologics: within 5 half-lives (if known) or 8 weeks, whichever is longer; (7) any phototherapy for skin disease (such as narrow band UVB, UVB, UVA1, psolaren+UVA within 4 weeks before baseline; (8) regular use (more than 2 visits per week) of a tanning booth/parlor within 4 weeks before the baseline visit; (9) treatment with a live attenuated vaccine within 12 weeks before the baseline visit; (10) treatment with an investigational drug within 8 weeks or within 5 half-lives, whichever is longer, before the baseline visit; (11) chronic or acute infection requiring treatment with oral or IV antibiotics, antivirals, antiparasitics, antiprotozoals, or antifungals within 4 weeks before the screening, or superficial skin infections within1 week before the screening visit; (12) history of invasive opportunistic infections such as histoplasmosis, listeriosis, coccidioidomycosis, candidiasis, pneumocystis jiroveci, aspergillosis, despite resolution, JC virus (progressive multifocal leukoencephalopathy; (13) known history of HIV infection; (14) positive or indeterminate hepatitis B surface antigen, hepatitis B core antibody, or hepatitis C antibody at the screening visit; (15) presence of any of the following laboratory abnormalities at the screening visit: creatine phosphokinase > 2x upper limit normal (ULN); aspartate aminotransferase (AST) and/or alanine aminotransferase (ALT) > 2x ULN; neutrophil count < 1.75 x 10³/ul; platelet count < 100 x 10³/ul; (16) onset of a new exercise routine or major change to a previous exercise routine within 2 weeks before randomization, or unwillingness to maintain (without increase) the current level of physical activity throughout the length of participation in the study; (17) history of a hypersensitivity reaction to doxycycline or other tetracyclines; (18) history of a clinical endoparasite infection within 12 months of the baseline visit, other than treated vaginal trichomoniasis; (19) history of malignancy within 5 years before the baseline visit, except completely treated in situ carcinoma of the cervix, completely excised non-metastatic squamous or basal cell carcinoma of the skin; (20) history of non-malignant lymphoproliferative disorders; (21) pregnant or breast-feeding women; (22) men or women of child-bearing potential who are unwilling to practice contraceptive measures; (23) history of alcohol or drug abuse within 2 years of the screening visit; (24) recent travel (within 12 months of randomization) to areas endemic for parasitic infections, such as developing countries in Africa or the tropical/subtropical regions of Asia; (25) prior or current history of significant concomitant illness(es) that would adversely affect the patient's participation in the study, e.g., stage III or IV cardiac failure, severe renal, neurological, endocrinological, Gl, hepato-biliary, metabolic, pulmonary or lymphatic disease; and (26) any other conditions that may present an unreasonable risk to the study patient, or may make the patient's participation unreliable, or may interfere with study assessments.

The primary endpoint of the study was the incidence and severity of adverse events. The secondary endpoints were exploratory in nature and included: (1) EASI50 index - Binary response variable of whether or not ≥ 50% reduction in EASI is achieved from baseline to day 29, and other post-baseline observation time points; (2) achieving IGA scores of ≤ 1 (clear or almost clear) at day 29, and at other post-baseline observation time points; (3) time to IGA≤1 and to EASI50; (4) changes in IGA, EASI, and SCORAD scores from baseline to day 29, and to other post-baseline observation time points; and (5) proportion of patients with IGA ≤ 1 at week 4 who remain relapse-free through the end of the observation period.

### B. Efficacy variables

The efficacy variables IGA, BSA, EASI, SCORAD, and Pruritus NRS rating have been described elsewhere herein (see, e.g., Example 7). The IGA, BSA, EASI, pruritus NRS and SCORAD scores were assessed at every clinic visit.

### C. Procedures and assessments

Safety was assessed by evaluating the incidence of adverse events (described elsewhere herein) (AEs) from day 1 to day 78, and by detailed medical history, thorough physical examination, vital signs, electrocardiograms (ECGs), and clinical laboratory testing. Blinded safety data was reviewed on an ongoing basis. Concomitant medications and procedures were collected from screening to day 78 (end of study) or early termination, if applicable. Safety, laboratory, and efficacy assessments were performed at each clinic visit. Blood samples were collected for the determination of systemic trough concentrations of functional mAb1 at every study visit prior to treatment starting at baseline (day 1). Blood samples were collected for the analysis of anti-mAb1 antibody levels at predetermined time points. Research samples and samples for exploratory biomarker analysis were also collected. Efficacy of mAb1 was assessed by the EASI, the IGA, the SCORAD, the Pruritus numerical rating scale (NRS), and % body surface area (BSA) of AD involvement. Blood samples were collected for pharmacokinetic (PK) analyses, and analysis of anti-mAb1 antibody levels at predetermined time points. Research samples and samples for exploratory biomarker analysis were also collected.

### D. Statistical Methods

As all statistical analyses described in this section were exploratory in nature, there was no multiplicity adjustment for the type I error. Each test was at the 5% significance level. All categorical variables (EASI-50 and IGA responders at each post-baseline visit, IGA responders at day 29 without later relapse) were analyzed using the Fisher's Exact test with calculated nominal p-value from comparison between mAb1 and placebo groups. The point estimates and confidence intervals of proportions were presented. The graphs of the proportions over time were provided. All continuous variables (change or percent change in IGA, EASI and SCORAD, NRS from baseline to each post-baseline visit) were analyzed by the ANalysis of COVAriance (ANCOVA). Unless otherwise specified, assessments of changes from baseline and construction of confidence intervals for continuous measures were based on an ANCOVA model which included treatment as the main factor and baseline value as covariates. The point estimate and 95% Cl of the difference in adjusted mean change from baseline between two treatment groups was provided. The nominal p-value from comparison between mAb1 and placebo groups will be provided. In the event that the model assumptions were not warranted, the Rank-based analysis of covariates was used. Graphs of mean change from baseline over time were provided. Time-to-event variables (time to EASI50 and time to IGA response) were analyzed with a log-rank test to compare mAb1 with placebo group. Kaplan-Meier survival curves across two treatment groups were provided. The following analysis approaches were implemented for this study: (a) Censored LOCF: The efficacy data was set to missing after prohibited medication was used or after the patient was discontinued from the study. Then all missing values were imputed by simple LOCF. (b) Simple Observed Case (OC) approach: Only observed cases were analyzed.

### E. Safety

Overall, mAb1 was safe and well tolerated in this study. No deaths were reported. A single serious adverse event (SAE) was recorded for a patient in the placebo group, who experienced loss of consciousness, and who withdrew from the study as a result. No other patients experienced adverse events leading to treatment discontinuation. A total of 19 out of 31 patients enrolled in the study reported at least one treatment emergent adverse event (TEAE) - 7 patients (70%) in the placebo group and 12 patients (57%) in the mAb1 group. By system and organ class (SOC), the most frequent TEAE reported for mAb1 treatment group were infections and infestations, 12 patients (57%) vs. 3 patients (30%) for placebo). The most frequent infection was nasopharyngitis - 5 patients (24%) in the mAb1 group vs. 2 patients (20%) for placebo. There were no serious or opportunistic infections. Other TEAEs reported in more than a single patient included nonspecific symptoms such as headache - 3 patients (14%) in the mAb1 group vs. 1 patient (10%) in the placebo group, somnolence - 2 patients (9.5%) in the mab1 group vs. 0% in the placebo group, oropharyngeal pain - 3 patients (14%) in the mAb1 group vs. 1 patient (10%) in the placebo group, and cough - 2 patients (9.5%) in the mAb1 group vs. 0% in the placebo group. Most AEs were mild to moderate and generally resolved within 2 weeks. A single severe AE was reported in the mAb1 group: bacterial bronchitis, with onset on Day 63 (last dose of study drug on Day 22), which was considered unrelated to the study treatment. There were no adverse events in the mAb1 group suggestive of untoward drug-drug (mAb1 - TCS) interactions at the skin level. The analysis of on-treatment potential clinically significant values (PCSV) for safety laboratory tests, vital signs, and ECC showed that the rate of PCSVs was generally balanced between the two study groups, with no systematic distribution or distinct trends, suggesting that PCSV occurrence was incidental and not related to the study treatment.

### F. Results

In this study, mAb1 was administered concomitantly with TCS to patients with moderate to severe AD. Consistent with the current standard of care in AD, a controlled TCS regimen was required during the first 4 weeks (i.e., concomitantly with the study treatment), as described elsewhere herein. Table 36 lists the TCS medications used by the patients participating in the study. Patients were to apply TCS to all active lesions, once daily, every day, until lesion clearance, followed by applications to lesion-prone areas (from which lesions had cleared) once daily, two days per week. A potent TCS (Class III) was required to be applied to at least 50% of lesions. For lesions located on face, skin folds, or genital areas (where potent TCS are usually not indicated) lower potency TCS (Class I or II) were allowed. The amount of TCS used each week was measured by weighing the TCS containers at the time they were dispensed to patients and upon their return to the clinic at the next study visit. Tables 37 and 38 summarize the TCS use from day 1 through day 29.

**Table 36: TCS medications**

| | Placebo (N=10) | 300 mg mAb1 (N=21) | All patients (N=31) |
|---|---|---|---|
| Subjects with at least one TCS med | 10 (100%) | 21 (100%) | 31 (100%) |
| Corticosteroids, Dermatological Preparations | 10 (100%) | 21 (100%) | 31 (100%) |
| Corticosteroids, Potent (Group III) | 10 (100%) | 21 (100%) | 31 (100%) |
| Mometasone (furoate) | 6 (60%) | 10 (47.6%) | 16 (51.6%) |
| Methylprednisolone (aceponate) | 6 (60%) | 8 (38.1%) | 14 (45.2%) |
| Fluticasone propionate | 0 | 4 (19.0%) | 4 (12.9%) |
| Corticosteroids, Moderately potent (Group II) | 0 | 2 (9.5%) | 2 (6.5%) |
| Hydrocortisone butyrate | 0 | 2 (9.5%) | 2 (6.5%) |
| Corticosteroids, Weak (Group I) | 2 (20.0%) | 1 (4.8%) | 3 (9.7%) |
| Hydrocortisone | 2 (20.0%) | 1 (4.8%) | 3 (9.7%) |

**Table 37: TCS used from Day 1 through Day 29 - all values in Mean (SD)**

| | Placebo (N=10) | 300 mg mAb1 (N=21) | All patients (N=31) |
|---|---|---|---|
| Total (g) | 99.4 (152.49) | 48.7 (40.27) | 65.0 (92.94) |
| Visit 3 (week 1) | 26.7 (37.53) | 14.0 (13.13) | 18.1 (23.96) |
| Visit 4 (week 2) | 2.5 (40.03) | 12.7 (9.96) | 16.5 (24.04) |
| Visit 5 (week 3) | 26.6 (37.05) | 11.5 (12.28) | 16.3 (23.75) |
| Visit 6 (week 4) | 26.9 (43.61) | 11.1 (14.71) | 15.6 (26.43) |

**Table 38: TCS in Class III used from Day 1 through Day 29**

| | Placebo (N=10) | 300 mg mAb1 (N=21) | All patients (N=31) |
|---|---|---|---|
| Total (g) | 93.4 (153.51) | 47.2 (39.78) | 62.1 (92.77) |
| Visit 3 (week 1) | 25.2 (37.83) | 13.5 (12.86) | 17.3 (23.89) |
| Visit 4 (week 2) | 23.0 (40.26) | 12.4 (9.76) | 15.8 (23.98) |
| Visit 5 (week 3) | 25.0 (37.34) | 11.0 (12.23) | 15.5 (23.71) |
| Visit 6 (week 4) | 25.2 (44.00) | 11.4 (14.92) | 15.5 (26.78) |

The demographic and disease characteristics were for the most part homogeneous between the two treatment groups (Tables 39 and 40). The mean baseline AD disease severity scores (IGA, EASI, SCORAD, BSA, and pruritus NRS) were reasonably balanced, as well.

**Table 39: Summary of Demographics**

| | Placebo (N=10) | 300 mg (N=21) | All Patients (N=31) |
|---|---|---|---|
| Mean age, years (SD) | 37.8 (16.73) | 36.0 (11.26) | 36.6 (13.01) |

| Race, % | | | |
|---|---|---|---|
| Caucasian | 10 (100%) | 20 (95.2%) | 30 (96.8%) |
| Other | 0 | 1 (4.8%) | 1 (3.2%) |

| Ethnicity, % | | | |
|---|---|---|---|
| Non-Hispanic | 10 (100%) | 21 (100%) | 31 (100%) |

| Gender, % | | | |
|---|---|---|---|
| Male | 5 (50.0%) | 8 (38.1%) | 13 (41.9%) |
| Female | 5 (50.0%) | 132 (61.9%) | 18 (58.1%) |
| Mean BMI, kg/m³ (SD) | 23.92 (3.469) | 25.26 (3.257) | 24.83 (3.330) |

**Table 40: Summary of Baseline Characteristics - all values as Mean (SD)**

| | **Placebo** | **mAb1 300 mg** | **All Subjects Combined** |
|---|---|---|---|
| No. Patients | 10 | 21 | 31 |
| Chronic Atopic Dermatitis Diagnosis Age | 5.4 (9.26) | 5.3 (11.12) | 5.4 (10.40) |
| BSA | 38.85 (24.052) | 40.43 (20.912) | 39.92 (21.579) |
| EASI Score | 24.10 (12.695) | 23.12 (12.354) | 23.43 (12.261) |
| IGA Score | 3.35 (0.474) | 3.43 (0.598) | 3.40 (0.554) |
| NRS Score | 5.00 (1.394) | 6.43 (2.002) | 5.97 (1.928) |
| SCORAD Score | 58.20 (13.834) | 66.31 (13.013) | 63.69 (13.607) |

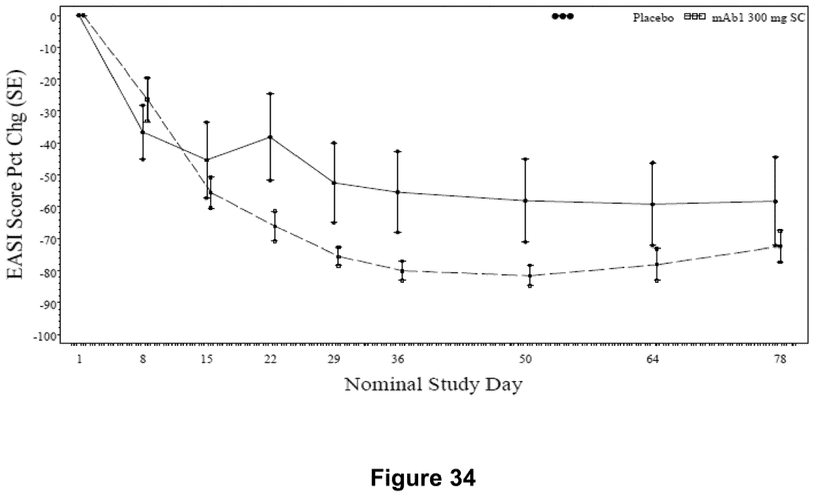

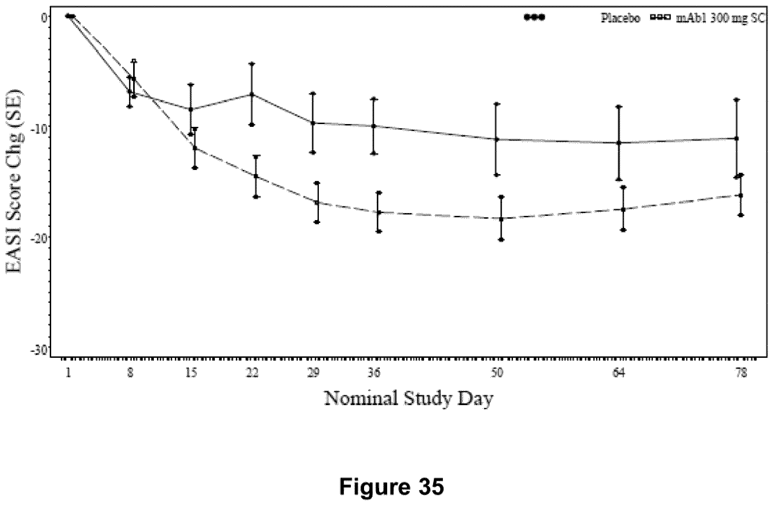

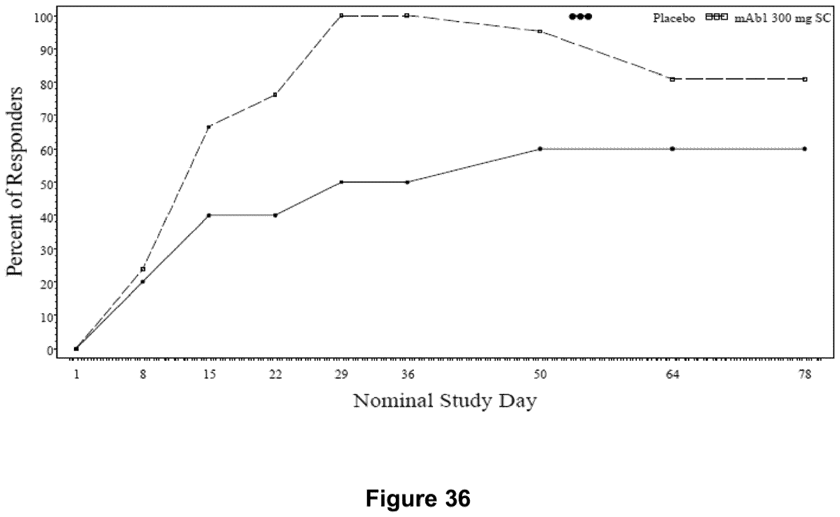

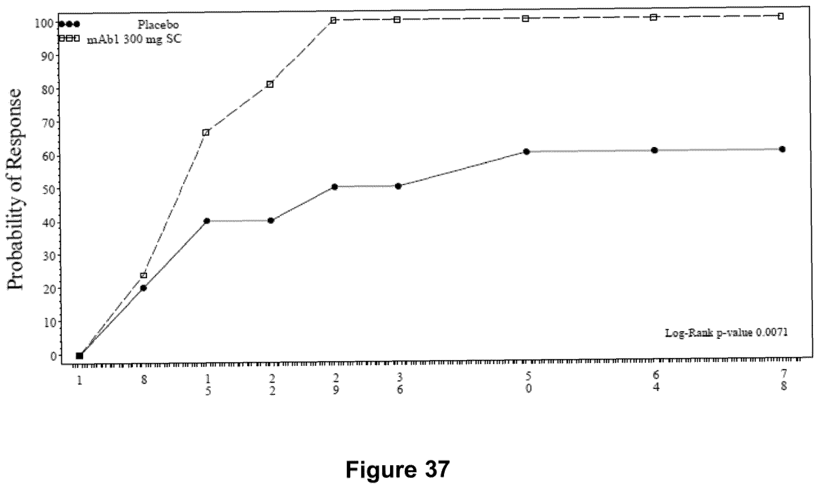

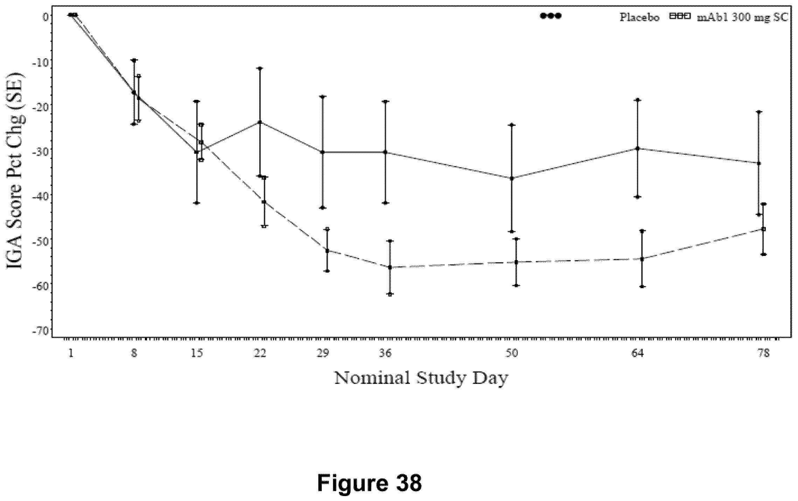

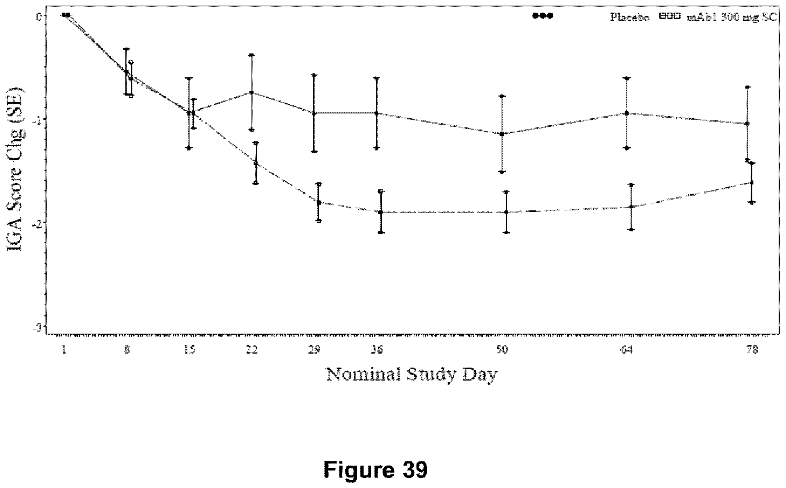

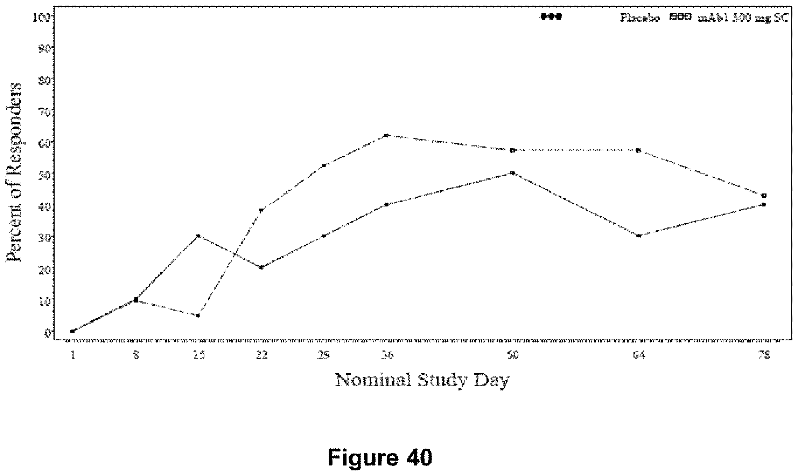

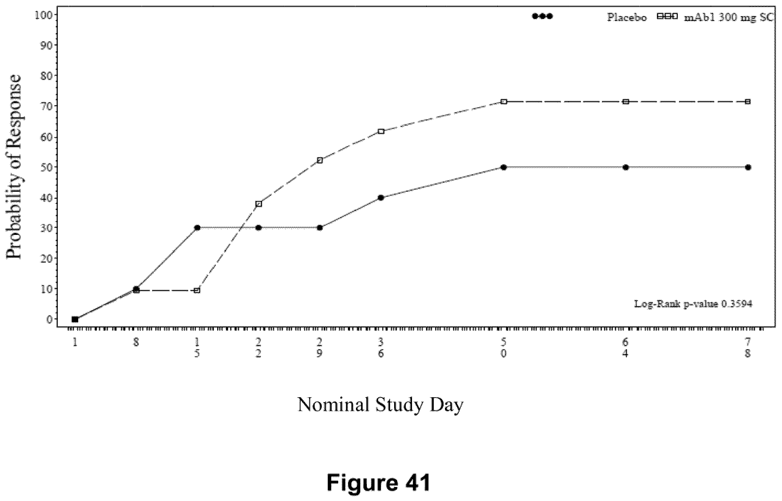

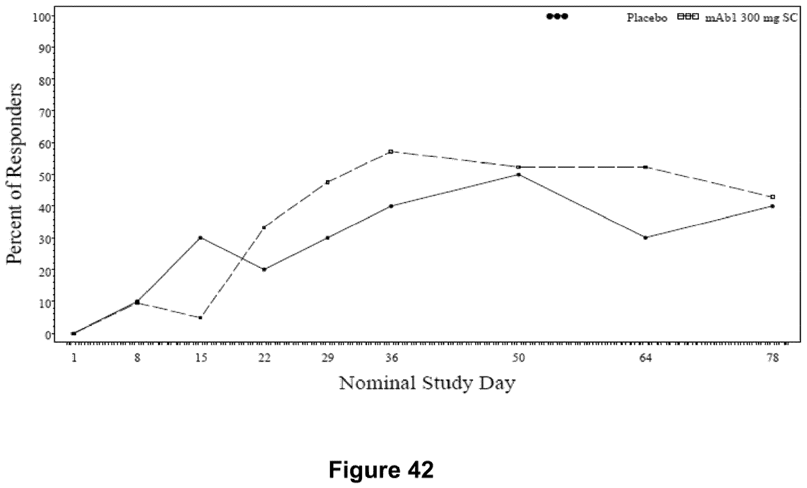

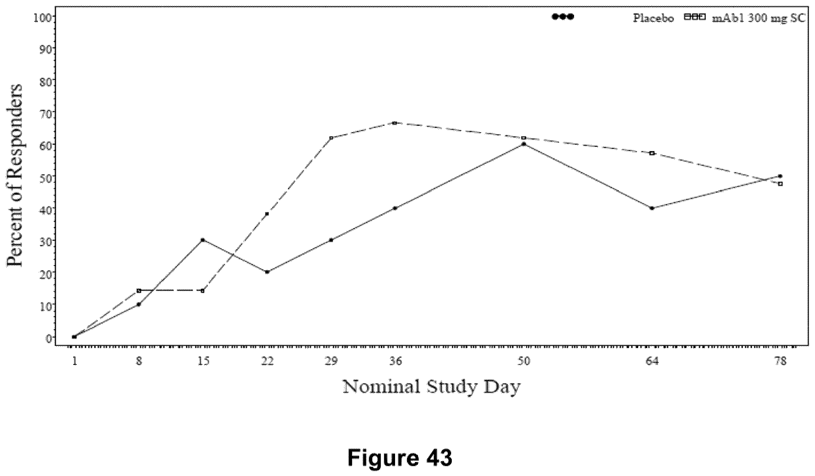

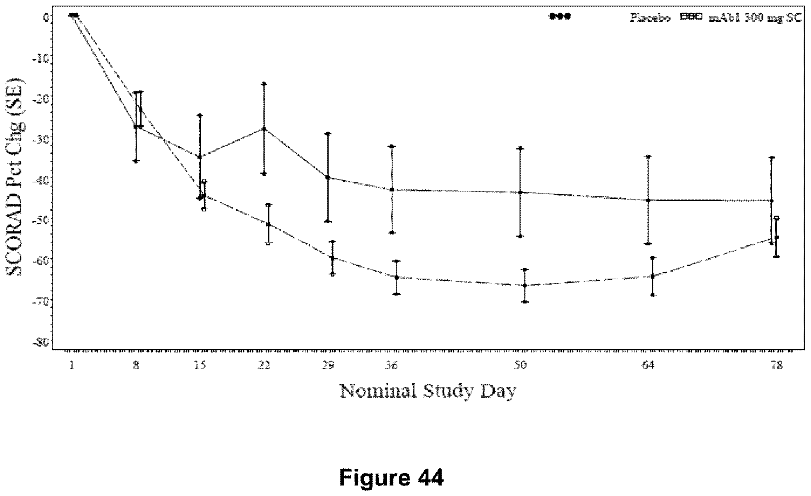

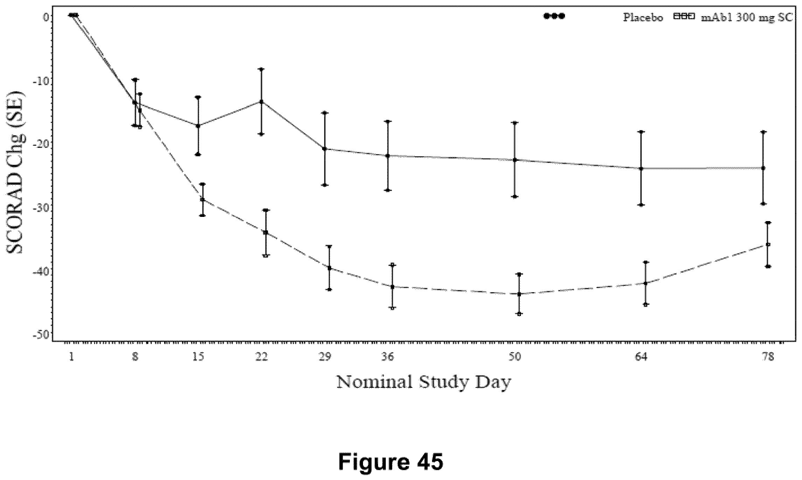

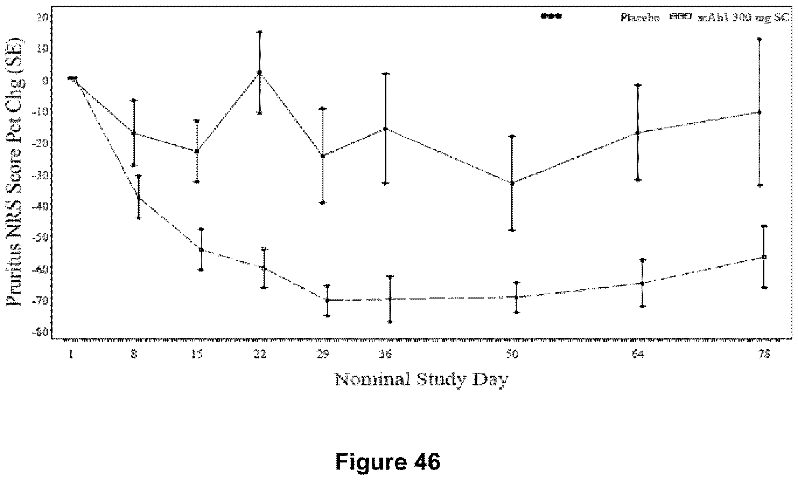

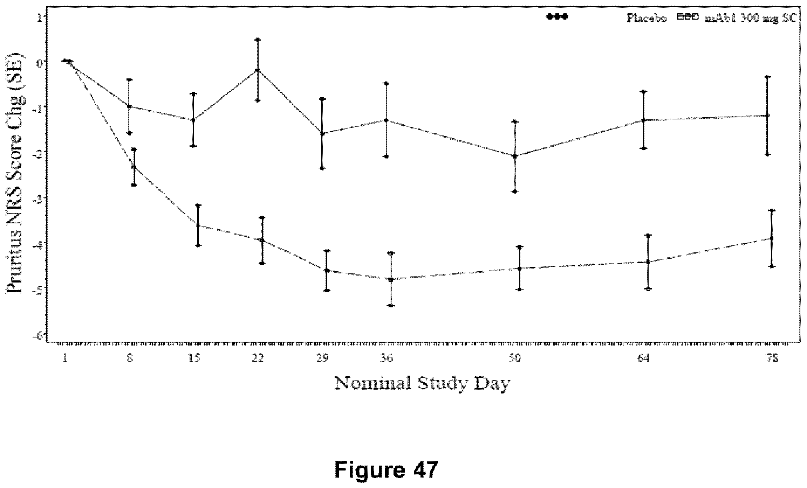

The exploratory efficacy results obtained from the study are summarized in Figures 34 - 47 and Tables 41 - 44. Despite the relatively small sample size and the limited treatment period, the analysis showed statistically significant and clinically relevant effects of mAb1 vs. placebo in key exploratory efficacy endpoints, including EASI-50 responder rate, as well as change and percent change from baseline in EASI, SCORAD, IGA, and pruritus NRS, with some improvements persisting for several weeks after the discontinuation of study treatment. For EASI-50, 100% of patients in the mAb1 plus TCS group met the responder criteria on Day 29, vs. 50% in the placebo plus TCS group (P-value 0.0015). Other endpoints like IGA 0-1 responder rate showed numerical superiority to placebo but did not reach statistical significance (47.6% vs. 30.0% for placebo). Notably, patients treated with mAb1 used on average approximately 50% less TCS, which might have underestimated the mAb1 treatment effect relative to the placebo (TCS alone) comparator group.

**Table 41: Summary of Percent and Absolute Change in EASI score from Baseline to Day 29**

| | **Placebo** | **300 mg mAb1** |
|---|---|---|
| No. Patients | 10 | 21 |
| Baseline EASI Score Mean (SD) | 24.1 (12.70) | 23.1 (12.35) |
| Day **29** EASI Score Mean (SD) | 14.4 (13.91) | 6.2 (6.68) |
| % Change from Baseline to Day **29** | -52.5 (39.53) | -75.6 (13.29) |
| Absolute change from Baseline to Day **29** | -9.7 (8.42) | -16.9 (8.06) |

**Table 42: Summary of Percent and Absolute Change in IGA score from Baseline to Day 29**

| | **Placebo** | **300 mg mAb1** |
|---|---|---|
| No. Patients | 10 | 21 |
| Baseline IGA Score Mean (SD) | 3.4 (0.47) | 3.4 (0.60) |
| Day **29** IGA Score Mean (SD) | 2.4 (1.43) | 1.6 (0.80) |
| % Change from Baseline to Day **29** | -30.6 (39.00) | -52.5 (21.44) |
| Absolute change from Baseline to Day **29** | -1.0 (1.17) | -1.8 (0.81) |

**Table 43: Summary of % and Absolute Change in SCORAD Score from Baseline to Day 29**

| | **Placebo** | **300 mg mAb1** |
|---|---|---|
| No. Patients | 10 | 21 |
| Baseline SCORAD Score Mean (SD) | 58.2 (13.83) | 66.3 (13.01) |
| Day **29** SCORAD Score Mean (SD) | 37.1 (25.11) | 26.4 (13.53) |
| % Change from Baseline to Day **29** | -40.0 (33.91) | -59.8 (18.35) |
| Absolute change from Baseline to Day **29** | -21.1 (17.99) | -39.9 (15.67) |

**Table 44: Summary of % and Absolute Change in Pruritus NRS from Baseline to Day 29**

| | **Placebo** | **300 mg mAb1** |
|---|---|---|
| No. Patients | 10 | 21 |
| Baseline NRS Score Mean (SD) | 5.0 (1.39) | 6.4 (2.00) |
| Day **29** NRS Score Mean (SD) | 3.4 (1.96) | 1.8 (1.33) |
| % Change from Baseline to Day **29** | -24.7 (47.30) | -70.7 (21.45) |
| Absolute change from Baseline to Day **29** | -1.6 (2.40) | -4.6 (2.01) |

Subcutaneous administration of mAb1 to adult patients with moderate-to-severe AD treated concomitantly with TCS was generally safe and well tolerated. Treatment with mAb1 administered concomitantly with TCS was associated with significantly superior outcomes compared with TCS treatment alone. The proportion of patients achieving an EASI-50 score was numerically much greater that was seen in recent studies in which mAb1 was used as monotherapy (the best EASI-50 so far had been 75%) which suggested that mAb1 and TCS acted additively or synergistically. However, it might also be in part due to the small sample size and slight differences in the patient populations between studies.

The study demonstrated additional efficacy provided by mAb1 in patients with moderate-to-severe AD who received TCS treatment. These results suggested that combination therapy could provide additional clinical benefits to patients with moderate-to-severe AD, compared to either treatment used as monotherapy. The results also suggested the possibility of a TCS sparing effect of mAb1, which could potentially lead to safer long term management of patients with AD.

### Example 12: Biomarker Analysis

Biomarker analysis was conducted on samples taken from subjects who participated in clinical trials of mAb1. In particular, IgE and thymus and activation chemokine (TARC) levels were measured in samples from patients at baseline and at different time points following initiation of study treatment(s). The Phadiatop^{™} test was performed to detect antigen-specific IgE. In addition, molecular profiling was carried out on skin lesions of patients who participated in clinical trials of mAb1.

### A. Administration of mAb1 to Healthy Subjects

In a first clinical trial, subjects were administered single intravenous (IV) (1.0, 3.0, 8.0 and 12.0 mg/kg) or subcutaneous (SC) (150 and 300 mg) doses of mAb, or placebo (see Example 2 herein). Samples for biomarker analysis were collected from the antibody- and placebo-treated subjects at days 1 (baseline), 8, 29, and 85 (or early termination). Levels of IgE and TARC were measured in each sample. A p-value of <0.10 was considered statistically significant to allow for small sample size. A mixed-effect repeated measures model was used for mean analyses and non-parametric test for median analyses. The median percent change in IgE and TARC levels from patient samples are summarized in Tables 45 and 46, respectively.

**Table 45: Median Percent Change in IgE Level from Baseline Following mAb1 or Placebo Administration**

| | **subcutaneous (SC)** | | | **intravenous (IV)** | | | |
|---|---|---|---|---|---|---|---|
| | Placebo | mAb1 | | Placebo | mAb1 | | |
| | | 150 mg | 300 mg | | 3 mg/kg | 8 mg/kg | 12 mg/kg |
| Baseline | - | - | - | - | - | - | - |
| Day 8 | -1.3 | -4.4 | 0.0 | -1.3 | -3.3 | 1.3 | 0.0 |
| Day 29 | 1.0 | -8.5 | -15.4 | 1.0 | -10.7 | -8.9 | -7.9 |
| Day 85 | 14.7 | -16.5 | -17.2 | 14.7 | -4.8 | -10.7 | -25.6 |

**Table 46: Median Percent Change in TARC Level from Baseline Following mAb1 or Placebo Administration**

| | **subcutaneous (SC)** | | | **intravenous (IV)** | | | |
|---|---|---|---|---|---|---|---|
| | Placebo | mAb1 | | Placebo | mAb1 | | |
| | | 150 mg | 300 mg | | 3 mg/kg | 8 mg/kg | 12 mg/kg |
| Baseline | - | - | - | - | - | - | - |
| Day 8 | 7.7 | -16.0 | -34.9 | 7.7 | -28.2 | -20.0 | 3.7 |
| Day 29 | 4.1 | -14.8 | -21.0 | 4.1 | 0.8 | -0.4 | -25.2 |
| Day 85 | -5.7 | -29.9 | -15.1 | -5.7 | -0.5 | -1.6 | -17.0 |

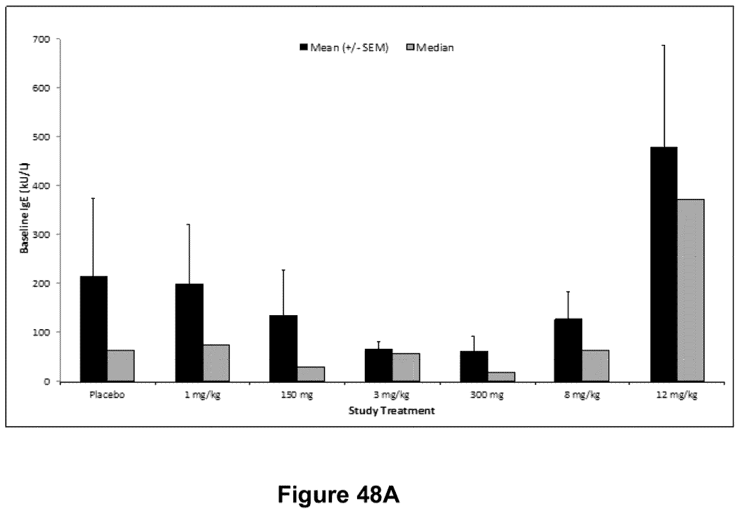

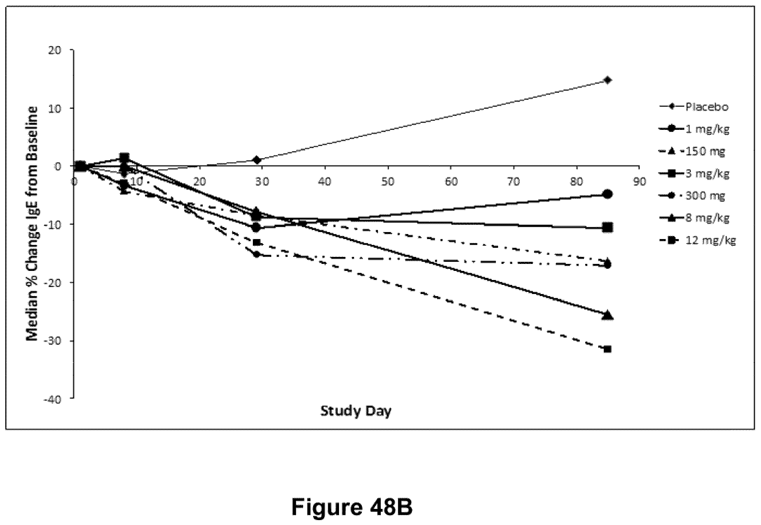

Baseline levels of IgE were highly variable as shown in the comparison of mean and median baseline IgE per treatment group (Figure 48A). The laboratory reference range for the utilized IgE assay is 0-114 kU/L and 15 of the 40 subjects had total IgE levels >114 kU/L at baseline. As shown in Table 43 and Figure 48B, IgE levels generally declined in proportion to the mAb1 dose and exposure time. By Day 85 after administration, subjects receiving SC-administered mAb1 exhibited a median decrease in IgE level of 16.5% (150 mg) and 17.2% (300 mg). Significant IgE decreases were also observed in patients receiving IV-administered mAb1, with decreases in IgE of 10.7% and 25.6% in the 8 mg/kg and 12 mg/kg groups, respectively. By contrast, IgE levels increased over time in placebo treated subjects.

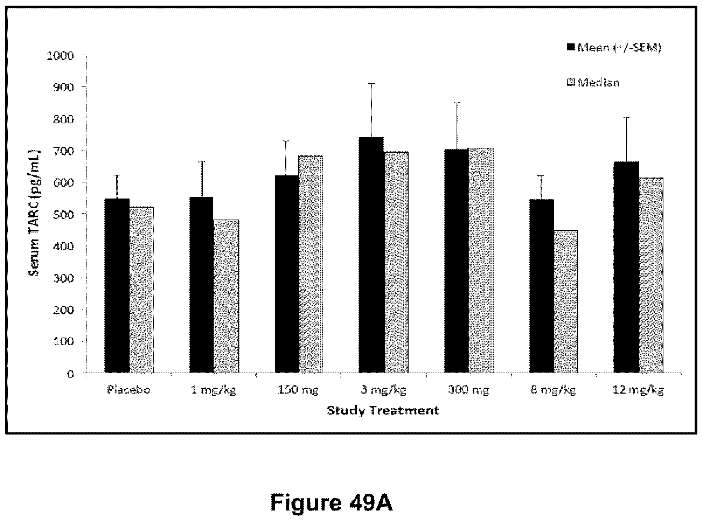

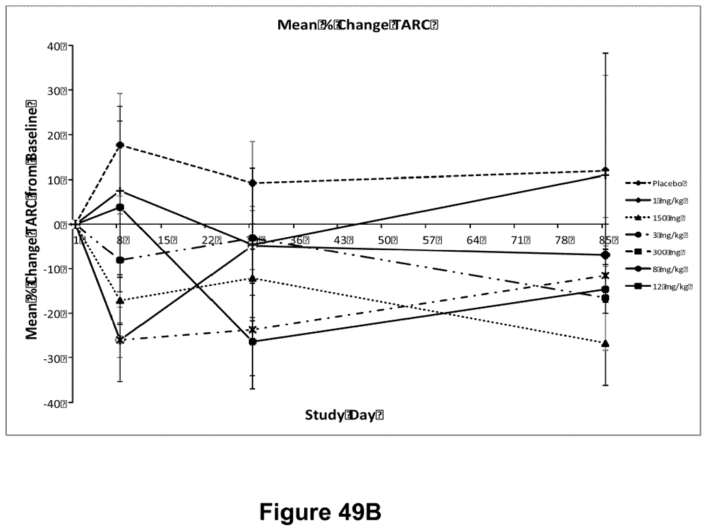

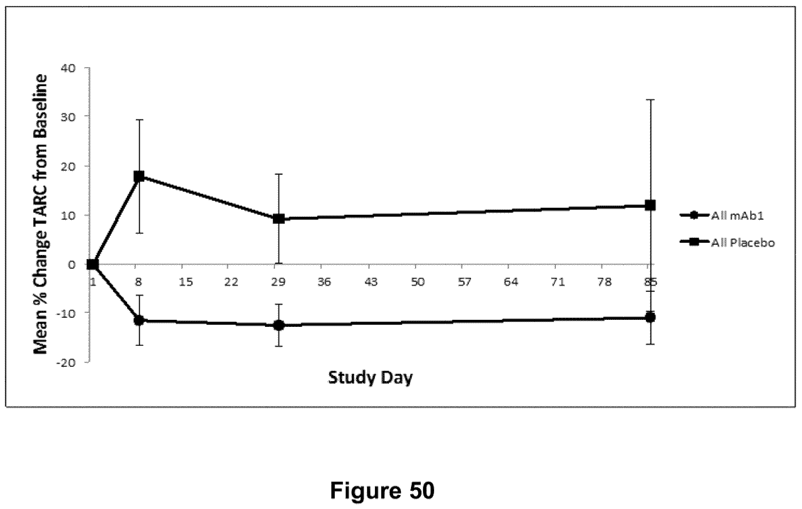

Median serum TARC levels at baseline were generally comparable between the treatment groups (Figure 49A), and were higher than those reported in the literature. Mean baseline TARC was 616 pg/mL with a range of 134-1327 pg/mL. TARC levels in healthy subjects have been reported in the range of 106-431 ng/L (Hijnen et al 2004, J. Allergy Clin. Immunol. 113: 334-340). Significant reductions in TARC were observed in samples taken from mAb1-treated subjects compared to placebo with both subcutaneous doses (p=0.044 for 150 mg and p=0.047 for 300mg) (Figure 49B and Table 44). For example, a single SC dose of 300 mg of mAb1 caused a median decrease in TARC level by almost 35% at day 8 (p=0.052), while TARC level increased by 7.7% in placebo-treated patients. Significant decreases in TARC levels were sustained in mAb1-treated patients at Day 29 through the end of the study (Day 85) in both SC- and IV-administered groups. When data was pooled for all subjects treated with mAb1, the overall difference between mAb1 and placebo for percent change from baseline in TARC was significant (p=0.004) (Figure 50). Significant differences were also observed at days 8 (p=0.012) and 29 (p=0.022).

### B. Administration of mAb1 to Subjects with Atopic Dermatitis

Biomarker levels were also measured in samples from two separate clinical trials involving subjects with atopic dermatitis (AD). In "Study A", AD subjects were administered either mAb1 (75, 150 or 300 mg) or placebo, on days 1, 8, 15 and 22 of the study (i.e., four weekly doses). In "Study B", AD subjects were administered 150 mg or 300 mg of mAb1, or placebo, on days 1, 8, 15 and 22 of the study (i.e., four weekly doses) (see Example 7 herein). All administrations for both studies were subcutaneous (SC). Samples for biomarker analysis were collected from the antibody- and placebo-treated subjects from both studies at days 1 (baseline), 4, 8, 15, 22, 25, 29, 36, 43, 50, 57, 64, 71 and 85 (or early termination). Levels of IgE, TARC, lactate dehydrogenase (LDH), and antigen-specific IgE (Phadiatop) were measured in each sample.

Serum TARC was measured using a validated assay (Human CCL17/TARC Quantikine ELISA kit, R&D Systems; validation and assays performed by Quest Diagnostics). Total serum IgE levels were determined using the ImmunoCAP^{®} Total IgE test (Thermo Scientific FDA cleared test; performed by Quest Diagnostics). Lactate dehydrogenase (LDH) was measured using the Roche Modular test (FDA cleared; performed by Covance Central Laboratories). Phadiatop^{®} (Thermo Scientific FDA cleared test) assays were performed by Viracor-IBT. Two-sample median test was used to compare the biomarker changes from baseline with mAb1 to placebo.

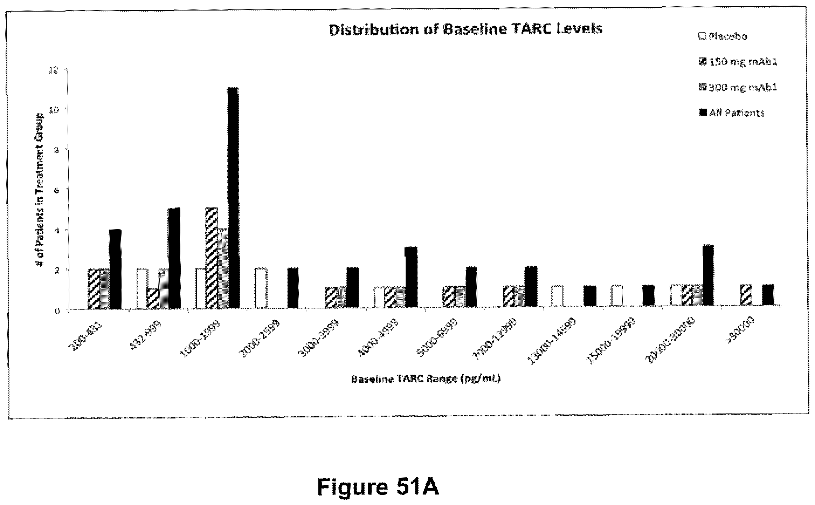

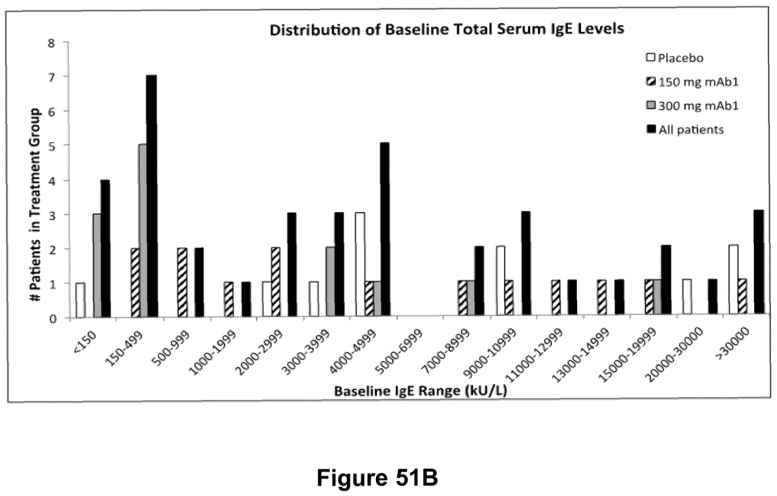

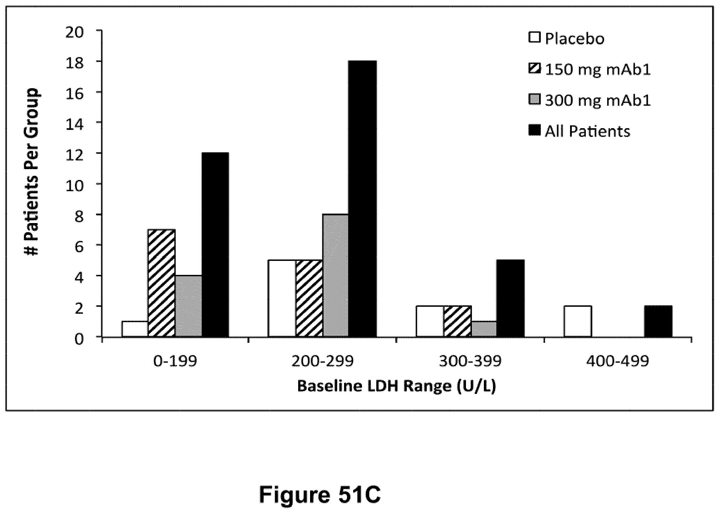

Mean baseline levels of serum TARC, total IgE and LDH for all AD patients enrolled in study 'B' were higher than the reported upper limit of normal (ULN) (Table 47 and Figure 51).

**Table 47: Summary of baseline biomarker characteristics**

| **Biomarker** | **Mean Baseline (SEM) All patients (n=37)** | **Mean Baseline (SEM) Placebo (n=10)** | **Mean Baseline (SEM) 150 mg DPL (n=14)** | **Mean Baseline (SEM) 300 mg DPL (n=14)** |
|---|---|---|---|---|
| **EASI** | 28.4 (2.56) | 25.64 (4.34) | 52.55 (4.96) | 25.91 (3.71) |
| TARC (pg/mL) | 6914.9 (2001.3) | 7001 (2669.8) | 9162.2 (4851.7) | 4601.4 (1957.3) |
| IgE (kU/L) | 8038.3 (2052.2) | 15026.5 (4748.6) | 7251.9 (2634.1) | 2951.7 (1589.4) |
| Phadiatop* | 34/36 patients were + | All + | All + | 2 patients were - |
| LDH (U/L) | 240.4 (13.4) | 296.7 (28.8) | 226.1 (21) | 212.5 (16.1) |
| Eos (10^9 cells/L) | 0.50 (0.06) | 0.65 (0.13) | 0.49 (0.09) | 0.41 (0.11) |
| Eos (%) | 6.37 (0.71) | 8.03 (1.06) | 6.17 (1.18) | 5.29 (1.32) |

Mean baseline eosinophil levels were at the high end of the reference range (Table 47). All but 2 patients with available data tested positive for the Phadiatop test. Both of these patients also had normal total serum IgE levels. Phadiatop results were unavailable for one patient.

A broad spectrum of baseline TARC and IgE was observed in the enrolled moderate-to-severe AD population. 27/36 of patients had serum TARC levels >1000pg/mL (∼twice the mean levels reported for healthy volunteers (Figure 51A). 32/36 of patients had IgE levels ≥150 kU/L (a cutoff often cited to distinguish extrinsic and intrinsic AD) (Figure 51B). 17/37 had LDH levels above 234 U/L (Figure 51C). No patients had LDH levels below 100 U/L.

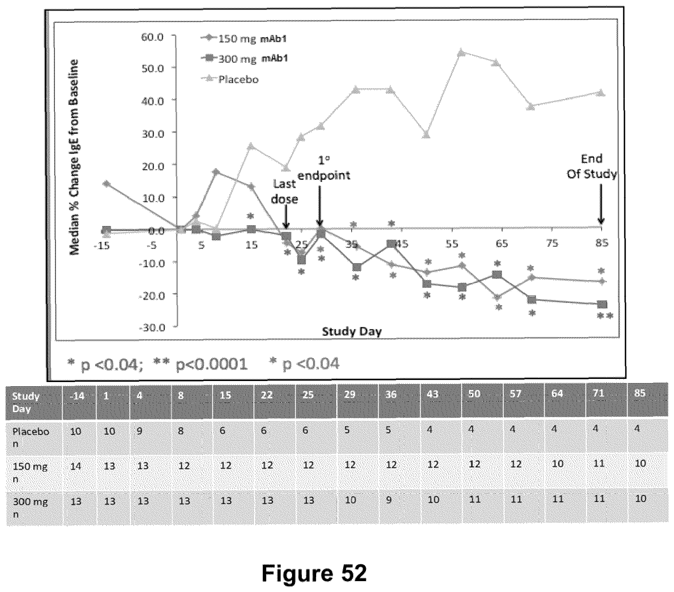

Using local linear regression, an overall mAb1 treatment effect (percent change from baseline) on total serum IgE was observed compared to placebo in both dose groups (p<0.0001) (Figure 52). Total serum IgE levels decreased with mAb1 treatment, while an overall increase was observed at the end of the study in the placebo treated group.

The median percent change in IgE levels from baseline for each group from both studies A and B (combined data) is summarized in Table 48.

**Table 48: Median Percent Change in IgE Level from Baseline (Study A & B Combined)**

| | **subcutaneous (SC)** | | | |
|---|---|---|---|---|
| | Placebo | mAb1 | | |
| | | 75 mg | 150 mg | 300 mg |
| *Baseline | - | - | - | - |
| Day 4 | 2.7 | TBD | 4.3 | 0.0 |
| *Day 8 | 0.2 | TBD | 17.6 | -2.2 |
| *Day 15 | 25.7 | TBD | 13.2 | 0.0 |
| *Day 22 | 19.0 | TBD | -4.4 | -2.1 |
| Day 25 | 28.4 | TBD | -7.4 | -9.5 |
| Day 29 | 32.0 | TBD | 0.2 | -1.6 |
| Day 36 | 43.0 | TBD | -5.5 | -12.1 |
| Day 43 | 43.0 | TBD | -11.2 | -4.8 |
| Day 50 | 28.9 | TBD | -13.7 | -17.3 |
| Day 57 | 54.2 | TBD | -11.7 | -18.3 |
| Day 64 | 51.2 | TBD | -21.6 | -14.3 |
| Day 71 | 37.5 | TBD | -15.4 | -22.3 |
| Day 85 | 41.7 | TBD | -16.8 | -23.9 |

| | | | | |
|---|---|---|---|---|
| * Denotes days when drug or placebo was administered | | | | |

As shown in Table 48 and Figure 52, a statistically significant decrease in IgE was observed in samples from mAb1-treated subjects compared to placebo. The median percent change IgE at day 85 was -23.9% in patients treated with 300 mg mAb1, compared to a 41.7% increase in the placebo group (p<0.0001). The median percent change from baseline in the 150mg group compared to placebo was significant at all time-points from days 29-85 (p<0.03). The median percent change from baseline in the 300mg group compared to placebo was significant at all time-points from days 15-85 (p<0.04).

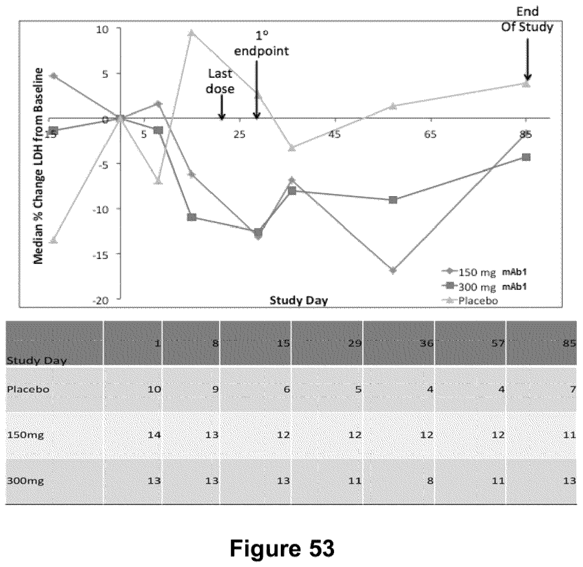

Using local linear regression, an overall treatment effect was observed for LDH. There was a statistically significant decrease in LDH in the 300 mg treatment group (p=0.0051) (Figure 53). Median percent change was not statistically significant at any single time point, however, a temporal trend was observed (p=0.008).

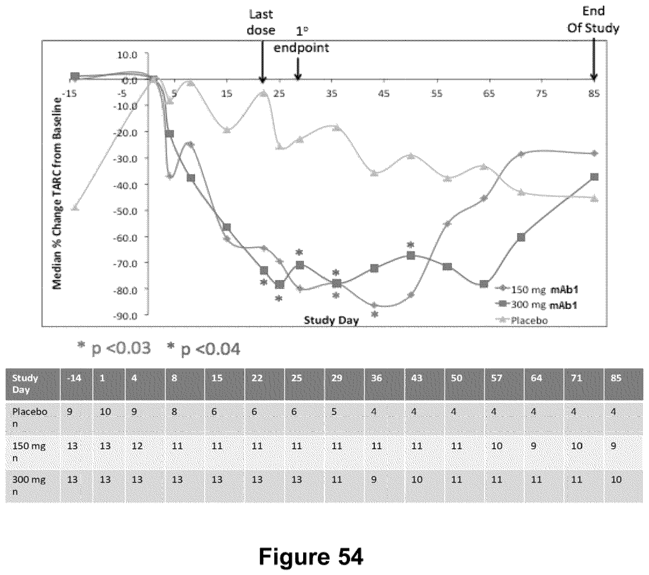

mAb1 treatment rapidly suppressed serum TARC levels in AD patients (Figure 54). The median percent change in TARC levels from baseline for each group from both studies (combined data) is summarized in Table 49.

**Table 49: Median Percent Change in TARC Level from Baseline (Study A and B combined)**

| | **subcutaneous (SC)** | | | |
|---|---|---|---|---|
| | Placebo | mAb1 | | |
| | | 75 mg | 150 mg | 300 mg |
| *Baseline | - | -- | - | - |
| Day 4 | -7.9 | TBD | -37.0 | -20.8 |
| *Day 8 | -1.1 | TBD | -24.8 | -37.5 |
| *Day 15 | -19.3 | TBD | -61.0 | -56.6 |
| *Day 22 | -5.0 | TBD | -64.4 | -73.1 |
| Day 25 | -25.5 | TBD | -69.5 | -78.5 |
| Day 29 | -22.7 | TBD | -79.9 | -70.9 |
| Day 36 | -18.3 | TBD | -78.1 | -77.9 |
| Day 43 | -35.3 | TBD | -86.3 | -72.3 |
| Day 50 | -28.9 | TBD | -82.2 | -67.4 |
| Day 57 | -37.4 | TBD | -55.2 | -71.4 |
| Day 64 | -33.2 | TBD | -45.5 | -78.1 |
| Day 71 | -43.0 | TBD | -28.6 | -60.3 |
| Day 85 | -45.2 | TBD | -28.3 | -37.3 |

| | | | | |
|---|---|---|---|---|
| * Denotes days when drug or placebo was administered | | | | |

A statistically significant reduction in serum TARC was observed in patients treated with 300 mg mAb1 compared to placebo (p<0.0001; local linear regression analysis). Statistically significant suppression was maintained through day 50 in patients treated with 300 mg mAb1, approximately one month after the last dose (administered on study day 21). The 150mg group achieved comparable magnitude of suppression, but levels were observed to increase sooner than in the 300mg group. Statistically significant suppression (median percent change TARC from baseline compared to placebo) was observed at days 36 and 43 in the 150mg group (p<0.03), as well as days 22, 25, 29, 36, and 50 with the 300mg group (p<0.04).

Intra-patient variability of TARC levels was observed over the course of the study in placebo-treated patients. Data from only 4 placebo-treated patients was available at the end of the study, due to a high dropout rate in that group.

In conclusion, TARC, IgE and LDH, biomarkers associated with Th2 inflammation and/or AD disease activity, were all suppressed by mAb1 treatment in AD patients. mAb1 rapidly decreased serum TARC levels in AD patients, compared to placebo. Duration of suppression appeared to be dose-related and data suggested that the effect might be sustained even after drug discontinuation. Total serum IgE levels significantly declined in mAb1 treated patients. IgE continued to decline (median percent change) in the 300mg group after the treatment phase, suggesting that maximal IgE suppression had not yet been achieved. A consistent reduction in LDH levels from baseline was observed in patients treated with mAb1. A direct link between LDH and IL-4 and IL-13 is unknown, but its association with disease severity suggested LDH might be a measure of the extent of skin damage in AD patients. The suppression of TARC and IgE demonstrated that mAb1 is a potent inhibitor of Th2 inflammation.

### Correlations among biomarkers and AD-associated parameters

In Study "B" (see Example 7), patients with severe AD were given 150 or 300 mg mAb1 or placebo (PBO) weekly for four weeks. Pruritus was measured using twice-daily pruritus Numeric Rating Scale (NRS; ranging from 0-10) to generate an average weekly NRS score & a bi-weekly 5-D Pruritus Scale assessments. The 5-D scale is a 5 question tool used to assess multiple dimensions of itch: degree, duration, direction, disability, and distribution. Mean baseline NRS & 5-D scores were 5.5 & 19, respectively. The average weekly NRS scores rapidly decreased (mean % change from baseline) by 31.9% at week 2 (p<0.02), & 55.2% at week 7 (p=0.01) in the 300 mg group vs +1.3% and -17.3% respectively in the PBO group. Rapid reduction in 5-D scores was also observed in patients treated with 300 mg mAb1 (mean % change -28.2% at day 15, p=0.0009; -37.1% at day 29, p=0.0007; -42.5% at day 43, p=0.012; +3.6%, +8.1% & -9.4% respectively in the PBO group). Serum levels of CCL17, a marker of IL4/IL13 activity, also rapidly declined on treatment. Both CCL17 and pruritus were suppressed for several weeks following the end of treatment. Table 50 shows the correlation of pruritus (5D and NRS) with outcomes of dermatitis (EASI) and CCL17.

**Table 50**

| Time point | 5D correlations | | | | NRS correlations | | | |
|---|---|---|---|---|---|---|---|---|
| | EASI | | CCL17 | | EASI | | CCL17 | |
| | Spearman *r* | *P* | Spearman *r* | *P* | Spearman *r* | *P* | Spearman *r* | *P* |
| Actual Values | | | | | | | | |
| Baseline | 0.41 | 0.0111 | 0.46 | 0.0044 | 0.35 | 0.0321 | 0.42 | 0.0117 |
| Day 29 | 0.62 | 0.0001 | 0.55 | 0.0024 | 0.64 | <0.0001 | 0.12 | 0.5413 |

| Percent change from baseline | | | | | | | | |
|---|---|---|---|---|---|---|---|---|
| Day15 | 0.65 | <0.0001 | 0.46 | 0.0089 | 0.51 | 0.0012 | 0.32 | 0.0795 |
| Day29 | 0.61 | <0.0001 | 0.48 | 0.0105 | 0.61 | <0.0001 | 0.11 | 0.5640 |

Overall, for all treatment groups, the 5D score significantly correlated with CCL17 (r=0.46, p=0.004 at baseline; r=0.55, p=0.002 at day 29) & EASI scores in this study (r=0.41, p=0.011 at baseline; 0.62, p<0.0001 at day 29). The percent change in 5D significantly correlated with the percent change from baseline in EASI (r=0.65, p<0.0001 for day 15; and r=0.61, p<0.0001 for day 29) and CCL17 (r=0.46, p=0.0089 for day 15; and r=0.48, p=0.0105 for day 29) for the overall treatment groups at Days 15 and 29. Treatment groups were also individually assessed for correlation of Pruritus 5D with EASI and CCL17. At day 15, only the 150mg group demonstrated strong and significant correlation between the percent change in EASI and percent change in 5D (r=0.81, p=0.0005). Similarly at day 29, the only significant correlation was for the 150mg group (r=0.57, p=0.0036). Although there was a significant overall correlation between percent change in CCL17 and percent change in 5D score at both day 15 and day 29, none of the individual treatment groups showed such a correlation at either day.

Pruritus severity, assessed using the NRS, showed moderate to strong correlations with EASI that were significant. However, NRS values correlated with CCL17 values only at baseline, with no significant correlation for percent change from baseline. The rapid & sustained improvement in pruritus observed in adult AD patients treated with mAb1 suggests IL-4/IL-13 signaling is a key mechanism for AD pruritus. The correlation between pruritus and CCL17 levels highlights the relationship between IL-4/IL-13 mediated inflammation, AD disease activity & pruritus in severe AD.

### C. Repeated Administration of mAb1 to Subjects with moderate-to-severe Atopic Dermatitis

IgE and TARC levels were measured in samples from a clinical trial involving subjects with moderate-to-severe atopic dermatitis (AD). AD subjects were administered 300 mg of mAb1, or placebo, on days 1, 8, 15, 22, 29, 36, 43, 50, 57, 64, 71 and 78 of the study (i.e., 12 weekly doses) (see Example 10 herein). All administrations for both studies were subcutaneous (SC). Serum samples for biomarker analysis were collected from the antibody- and placebo-treated subjects from both studies at days 1 (baseline), 8, 15, 22, 25, 29, 36, 43, 50, 57, 64, 71, 85, 99, 113, 127, 141, 155, 169, 183 and 197 (end of study) or early termination. Levels of IgE, TARC and antigen-specific IgE (Phadiatop^{™} test) were measured in each sample.

TARC is a chemokine induced by IL-4/IL-13, shown to be strongly associated with disease severity of AD, and may be involved in pathogenesis of the disease. Baseline TARC levels were assessed for potential predictive value for treatment response. Post-treatment samples were evaluated for pharmacodynamics effect of mAb1 on TARC.

Patients with AD often have elevated IgE. Total IgE levels have been found to correlate with AD severity and may be involved in the pathogenesis of the disease. Baseline IgE levels were assessed for potential predictive value for treatment response. Post-treatment samples were evaluated for pharmacodynamics effects of mAb1 on total IgE.

The Phadiatop^{™} test is an invitro diagnostic screening tool used to detect antigen-specific IgE for common inhalants. Baseline results of the Phadiatop^{™} test were assessed for potential predictive value for treatment response. Post-treatment samples were evaluated for pharmacodynamics effects of mAb1 on the Phadiatop^{™} antigen panel.

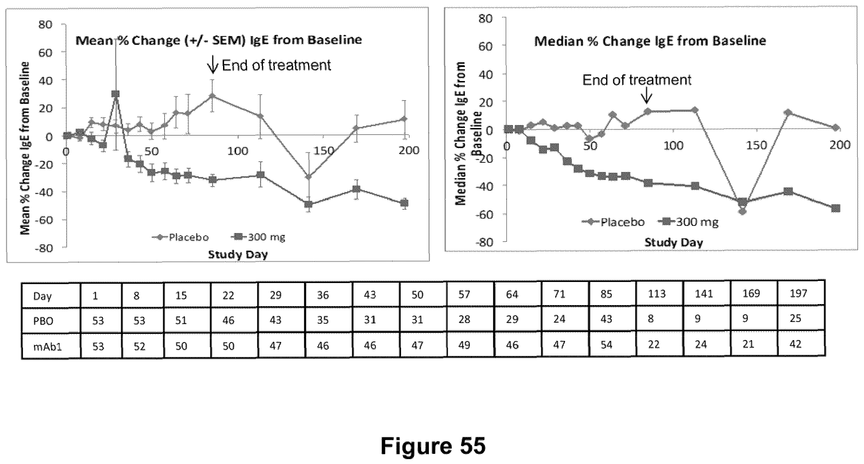

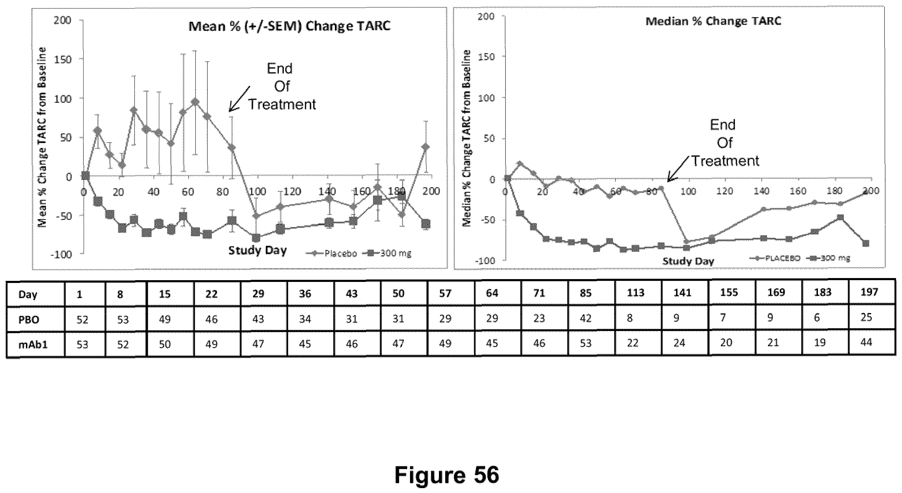

In line with the results obtained from earlier clinical trials (see sections A and B above), the TARC and IgE levels decreased and remained suppressed below baseline through the 16-week post-treatment follow-up period (Figures 55 - 56).

Greater magnitude of IgE suppression was observed with 12 weeks of 300mg mAb1 treatment during a 16-week followup (median -57%) as compared 4 weeks of mAb1. Magnitude of TARC suppression was comparable at the end-of-treatment after 12 weeks (median -83%) and weeks (median -76%) of mAb1 treatment.

### D. Concomitant administration of mAb1 with topical corticosteroids in patients with moderate-to-severe atopic dermatitis

TARC and IgE modulation was studied in a trial evaluating the safety and efficacy of mAb1 in combination with topical corticosteroids (TCS) in adult patients with moderate-to-severe AD. Two treatment groups were compared (weekly dosing for 4 weeks): (300 mg mAb1 + TCS), versus (placebo + TCS). TCS were administered from day 1 up to day 28 (patients stopped TCS treatment if lesions cleared) (see Example 11 herein). Patients were evaluated at screening, baseline (day 1), weekly through week 5, then every other week through week 11. TARC levels decreased in both treatment groups, with a trend for greater suppression in the mAb1 + TCS group compared to placebo (PBO) + TCS. Differences were statistically significant at days 22, 29 and 50. IgE levels also decreased in both treatment groups. There was no statistically significant difference in IgE suppression between groups.

**Table 51: Baseline TARC by treatment group**

| Treatment group | Placebo+TCS | 300mq mAb1+TCS |
|---|---|---|
| n | 9 | 20 |
| Mean (SD; pg/mL) | 2999.2 (4063.88) | 2703.4 (3411.76) |
| Median (pg/mL) | 913 | 1444 |
| Min:max | 325:11966 | 347:14100 |

TARC was measured using the R&D Systems human TARC Quantikine ELISA kit. Table 51 summarizes the baseline TARC levels by treatment group. The mean and median baseline TARC levels for both treatment groups were above the normal range of 106-431 pg/mL (Weihrauch et al 2005; Cancer Res. 65: 13), as well as the observed baseline levels as in Section A above.

**Table 52: Mean percent change TARC from baseline with standard deviation (SD)**

| Treatment group | Study day | n | Mean % change from baseline | SD |
|---|---|---|---|---|
| Placebo+TCS | 8 | 9 | -25.5 | 36.8 |
| | 15 | 9 | -15.6 | 70.5 |
| | 22 | 9 | -5.9 | 48.5 |
| | 29 | 8 | -24.4 | 23.2 |
| | 36 | 8 | -39.7 | 35.7 |
| | 50 | 9 | -33.1 | 47.8 |
| | 64 | 8 | -25.1 | 53.2 |
| | 78 | 9 | -20.8 | 73.0 |
| 300mq mAb1+TCS | 8 | 20 | -43.8 | 30.9 |
| | 15 | 21 | -49.6 | 38.7 |
| | 22 | 20 | -51.0 | 44.1 |
| | 29 | 21 | -55.2 | 41.3 |
| | 36 | 20 | -57.9 | 42.9 |
| | 50 | 21 | -61.1 | 35.4 |
| | 64 | 21 | -36.8 | 66.8 |
| | 78 | 19 | -35.3 | 47.0 |

TARC levels decreased from baseline in both treatment groups, thus TCS alone may lower serum TARC levels in AD patients. Although the magnitude of median % change TARC from baseline was consistently larger in the mAb1+TCS group compared to placebo+TCS, the difference was only statistically significant at days 22, 29 and 50 (least square mean difference estimated from analysis of covariance) (Table 52).

The mean and median baseline IgE levels are summarized in Table 53.

**Table 53: Baseline IgE by treatment group**

| Treatment group | Placebo+TCS | 300mq mAb1+TCS |
|---|---|---|
| n | 9 | 21 |
| Mean (SD; kU/L) | 12016.4 (19659.15) | 4197.8 (5293.78) |
| Median (kU/L) | 4250 | 1728 |
| Min:Max | 119:61600 | 61:19790 |

IgE levels declined in both treatment groups. After day 29, there was a trend for the magnitude of median percent change IgE to be greater in the mAb1+TCS group, there was no statistically significant difference at any time point in the study (LS mean difference estimated from analysis of covariance; Table 54).

**Table 54: mean percent change from baseline with SD**

| Treatment group | Study day | n | Mean % change from baseline | SD |
|---|---|---|---|---|
| Placebo+TCS | 8 | 9 | -1.1 | 8.7 |
| | 15 | 9 | -10.0 | 15.7 |
| | 22 | 9 | -9.5 | 16.7 |
| | 29 | 8 | -9.4 | 23.1 |
| | 36 | 8 | -13.2 | 19.2 |
| | 50 | 9 | -13.0 | 18.3 |
| | 64 | 8 | -20.8 | 15.8 |
| | 78 | 9 | -21.5 | 18.3 |
| 300mq mAb1+TCS | 8 | 20 | 0.3 | 16.1 |
| | 15 | 21 | 8.0 | 46.4 |
| | 22 | 20 | 0.7 | 44.0 |
| | 29 | 21 | -7.3 | 40.5 |
| | 36 | 20 | -1.8 | 49.2 |
| | 50 | 21 | -10.0 | 43.7 |
| | 64 | 21 | -22.2 | 44.0 |
| | 78 | 19 | -29.0 | 39.4 |

Approximately 50% of patients achieved at least an EASI50 by day 29 on placebo+TCS and the suppression of TARC and IgE in this group was consistent with the clinical improvement observed. All patients on mAb1+TCS achieved at least an EASI50 by day 29 (see Example 11). Trends were observed for greater suppression of TARC and IgE in the mAb1+TCS compared to placebo+TCS. However, the only statistically significant differences observed were in TARC suppression at days 22, 29 and 50.

## Claims

1. An anti-interleukin-4-receptor (IL-4R) antibody for use in a method of treating moderate-to-severe atopic dermatitis (AD) in a patient, the method comprising administering an initial dose of the antibody followed by at least eight secondary doses of the antibody, wherein:
- the initial dose is a loading dose of 300mg or 600mg, followed by the secondary doses which are maintenance doses of 300mg;
- each secondary dose is administered to the patient 1 to 2 weeks after the immediately preceding dose; and
- the antibody comprises an HCVR having the amino acid sequence of SEQ ID NO: 162 and an LCVR having the amino acid sequence of SEQ ID NO: 164.

2. An anti-interleukin-4-receptor (IL-4R) antibody for use according to claim 1, wherein the patient is resistant, non-responsive or inadequately responsive to treatment by either a topical corticosteroid (TCS) or a calcineurin inhibitor.

3. An anti-interleukin-4-receptor (IL-4R) antibody for use according to claim 1 or 2, wherein the antibody is administered concomitantly with a topical corticosteroid (TCS).

4. An anti-interleukin-4-receptor (IL-4R) antibody for use according to claim 2 or 3, wherein the dosage of TCS is reduced by more than 10%, 20%, 30%, 40%, or 50% as compared to the dosage used by the patient before treatment with the anti-interleukin-4-receptor (IL-4R) antibody.

5. An anti-interleukin-4-receptor (IL-4R) antibody for use according to any one of the preceding claims, wherein the secondary doses are administered weekly.

6. An anti-interleukin-4-receptor (IL-4R) antibody for use according to any one of the preceding claims, wherein the antibody is administered subcutaneously.

7. An anti-interleukin-4-receptor (IL-4R) antibody for use according to any one of the preceding claims, wherein the antibody is present in a reusable or disposable pen, autoinjector delivery device, or needle and syringe.

8. An anti-interleukin-4-receptor (IL-4R) antibody for use according to claim 7, wherein the antibody is present in a disposable pen delivery device prefilled with a pharmaceutical composition comprising the antibody, where the pharmaceutical composition is held in a reservoir within the device.

## Patentansprüche

1. Anti-Interleukin-4-Rezeptor- (IL-4R-) Antikörper für die Verwendung in einem Verfahren zum Behandeln von mittel schwerer bis schwerer atopischer Dermatitis (AD) bei einem Patienten, wobei das Verfahren das Verabreichen einer Anfangsdosis des Antikörpers, gefolgt von wenigstens acht Sekundärdosen des Antikörpers umfasst, wobei:
- die Anfangsdosis eine Aufsättigungsdosis von 300 mg oder 600 mg ist, gefolgt von den Sekundärdosen, die Erhaltungsdosen von 300 mg sind;
- jede Sekundärdosis dem Patienten 1 bis 2 Wochen nach der unmittelbar vorhergehenden Dosis verabreicht wird; und
- der Antikörper eine HCVR mit der Aminosäuresequenz von SEQ ID NO: 162 und eine LCVR mit der Aminosäuresequenz von SEQ ID NO: 164 umfasst.

2. Anti-Interleukin-4-Rezeptor- (IL-4R-) Antikörper für die Verwendung nach Anspruch 1, wobei der Patient resistent ist, nicht oder nur unzureichend auf die Behandlung mit entweder einem topischen Kortikosteroid (TCS) oder einem Calcineurin-Inhibitor anspricht.

3. Anti-Interleukin-4-Rezeptor- (IL-4R-) Antikörper für die Verwendung nach Anspruch 1 oder 2, wobei der Antikörper gleichzeitig mit einem topischen Kortikosteroid (TCS) verabreicht wird.

4. Anti-Interleukin-4-Rezeptor- (IL-4R-) Antikörper für die Verwendung nach Anspruch 2 oder 3, wobei die Dosierung von TCS um mehr als 10 %, 20 %, 30 %, 40 % oder 50 % im Vergleich zu der von dem Patienten vor der Behandlung mit dem Anti-Interleukin-4-Rezeptor- (IL-4R-) Antikörper verwendeten Dosierung reduziert ist.

5. Anti-Interleukin-4-Rezeptor- (IL-4R-) Antikörper für die Verwendung nach einem der vorhergehenden Ansprüche, wobei die Sekundärdosen wöchentlich verabreicht werden.

6. Anti-Interleukin-4-Rezeptor- (IL-4R-) Antikörper für die Verwendung nach einem der vorhergehenden Ansprüche, wobei der Antikörper subkutan verabreicht wird.

7. Anti-Interleukin-4-Rezeptor- (IL-4R-) Antikörper für die Verwendung nach einem der vorhergehenden Ansprüche, wobei der Antikörper in einem wiederverwendbaren oder Einweg-Stift, einer Autoinjektorabgavevorrichtung oder einer Nadel und Spritze vorhanden ist.

8. Anti-Interleukin-4-Rezeptor- (IL-4R-) Antikörper für die Verwendung nach Anspruch 7, wobei der Antikörper in einer Einweg-Stiftabgabevorrichtung vorhanden ist, die mit einer pharmazeutischen Zusammensetzung vorgefüllt ist, die den Antikörper umfasst, wobei die pharmazeutische Zusammensetzung in einem Reservoir innerhalb der Vorrichtung gehalten wird.

## Revendications

1. Anticorps anti-récepteur de l'interleukine-4 (IL-4R) destiné à être utilisé dans un procédé de traitement de la dermatite atopique (MA) modérée à sévère chez un patient, le procédé comprenant l'administration d'une dose initiale de l'anticorps suivie d'au moins huit doses secondaires de l'anticorps, dans lequel :
- la dose initiale est une dose d'attaque de 300 mg ou 600 mg, suivie des doses secondaires qui sont des doses d'entretien de 300 mg ;
- chaque dose secondaire est administrée au patient 1 à 2 semaines après la dose immédiatement précédente ; et
- l'anticorps comprend une HCVR ayant la séquence d'acides aminés de SEQ ID NO : 162 et une LCVR ayant la séquence d'acides aminés de SEQ ID NO : 164.

2. Anticorps anti-récepteur de l'interleukine-4 (IL-4R) destiné à être utilisé selon la revendication 1, dans lequel le patient est résistant, ne répond pas ou ne répond pas de manière adéquate au traitement par un corticostéroïde topique (TCS) ou un inhibiteur de la calcineurine.

3. Anticorps anti-récepteur de l'interleukine-4 (IL-4R) destiné à être utilisé selon la revendication 1 ou 2, dans lequel l'anticorps est administré de manière concomitante avec un corticostéroïde topique (TCS).

4. Anticorps anti-récepteur de l'interleukine-4 (IL-4R) destiné à être utilisé selon la revendication 2 ou 3, dans lequel la dose de TCS est réduite de plus de 10 %, 20 %, 30 %, 40 % ou 50 % par rapport à la posologie utilisée par le patient avant le traitement par l'anticorps anti-récepteur de l'interleukine-4 (IL-4R).

5. Anticorps anti-récepteur de l'interleukine-4 (IL-4R) destiné à être utilisé selon l'une quelconque des revendications précédentes, dans lequel les doses secondaires sont administrées chaque semaine.

6. Anticorps anti-récepteur de l'interleukine-4 (IL-4R) destiné à être utilisé selon l'une quelconque des revendications précédentes, dans lequel l'anticorps est administré par voie sous-cutanée.

7. Anticorps anti-récepteur de l'interleukine-4 (IL-4R) destiné à être utilisé selon l'une quelconque des revendications précédentes, dans lequel l'anticorps est présent dans un stylo réutilisable ou jetable, un dispositif d'administration par auto-injecteur ou une aiguille et une seringue.

8. Anticorps anti-récepteur de l'interleukine-4 (IL-4R) destiné à être utilisé selon la revendication 7, dans lequel l'anticorps est présent dans un dispositif d'administration par stylo jetable prérempli d'une composition pharmaceutique comprenant l'anticorps, où la composition pharmaceutique est contenue dans un réservoir au sein du dispositif.
